# EUROPEAN PATENT SPECIFICATION

(11) **EP 1 021 403 B1**
(45) Date of publication and mention of the grant of the patent: **20.04.2005**
(21) Application number: 98942990.7
(22) Date of filing: 10.09.1998
(51) Int. Cl.: C07D 201/00

(54) **2,3-DIOXABICYCLO 3.3.1]NONANE DERIVATIVES AND ANTIMALARIAL PHARMACEUTICAL COMPOSITIONS COMPRISING THEM**
2,3-DIOXABICYCLO [3.3.1] NONANE DERIVATE UND DIE ENTHALTENDE ANTIMALARIA PHARMAZEUTISCHE ZUSAMMENSETZUNGEN
DERIVES DE 2,3-DIOXABICYCLO 3.3.1]NONANE ET COMPOSITIONS PHARMACEUTIQUES ANTIPALUDIQUES LES RENFERMANT

(30) Priority: 11.09.1997 IL 12174997
(43) Date of publication of application: 26.07.2000
(73) Proprietor: YEDA RESEARCH AND DEVELOPMENT COMPANY, LTD., Rehovot 76100 (IL); JOHNS HOPKINS UNIVERSITY, Baltimore, MD 21218 (US)
(72) Inventor: BACHI, Mario, 76354 Rehovot (IL); POSNER, Gary, H., Baltimore, MD 21208 (US); KORSHIN, Edward, Baltimore, MD 21218 (US)
(74) Representative: VOSSIUS & PARTNER
(86) International application number: PCT/IL1998/000440
(87) International publication number: WO 1999/012900

(56) References cited:
- EP-A- 0 311 955
- X.-X. XU ET AL.: TETRAHEDRON LETTERS, vol. 32, no. 41, 1991, pages 5785-5788, XP002094587 OXFORD GB

## Description

The invention described and claimed herein was made in part under a grant from the National Institutes of Health, NIH-AI-34885. The US Government has certain rights in the invention.

### FIELD OF THE INVENTION

The present invention is in the field of antiparasitic drugs and particularly relates to novel antimalarial 2,3-dioxabicyclo[3.3.1]nonane compounds.

### BACKGROUND OF THE INVENTION

Malaria is the world's most widespread infectious disease. More than 300 million people are infected, and about 2 million die of it each year.¹ Curing malaria by treatment with alkaloids like quinine and chloroquine is becoming less effective due to the continuous emergence of malaria parasites resistant to these drugs.

A new approach to malaria chemotherapy, which is raising high expectations, is based on the use of cyclic peroxides.² Indeed, artemisinin , an endoperoxide sesquiterpene, was introduced in China as a drug in 1987.³ In Western countries several compounds structurally related to artemisinin were clinically tested, but due to pharmacokinetic drawbacks and incomplete toxicological profiles, none of them were approved as a drug. Cyclic peroxide yingzhaosu A was isolated from a Chinese folk medicine, but no significant physiological and pharmacological data have so far been reported.⁴

Recently, scientists at Hoffmann-La Roche reported on the synthesis, antimalarial screening and clinical trials of 7-oxo-2,3-dioxabicyclo[3.3.1]nonanes, bearing at C(4) alkyl- or alkenyl substituents .⁵⁻⁷ *In vitro* and *in vivo* antimalarial screening, as well as clinical trials of arteflene, the most active compound in this series, gave encouraging results.

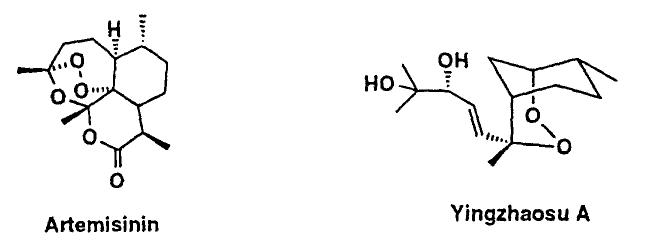

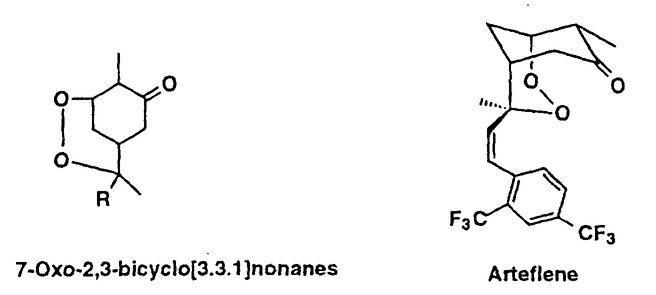

Biological evaluation of a variety of cyclic peroxides indicates that a number of them are effective non-toxic antiparasitic agents. However, parasitic infections, particularly malaria, remain a serious and widespread public health problem, and concern exists about possible side-effects of compounds developed to date. For example, neurotoxicity has been seen in rats which were administered high doses of artemether, and in mouse neuroblastoma cells treated with dihydroartemesinin.⁸

It would be highly desirable to develop improved therapeutic agents for prevention and treatment of malaria.

### SUMMARY OF THE INVENTION

The object of the present invention is to provide novel compounds for the treatment of parasitic infections, particularly malaria.

To accomplish this goal, the present invention provides 2,3-dioxabicyclo[3.3.1]nonane derivatives, carrying, at position 4, a sulfur-containing functionality selected from the group consisting of sulfonyl, sulfinyl and sulfenyl, adhered to C(4) via methylene group, represented by general structural formula A. 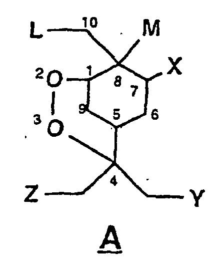 wherein:
X is hydrogen, hydroxy, C₁- C₂₀ alkoxy optionally substituted by C₁-C₂₀ alkoxy or by C₂-C₂₀ acyloxy, C₇-C₂₅ aralkoxy optionally substituted by C₁-C₂₀ alkoxy or by C₆-C₁₂ aryloxy, or C₂-C₂₀ acyloxy optionally substituted by C₁-C₂₀ alkoxy or by C₆-C₁₂ aryloxy; and
M is hydrogen, hydroxy, C₁-C₂₀ alkoxy, C₃-C₂₀ alkenyloxy, C₂-C₂₀ acyloxy optionally substituted by C₂-C₂₀ acyl or by C₂-C₂₀ acyloxy, C₇-C₂₅ aralkoxy, C₇-C₂₅ arylalkenyloxy, oxalyloxy substituted by C₁-C₂₀ alkoxy, di(C₁-C₂₀ alkyl)amino, C₁-C₂₀ alkyl(C₆-C₁₂ aryl)amino, or di(C₇-C₂₅ aralkyl)amino, or carbonyloxy substituted by C₆-C₁₂ aryloxy, di(C₁-C₂₀ alkyl)amino, C₁-C₂₀ alkyl (C₆-C₁₂ aryl)amino, or di(C₇-C₂₅ aralkyl)amino; or
X and M together represent a carbon-carbon bond or an oxygen atom;
L is hydrogen or Land M together represent a carbon-carbon bond; and
either Z is a radical R-S(=O)ₙ- and Y is hydrogen, or
Y is R-S(=O)ₙ- and Z is hydrogen,
wherein R is straight or branched C₁-C₂₀ alkyl optionally substituted by C₁-C₂₀ alkoxy or by C₂-C₂₁ alkoxycarbonyl, C₅-C₇ cycloalkyl, C₆-C₁₂ aryl optionally substituted by C₁-C₂₀ alkyl, halogen or CF₃, or C₇-C₂₅ arylalkyl optionally substituted by C₁-C₂₀ alkyl, halogen or CF₃ ; and
n is 0, 1 or 2.

The term "alkyl" as used herein tor R stands for a straight or branched C1-C20, preferably C4-C14, alkyl radical such as, but not being limited to, *n*-butyl, *tert*-butyl, 3-octyl, and *n*-dodecyl, and "substituted alkyl" includes radicals such as octyloxyethyl and ethoxycarbonylmethyl.

The term "cycloalkyl" as used herein for R stands for a C₅-C₇ cycloalkyl, preferably cyclohexyl.

The term "aryl" as used herein for R stands for a C6-C12 aryl, optionally mono or disubstituted by C1-C12 alkyl, halogen such as chloro and fluoro, or CF₃ such as, but not being limited to phenyl, biphenyl, naphthyl, 4-methylphenyl, 2,4-dimethylphenyl, 4-ffuorophenyl, 2,4-dichlorophenyl, 2,4-difluorophenyl, 4-trifluoromethylphenyl, 2,4-[di(trifluoromethyl)]phenyl and 3,5-[di(trifluoromethyl)]phenyl.

The term "arylkyl" as used herein for R stands for a C7-C25, preferably C7-C19, radical optionally substituted by alkyl, halogen such as chloro and fluoro, or CF₃ such as, but not being limited to benzyl, 4-phenylbutyl, 4-(trifluoromethyl)-benzyl and (triphenyl)methyl.

The term "alkoxy" as used herein for X and M stands for a C1-C20, preferably C1-C14, alkoxy radical. With regard to X, the alkoxy group may be substituted by alkoxy or acyloxy, and examples are, but not being limited to, methoxy, hexyloxy, dodecyloxy, butoxyethoxy, (hexanoyloxy)ethoxy, and phenethyloxy. With regard to M, examples of alkoxy are methoxy, octyloxy.

The terms "acyl" and alcyloxy" as used herein for X and M refer to C2-C20, preferably C2-C14, carboxylic acyl derived from a straight or branched, saturated or unsaturated aliphatic carbonylic acid or from an aromatic carboxylic acid. Examples of acyloxy radicals for X are, without being limited to, acetyloxy, benzoyloxy, biphenyl-4-carboxyloxy, di(phenyl)acetyloxy, butanoyloxy, octanoyloxy, dodecanoyloxy, methoxyacetyloxy, phenoxyacetyloxy. Examples of acyl and acyloxy radicals for M are, without being limited to, acetyloxy, acetylacetyloxy, 3-(acetyloxy)-but-2-enoyloxy.

The term "alkenyloxy" as used herein for M stands for a C3-C20, preferably C3-C14, alkenyloxy radical such as, but not being limited to, allyloxy, 3(methyl)but-2-enyloxy, cinnamyloxy.

Examples of substituted oxayloxy and carbonyloxy radicals for M are, without being limited to, ethoxyoxalyloxy, (methyl)-phenylaminooxalyloxy, di(benzyl)-aminooxalyloxy, phenoxycarbonyloxy.

When L and M together represent a carbon-carbon bond, this means that an alkenyl residue H₂C= is linked to the ring carbon at position 8, and when X and M together represent a carbon-carbon bond, this means that there is a double bond between the carbon atoms at positions 7 and 8 of the ring.

When X and M represent one oxygen atom, this means that they, together with the ring carbon atoms at positions 7 and 8, form an oxirane ring.

When n is 0, 1 or 2, the compounds are sulfides, sulfoxides and sulfones, respectively, and the sulfur-tethered appendages are, for example, without being limited to, alkylthio, cycloalkylthio, arylthio, aralkylthio, alkylsulfinyl, cycloalkylsulfinyl, arylsulfinyl, aralkylsulfinyl, alkylsulfony;l, arylsulfonyl, aralkylsulfonyl and substituted radicals as defined above for R.

The central molecular framework of the compounds of the invention consists of two *cis*-bridged 6-membered rings containing 4 or 5 stereogenic centers. The invention relates preferentially to compounds A represented by either three dimensional compounds of structure I characterized by stereogenic centers (1*R*, 5*R*) and compounds of structure II characterized by stereogenic centers (1*S*, 5*S*).

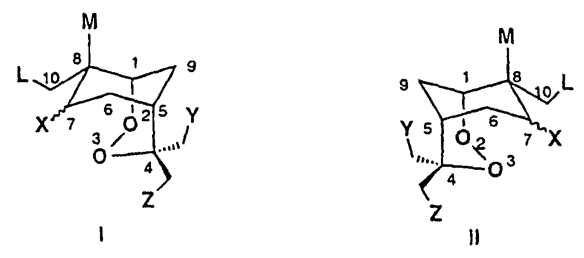

In **I** and **II**, either Y represents a sulfur-tethered appendage and Z represents hydrogen; or Z represents a sulfur-tethered appendage and Y represents hydrogen.

Structure **I** is subdivided into structures **Ia** and **Ib**, and structure **II** is subdivided into structures **IIa** and **IIb**.

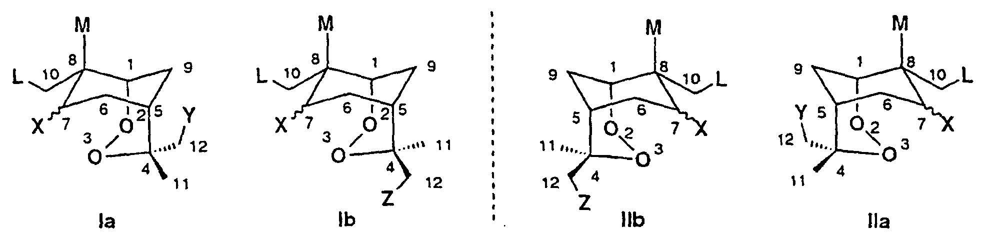

Throughout the specification C-4 epimers are given the same Arabic number and are differentiated by a and b. In sulfoxides, where there is an additional stereogenic center on the sulfur atom, epimers on sulfur are differentiated by 'e.g., a and a'.

The invention further relates to pharmaceutical compositions for the treatment of malaria comprising a compound of formula A and a pharmaceutically acceptable carrier.

In another embodiment, the invention relates to the use of a compound of formula A for the preparation of a pharmaceutical composition for the prevention and/or treatment of malaria

### DETAILED DESCRIPTION OF THE INVENTION

### Abbreviations:

- Ac: acetyl
- B n: benzyl
- br.: broad (in NMR spectroscopy)
- Bz: benzoyl
- CI: chemical ionization (in mass-spectroscopy)
- DBPO: di-*tert*-butyl peroxalate
- DCI: desorption chemical ionization (in mass-spectroscopy)
- DMAP: (4-dimethylamino)pyridine
- DP: direct phase (in chromatography)
- HPLC: high-performance liquid chromatography
- HRMS: high-resolution mass spectrum
- MCPBA: *m*- chloroperoxybenzoic acid
- MPLC: medium-pressure liquid chromatography
- R_{f}: retention factor (in chromatography)
- RP: reverse phase (in chromatography)
- rt: room temperature
- Tf: trifluoromethanesulfonyl (triflyl)
- TLC: thin layer chromatography
- TMS: trimethylsilyl
- TOCO: thiol-oxygen cooxidation
- v.s.: very strong (in IR-spectroscopy)
- τ_{R}: retention time (in HPLC)

The methodology applied for the synthesis of the novel antimalarial 2,3-dioxabicyclo[3.3.1]nonanes of type A involves one or more of the following processes:
(1) Submission of monoterpenes like *R*-(+)-limonene 1, *S*-(-)-limonene) 1' and carveol derivatives 3 to thiol oxygen cooxidation reactions (TOCO reactions), followed by treatment with a reducing agent, such as triphenylphosphine. This process is suitable for the preparation of compounds of type A (R represents an alkyl-, aralkyl- or aryl group, M represents hydroxyl, n=0) as described in General Procedure 1.
(2) Oxidation of the sulfide group in compounds of type A (R represents an alkyl, aralkyl- or aryl group, n=0) to the corresponding sulfones of type A (R represents an alkyl-, aralkyl- or aryl group, n=2) as described in General Procedure 2.
(3) Oxidation of the sulfide group in compounds A (R represents an alkyl-, aralkyl- or aryl group; n=0) to sulfoxides of type A (R represents an alkyl-, aralkyl- or aryl group; n=1), as described in General Procedure 3.
(4) Manipulations of functional groups in compounds of type A (R represents an alkyl-, aralkyl- or aryl group, n=0 or n=2) as described in General Procedures 4-8.

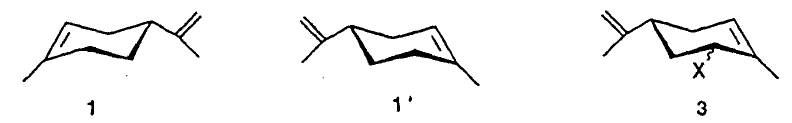

### General Procedure 1:

**Synthesis of 2,3-dioxabicyclo[3.3.1]nonane-8-ols A (R represents alkyl, aralkyl- and aryl; n= 0; M= OH)**. Through a stirred solution of monoterpene (1, 1' or 3) (*ca*. 3 equivalents, 0.02-0.10 M) and di-*tert*- butyl peroxalate (DBPO) (0.02-0.04 equivalents) in *n*-heptane -benzene mixture (*ca*. 2:1) oxygen gas is gently bubbled with simultaneous addition of a solution of thiol (1 equivalent) in benzene or heptane (5-20 mL) over a period 12 h (syringe pump). After the addition of thiol is completed the mixture is kept under oxygen for additional 12-14 h. It is then diluted with CH₂Cl₂, cooled to 0-5°C and powdered Ph₃P (1 equivalent) is added. The mixture is stirred for an additional 2 h at 0-5°C and for 1 h at rt, solvents are evaporated at rt Flash chromatography affords the title compounds, which can be used for the preparation of additional antimalarial agents of type A as described in General Procedures 2-7.

### General procedure 2:

**Preparation of sulfones A (n= 2).** A solution of a sulfide **A** (R represents alkyl, aralkyl or aryl; n= 0) (1 equivalent, *ca*. 0.2 M) and MCPBA (*ca*. 2.2-2.5 equivalents) in EtOAc is stirred at rt for 4-10 h. After consumption of the intermediate sulfoxide (TLC monitoring) the mixture is poured into a saturated solution of NaHCO₃, extracted with an organic solvent, dried (Na₂SO₄ and NaHCO₃) and evaporated. Chromatography of the residue affords the title compounds.

### General procedure 3:

**Preparation of sulfoxides A (n= 1)**. To a solution of a sulfide **A** (R represents alkyl, aralkyl or aryl; n= 0) (1 equivalent, *ca*. 0.05 M) in EtOAc at -30 ö -50°C, a solution of MCPBA (*ca*. 1.05 equivalents, *ca*. 0.06 M solution) in EtOAc is added and the mixture is stirred at -30 ö -40°C until the starting material is consumed (monitoring by TLC). The mixture is poured into a saturated solution of NaHCO₃, extracted with an organic solvent, dried (Na₂SO₄ and NaHCO₃) and evaporated. Chromatography of the residue affords the title compounds.

### General procedure 4:

**Synthesis of 2,3-dioxabicyclo[3.3.1]non-7-enes A (M and X together represent a** **carbon-carbon bond; n= 0 or n=2) and 8-methylene-2,3-dioxabicyclo[3.3.1]nonanes A (L and M together represent a carbon-carbon bond; n= 0 or n=2).** To a mixture of SOCl₂ (*ca*. 4 equivalents) and pyridine (10 equivalents) in dry CH₂Cl₂ (*ca*. 40 mL/mmol substrate) at -30 - -40°C is added during 1.5 h, a solution of a 2,3-dioxabicyclo[3.3.1]nonan-8-ol A (M=OH, X=H, n=0 or n=2) (1 equivalent, *ca*. 1 mmol/10 mL) in CH₂Cl₂. The temperature is raised to 25°C and after stirring for 5 h, the reaction mixture is poured into ice-cold 0.1 M HCl (*ca*. 25 mL/mmol of substrate), extracted with hexane/EtOAc, washed with saturated NaHCO₃, dried (Na₂SO₄ + NaHCO₃) and evaporated. Chromatography of the residue affords a mixture of the two title compounds.

### General procedure 5:

**Synthesis of 2,3-dioxabicyclo[3.3.1]nonanes A (M represents a hydrogen atom; n= 0 or n=2).** To a mixture of a 2,3-dioxabicyclo[3.3.1]non-7-enes of type A (M and X together represent a carbon-carbon bond; n= 0 or n=2) and/or 8-methylene-2,3-dioxabicyclo[3.3.1]nonanes A (L and M together represent a carbon-carbon bond; n= 0 or n=2) (1 equivalent, *ca*. 0.01 M solution), and potassium azodicarboxylate (10-15 equivalents) in MeOH/CH₂Cl₂ at 0°C is added during *ca*. 1 h, a solution of AcOH (20-30 equivalents) in CH₂Cl₂. The mixture is stirred for 2 days at rt, diluted with ether, filtered through Celite® and evaporated. Chromatography of the residue affords the title compounds.

### General procedure 6:

### Synthesis of 8-acyloxy-2,3-dioxabicyclo[3.3.1]nonanes A (M represents an acyloxy

**function; n= 0 or n=2).** To a solution of a 2,3-dioxabicyclo[3.3.1]nonan-8-ol A (M represents a hydroxyl group, n= 0 or n=2) (1 equivalent, *ca*. 0.5 M solution) and 2,6-lutidine (*ca*. 3 equivalents) in CH₂Cl₂ at 0°C is added TMSOTf (*ca*. 2 equivalent). The mixture is stirred for 30 min at 0°C and then at rt until the starting material is consumed. The reaction mixture is poured into cold water, extracted with an organic solvent, dried (Na₂SO₄ + NaHCO₃) and evaporated to give A (M represents the OTMS group; n=0 or n=2) in practically quantitative yield. A mixture of the crude TMS-derivative (1 equivalent), acyl chloride (5-8 equivalents) and oven-dried CsF (3-5 equivalents) in acetonitrile (*ca*. 10 mL/mmol) is stirred for 5-10 days at rt The mixture is poured into a saturated NaHCO₃ solution, extracted with organic solvent, dried (Na₂SO₄ + NaHCO₃) and evaporated. Chromatography of the residue affords the title compounds.

### General procedure 7:

**Synthesis of 8-alkoxy-2,3-dioxabicyclo[3.3.1] nonanes A (M represents an aralkoxy or alkenyloxy function; n=2).** To a suspension of a 2,3-dioxabicyclo[3.3.1]nonan-8-ol of type A (M represents a hydroxyl group; n= 2) (1 equivalent, *ca*. 0.3 M solution) in dry ether, at 0°C is added a concentrated solution of CCl₃C(=NH)OR (R represents an alkenyl or an (aryl)alkyl group) (3-4 equivalents) in CH₂Cl₂. Following formation of a homogeneous mixture, a solution of TfOH (*ca.* 0.03 equivalents, *ca*. 0.1 M) in dry ether is added. The reaction mixture is stirred for *ca*. 5 h at 0°C. Addition of solutions of trichloroacetimidate and TfOH is repeated until the starting hydroxysulfone is consumed. The mixture is then diluted with an organic solvent, excess of (Na₂SO₄ + NaHCO₃) is added and the mixture is stirred overnight. Concentration *in vacuo*, followed by chromatography affords the title compounds.

### General procedure 8:

**Synthesis of 7,8-epoxy-2,3-dioxabicyclo[3.3.1] nonanes A (M and X together represent an oxygen atom; n= 2).** A mixture of a 2,3-dioxabicyclo[3.3.1] non-7-ene A (M and X together represent a carbon-carbon bond; n= 2) (1 equivalent, *ca*. 0.05 M solution) and MCPBA (*ca*. 1.1 equivalents) in CH₂Cl₂ is stirred at rt for 12 h, diluted with hexane/EtOAc, washed with saturated NaHCO₃, dried (Na₂SO₄ + NaHCO₃) and evaporated. Chromatography of the residue affords the title compounds.

### EXAMPLES

The compounds in the following Examples were prepared according to Schemes 1-15, presented hereinafter.

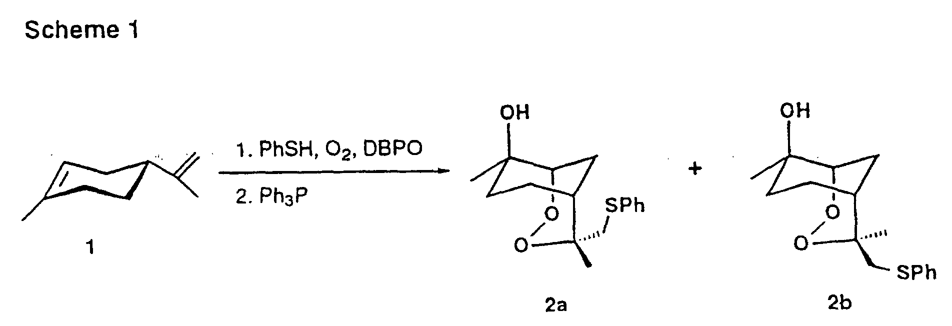

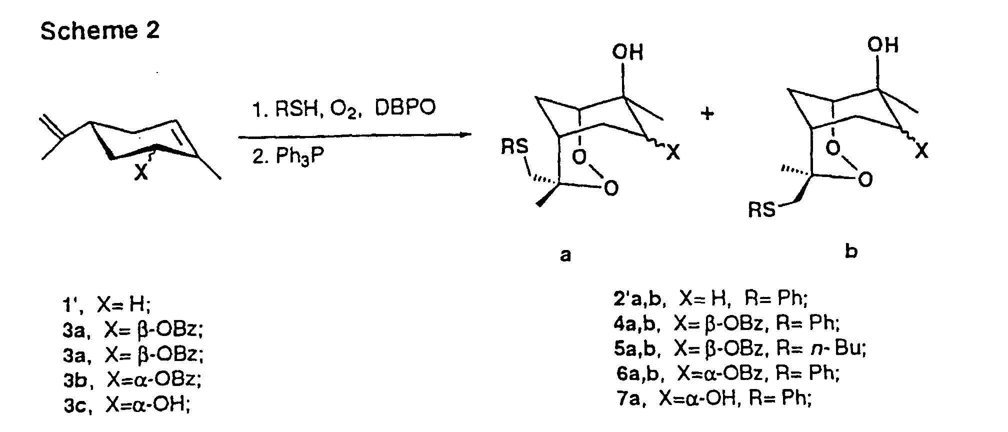

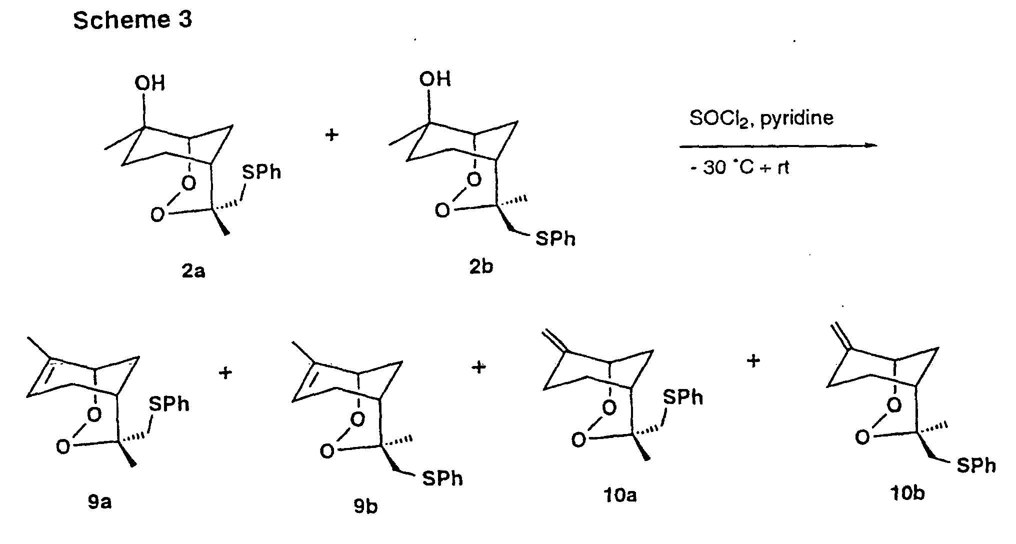

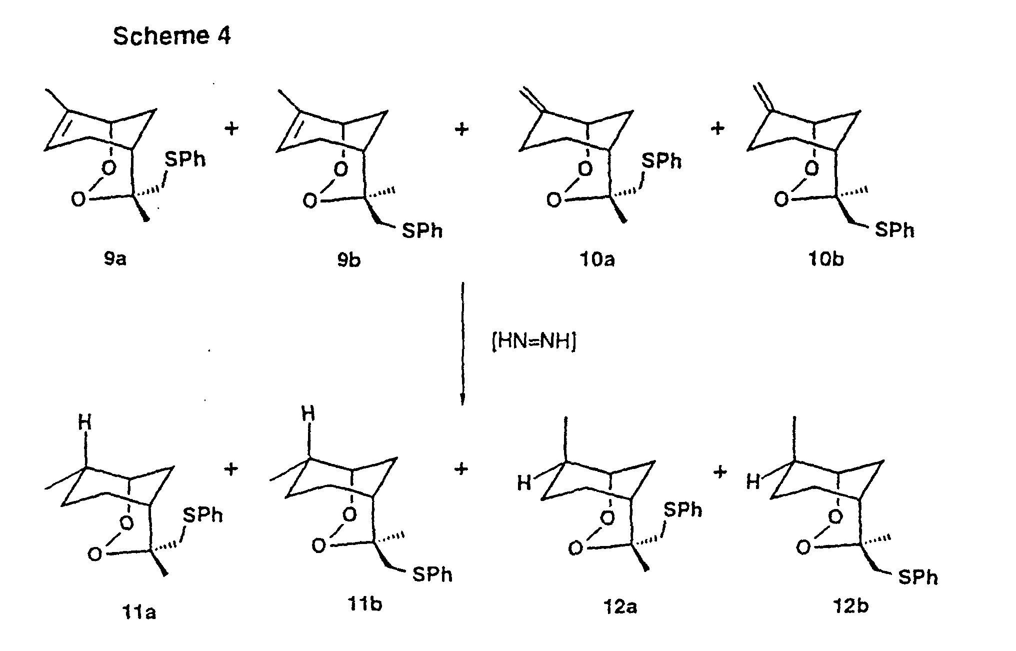

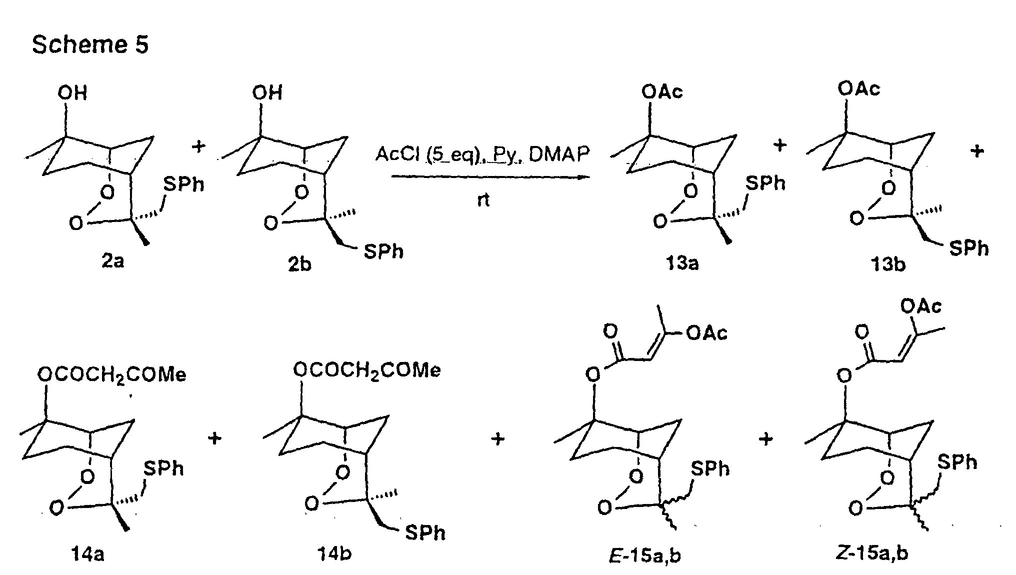

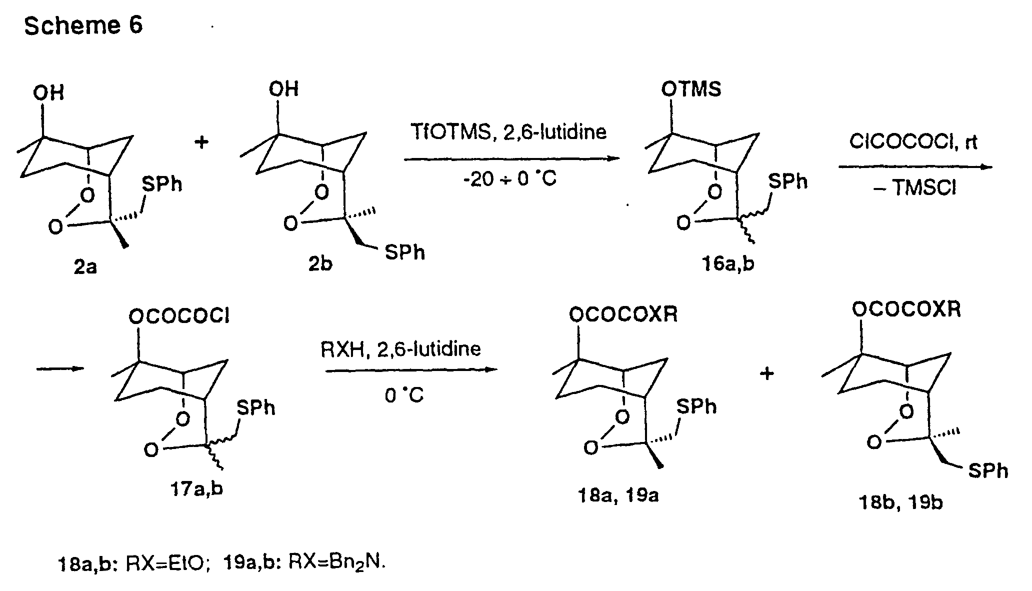

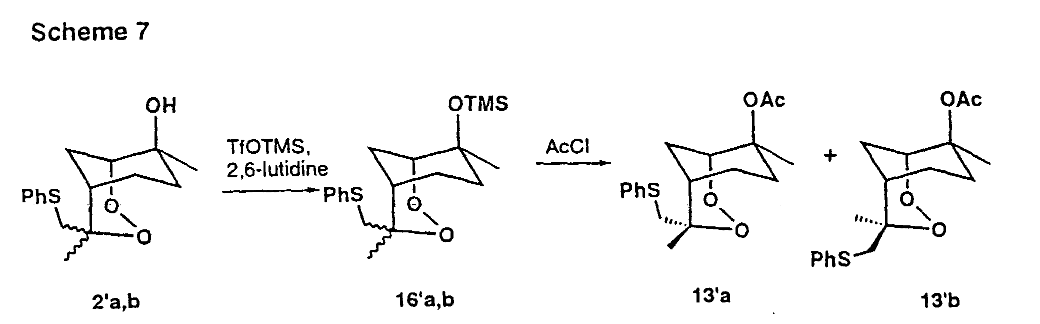

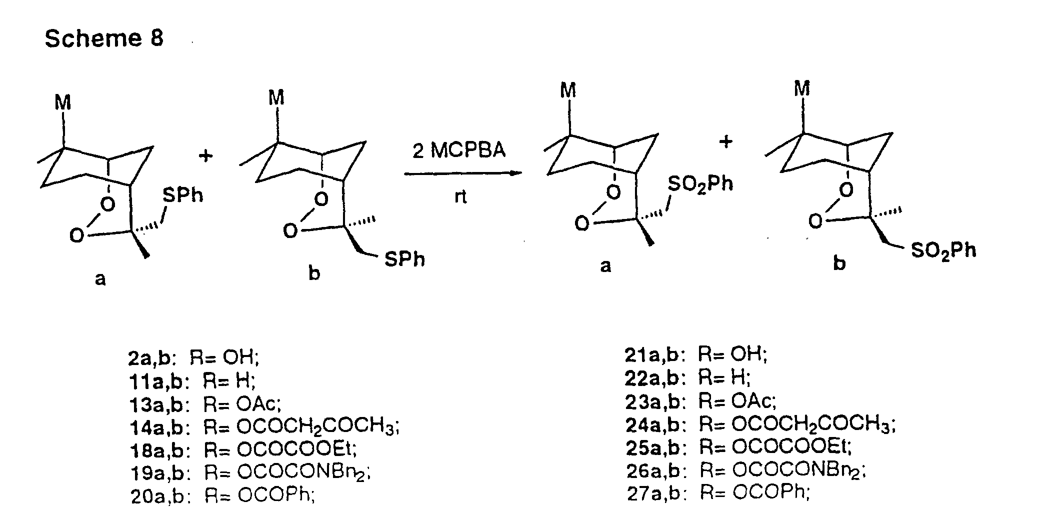

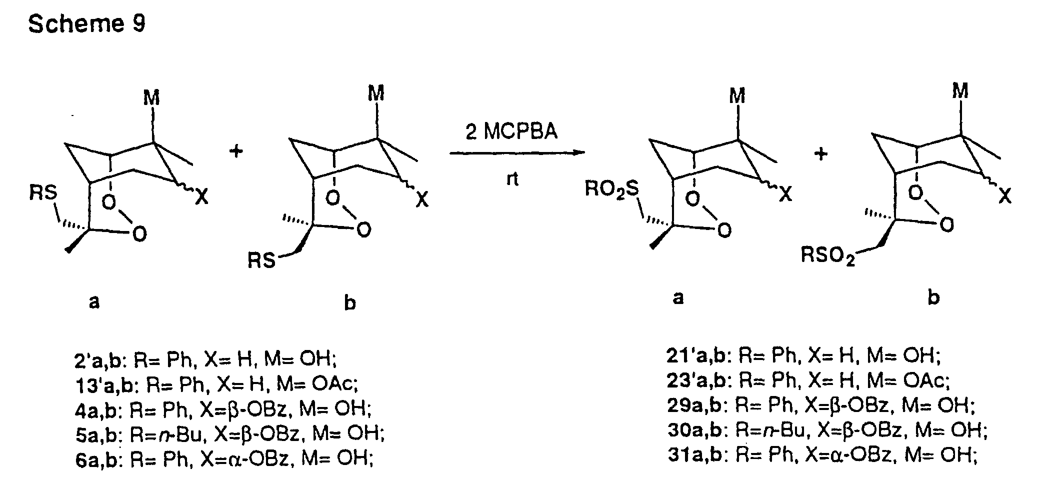

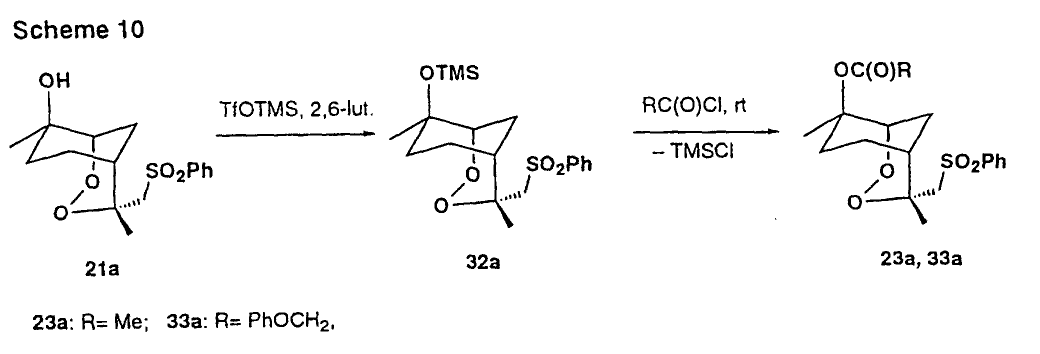

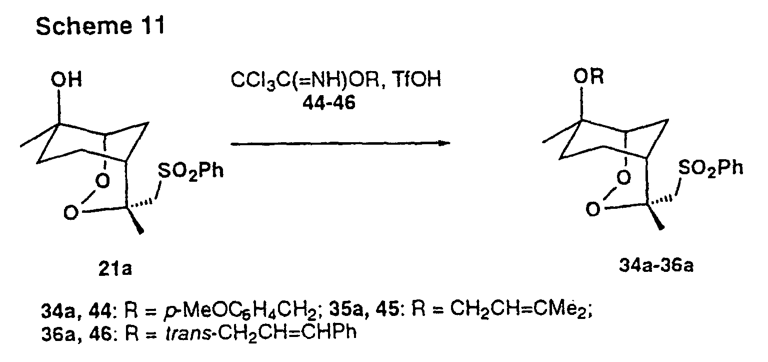

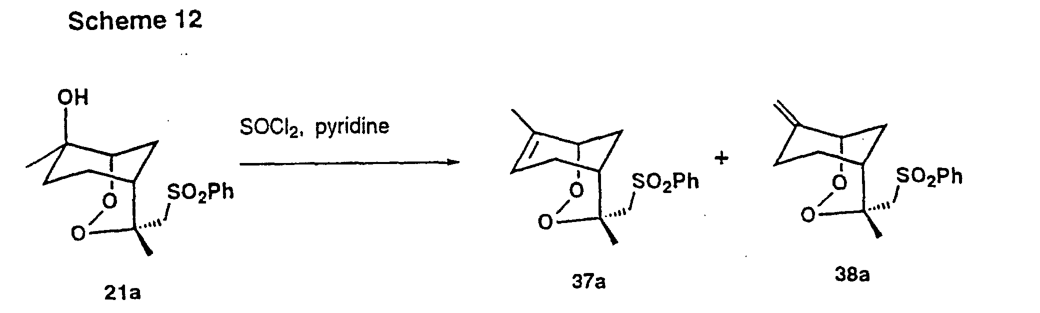

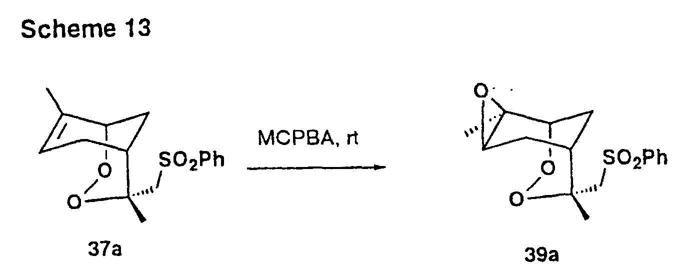

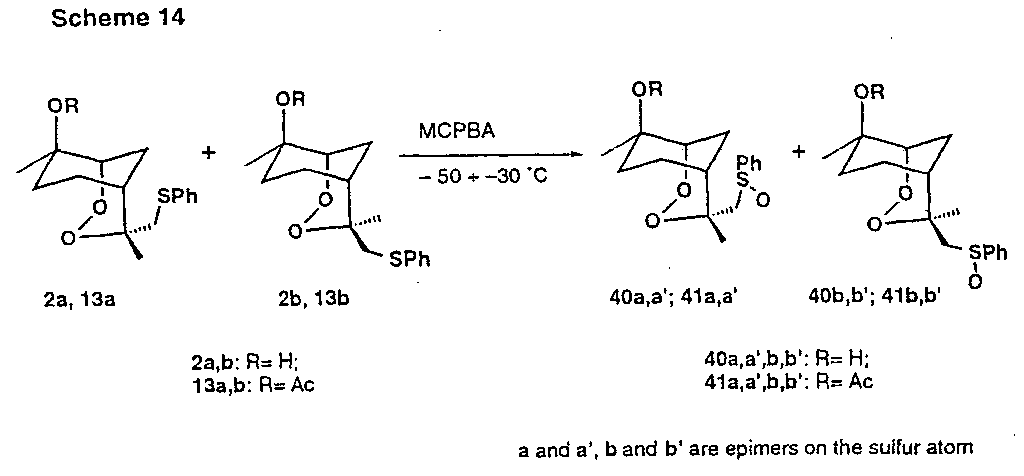

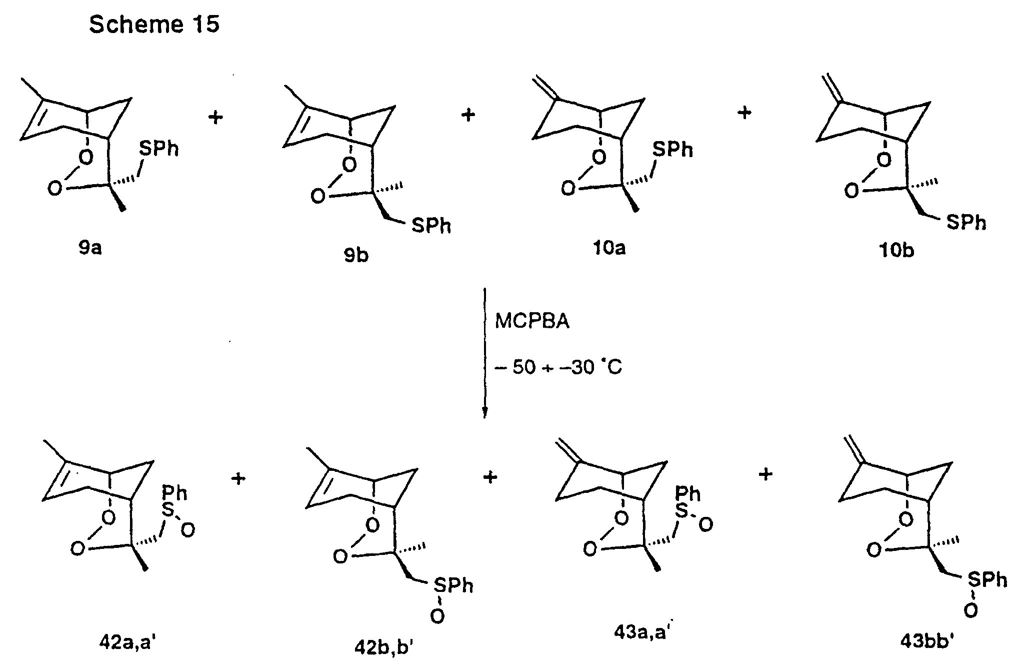

### Example 1: Preparation of (1R,4R,5R,8R)-4,8-dimethyl-4-phenylthiomethyl-2,3-dioxabicyclo [3.3.1]nonan-8-ol (2a) and (1R,4S,5R,8R)-4,8-dimethyl-4-phenylthiomethyl-2,3-dioxabicyclo[3.3.1]nonan-8-ol (2b) (Scheme 1).

To a stirred solution of R-(+)-limonene 1 (6.13 g, 45 mmol) in a mixture of *n*-heptane (500 mL) and benzene (200 mL) was added in one portion di-*tert*-butyl peroxalate (DBPO) (105 mg, 0.45 mmol). Oxygen gas was bubbled through the mixture at rt, with simultaneous addition of a solution of thiophenol (PhSH) (1.653 g, 15 mmol) in benzene (20 mL) (syringe pump) over a period of 12 h. After the addition of PhSH was completed the mixture was exposed to oxygen for an additional 14 h. After addition of CH₂Cl₂ (100 mL) the mixture was cooled to 0-5°C and powdered Ph₃P (3.93 g, 15 mmol) was added in one portion. The mixture was then stirred for 2 h at 0-5°C and for 1 h at rt, solvents were evaporated at rt Flash chromatography (silica gel 60, 230-400 mesh from Merck, gradient elution, EtOAc-hexane, from 1:49 to 3:7) followed by additional flash chromatography (EtOAc-hexane, 3:7) afforded a mixture of (1*R*,4*R*,5*R*,8*R*)-4,8-dimethyl-4-phenylthiomethyl-2,3-dioxabicyclo[3.3.1]nonan-8-ol (**2a**) and (1*R*,4*S*,5*R*,8*R*)-4,8-dimethyl-4-phenylthiomethyl-2,3-dioxabicyclo[3.3.1]nonan-8-ol (**2b**) (**2a/2b** *ca*. 55:45), 2.41 g (8.19 mmol, 54.6% yield), as a colorless or pale yellow oil, R_{f} 0.29 (EtOAc-hexane 3:7). IR (neat): 690, 740, 1015, 1055, 1373, 1440, 1454, 1480, 1584, 2921, 2928, 2964, 3420 cm⁻¹. ¹H NMR (CDCl₃, 400 MHz) (δ): 1.25 (br.s, Me¹¹, 2a), 1.56 (d, ⁴*J*_{11,12} = 0.6 Hz, Me¹¹, 2b), total 3H; 1.39 (s, Me¹⁰, 2b), 1.40 (s, Me¹⁰, 2a), total 3H; 1.58 (m, 1H, Hₑ⁷, 2a + 2b); 1.76-1.85 (m, 2H, Hₑ⁶ + Hₐ⁶ , 2a+ 2b); 1.81 (m, Hₑ⁵ , 2b), 1.91 (dddd, ³*J*₅ₑ₆ₑ = ³*J*₅ₑ₉ₑ = ³*J*₅ₑ₆ₐ = ³*J*₅ₑ₉ₐ = 3.2 Hz, Hₑ⁵ , 2a), total 1H ; 1.98 (ddd, ²*J*₉ₐ₉ₑ = 13.6 Hz, ³*J*₉ₐ₅ₑ = 3.2 Hz, ³*J*₉ₐ₁ₑ = 2.0 Hz, Hₐ⁹, 2a), 2.01 (ddd, ²*J*₉ₐ₉ₑ = 14.2 Hz, ³*J*₉ₐ₅ₑ = 3.2 Hz, ³*J*₉ₐ₁ₑ = 1.9 Hz, Hₐ⁹, 2b), total 1H; 2.10 (ddd, ²*J*₉ₑ₉ₐ = 13.6 Hz, ³*J*₉ₑ₁ₑ = 5.5 Hz, ³*J*₉ₑ₅ₑ = 3.2 Hz, Hₑ⁹, 2a), 2.26 (ddd, ²*J*₉ₑ₉ₐ = 14.2 Hz, *J*₉ₑ₁ₑ = 4.4 Hz,³*J*₉ₑ₅ₑ = 3.0 Hz, Hₑ⁹, **2b**), total 1H; 2.31-2.41 (m, 1H, Hₐ⁷, **2a + 2b**); 2.96 (d, ²*J* = 12.0 Hz, H¹², **2b**), 3.35 (d, ²*J* = 12.8 Hz, H¹², **2a**), total 1H; 3.03 (dd, ²*J* = 12.0 Hz, ⁴*J*_{12,11} = 0.6 Hz, H'¹², **2b**), 3.71 (dd, ²*J* = 12.8 Hz, ⁴*J*_{12,11} = 0.5 Hz, H¹², **2a**), total 1H; 3.68 (m, H¹, **2a**), 3.71 (m, H¹, **2b**), total 1H; 7.17-7.23 (m, 1H, H¹⁶, **2a + 2b**); 7.27-7.33 (m, 2H, H¹⁵, **2a + 2b**); 7.36-7.43 (m, 2H, H¹⁴, **2a + 2b**). ¹³C NMR (CDCl₃, 100 MHz) (δ): Isomer 2a: 21.89 (Me¹¹), 23.58 (C⁶H₂), 24.17 (C⁹H₂), 28.04 (Me¹⁰), 29.23 (C⁵H), 35.51 (C⁷H₂), 40.68 (C¹²H₂), 71.40 (C⁸), 81.57 (C¹H), 83.77 (C⁴), 126.14 (C¹⁶H), 128.87 (2C¹⁴H), 129.63 (2C¹⁵H), 136.84 (C¹³); Isomer 2b: 21.76 (Me¹¹), 23.30 (C⁶H₂), 24.26 (C⁹H₂), 28.04 (Me¹⁰), 30.50 (C⁵H), 35.84 (C⁷H₂), 40.73(C¹²H₂), 71.35 (C⁸), 82.02 (C¹H), 83.83 (C⁴), 126.33 (C¹⁶H), 128.93 (2C¹⁴H), 129.63 (2C¹⁵H), 136.42 (C¹³). *R*-(+)-Limonene **1** (2.73 g, 20.0 mmol, 66.8%) was recovered in the first chromatography.

### Example 2: Preparation of (1S,4S,5S,8S)-4,8-dimethyl-4-phenylthiomethyl-2,3-dioxabicyclo [3.3.1]nonan-8-ol (2'a) and (1S,4R,5S,8S)-4,8-dimethyl-4-phenylthiomethyl-2,3-dioxabicyclo[3.3.1]nonan-8-ol (2'b) (Scheme 2).

The title compounds were obtained in 53.7% yield (8.0 mmol) from *S*(-)-limonene **1'** using the procedure for the preparation of compounds **2a,b** from *R*(+)-limonene. ¹H NMR and IR spectra are consistent with those described for (1*R*,4*R*,5*R*,8*R*)- and (1*R*,4*S*,5*R*, 8*R*)-diastereomers **2a,b.**

### Example 3: Preparation of (1S,4S,5S,7S,8S)-7-benzoyloxy-4,8-dimethyl-4-phenylthiomethyl-2,3-dioxabicyclo[3.3.1]nonan-8-ol (4a) and (1S,4R,5S,7S,8S)-7-benzoyloxy-4,8-dimethyl-4-phenylthio-methyl-2,3-dioxabicyclo[3.3.1]nonan-8-ol (4b) (Scheme 2).

Oxygen was bubbled through a solution of the β-benzoate **3a** (350 mg, 1.36 mmol) and DBPO (4.3 mg, 0.018 mmol) in benzene-n-heptane (20 mL, 1:1) for 10 h with simultaneous addition of a solution of PhSH (50 mg, 0.45 mmol) in heptane (10 mL) (syringe pump). After completion of the thiol addition, the mixture was kept under oxygen for an additional 15 h, diluted with CH₂Cl₂ (10 mL), cooled to 4 °C and Ph₃P (118 mg, 0.45 mmol) was added in 4 portions. The mixture was stirred at 4 °C for 1 h and at rt for an additional 1 h, the solvents were evaporated, and flash chromatography (hexane-EtOAc, 3:1) afforded a mixture (42 mg, 22.3% yield) of (1*S*,4*S*,5*S*,7*S*,8*S*)-7-benzoyloxy-4,8-dimethyl-4-phenylthiomethyl-2,3-dioxabicyclo[3.3.1]nonan-8-ol **4a** (less polar isomer) and (1*S*,4*R*,5*S*,7*S*,8*S*)-7-benzoyloxy-4,8-dimethyl-4-phenylthiomethyl-2,3-dioxabicyclo[3.3.1]nonan-8-ol **4b** (more polar isomer), (**4a/4b** *ca*. 50:50), R_{f} 0.26 (EtOAc-hexane,1:3), colorless semisolid. IR (neat): 3547, 1713,1271, 711 cm⁻¹. Additional flash chromatography afforded a sample of crystalline **4a** (less polar isomer), mp 139-140°C. ¹H NMR (400 MHz, CDCl₃, δ): 1.37 (br.s, 3H, Me¹¹), 1.44 (s, 3H, Me¹⁰), 1.83 (ddd, 1H, ²*J* = 13.1 Hz, ³*J*₆ₐ₇ₐ = 11.6 Hz, ³*J*₆ₐ₅ₑ = 3.3 Hz, Hₐ⁶), 2.08 (m, 2H, Hₐ⁹ + Hₑ⁹), 2.20 (m, 1H, Hₑ⁵), 2.37 (m, 1H, Hₑ⁶), 3.35 (d, 1H, ²*J* = 12.9 Hz, H¹²), 3.73 (br.d, 1H, ²*J* = 12.9 Hz, H'¹²), 3.96 (dd, 1H, ³*J*₁ₑ₉ₑ = ³*J*₁ₑ₉ₐ = 2.4 Hz, Hₑ¹), 5.76 (dd, 1H, ³*J*₇ₐ₆ₐ = 11.6 Hz, ³*J*₇ₐ₆ₑ = 6.3 Hz, Hₐ⁷), 7.20 (m, 1H, H¹⁶), 7.30 (m, 2H, H^{15, 15'}), 7.42 (m, 2H, H^{14, 14'}), 7.48 (m, 2H, H^{meta-benz.}), 7.60 (m, 1H, H^{para-benz.}), 8.03 (m, 1H, H^{ortho-benz.}). ¹³C NMR (100 MHz, CDCl₃, δ): 22.30 (Me¹¹), 23.68 (C⁹H₂), 24.46 (Me¹⁰), 29.23(C⁶H₂), 31.31 (C⁵H), 40.54 (C¹²H₂), 72.81 (C⁸), 74.75 (C⁶H), 82.75 (C¹H), 83.79 (C⁴), 126.43 (C¹⁶H), 128.54 (2CH), 129.03 (2CH), 129.48 (2CH), 129.94 (2CH), 130.07 (C^{1-benz.}), 133.24 (C^{p-benz.}H), 136.56 (C¹³), 165.59 (C = O). More polar isomer **4b**: ¹H NMR (400 MHz, CDCl₃, δ): 1.43 (s, 3H, Me¹⁰), 1.58 (br.s, 3H, Me¹¹), 1.77 (ddd, 1H, ²*J* = 12.8 Hz, ³*J*₆ₐ₇ₐ = 11.2 Hz, ³*J*₆ₐ₅ₑ = 3.4 Hz, Hₐ⁶), 2.07-2.36 (m, 4H, Hₑ⁵+Hₑ⁶+ H_{e,a}⁹), 3.03 (d, 1H, ²*J* = 12.3 Hz, H¹²), 3.24 (br.d, 1H, ²*J* = 12.3 Hz, H'¹²),3.99 (m, 1H, Hₑ¹), 5.78 (dd, 1H, ³*J*₇ₐ₆ₐ = 11.2 Hz, ³*J*₇ₐ₆ₑ = 6.3 Hz, Hₐ⁷), 7.21 (m, 1H, H¹⁶), 7.30 (m, 2H, H^{15, 15'}), 7.42 (m, 2H, H^{14, 14'}), 7.48 (m, 2H, H^{meta-benz.}), 7.59 (m, 1H, H^{para-benz.}), 8.03 (m, 1H, H^{ortho-benz.}), ¹³C NMR (100 MHz, CDCl₃, δ): 21.40(Me¹¹), 23.80 (C⁹H₂), 24.46 (Me¹⁰), 28.99 (C⁶H₂), 32.53 (C⁵H), 40.70 (C¹²H₂), 72.83 (C⁸), 74.81 (C⁷H), 83.23 (C¹H), 83.88 (C⁴), 126.51 (C¹⁶H), 128.52 (2CH), 129.03 (2CH), 129.48 (2CH), 129.90 (2CH), 130.08 (C^{1-benz.}), 133.20 (C^{p-benz.}H), 135.99 (C¹³), 165.52 (C=O).

### Example 4: Preparation of (1S,4S,5S,7S,8S)-7-benzoyloxy-4-n-butylthiomethyl-4,8-dimethyl-2,3-dioxabicyclo[3.3.1]nonan-8-ol (5a) and (1S,4R,5S,7S,8S)-7-benzoyloxy-4-n-butylthiomethyl-4,8-dimethyl-2,3-dioxabicyclo[3.3.1]nonan-8-ol (5b) (Scheme 2).

Oxygen was bubbled through a solution of the β-benzoate 3a (993 mg, 3.87 mmol) and DBPO (23 mg, 0.1 mmol) in hexane-benzene (50 mL, 4:1) with simultaneous addition (syringe pump) during 10 h a solution of *n*-BuSH (180 mg, 2.0 mmol) in hexane (20 mL). After completion of the addition the mixture was kept under oxygen for an additional 14 h, cooled to 5°C, and Ph₃P (526 mg, 2.0 mmol) was added. The reaction mixture was stirred for 2h at 5°C, for additional 1h at rt and then evaporated. Flash chromatography (hexane-EtOAc, 9:1 → 7:3) afforded a mixture of diastereomeric (1*S*,4*S*,5*S*,7*S*,8*S*)-7-benzoyloxy-4-*n*-butylthiomethyl-4,8-dimethyl-2,3-dioxabicyclo[3.3.1]nonan-8-ol **5a** and (1*S*,4*R*,5*S*,7*S*,8*S*)-7-benzoyloxy-4-*n*-butylthiomethyl-4,8-dimethyl-2,3-dioxabicyclo[3.3.1]nonan-8-ol **5b** (59 mg of ∼ 85% purity, *ca*. 6.5% yield; **5a/5b** *ca*. 55:45) as a pale yellow viscous liquid, R_{f} 0.26 (hexane-EtOAc, 4:1). ¹H NMR (400 MHz, CDCl₃, δ): 0.91 (t, ³*J* = 7.3 Hz, Me¹⁶CH₂, **5b**), 0.92 (t, ³*J* = 7.3 Hz, Me¹⁶CH₂, **5a**), total 3H; 1.36 (br.s, Me¹¹, **5a**), 1.58 (br.s, Me¹¹, **5b**), total 3H; 1.42 (m, 2H, MeC¹⁵H₂), 1.43 (s, Me¹⁰, **5b**), 1.44 (s, Me¹⁰, **5a**), total 3H; 1.60 (m, 2H, C¹⁴H₂CH₂S), 1.78 (ddd, ²*J* = 13.3 Hz, ³*J*₆ₐ₇ₐ = 11.6 Hz,³*J*₆ₐ₅ₑ = 3.4 Hz, Hₐ⁶, **5b**), 1.82 (ddd, ²*J* = 13.2 Hz, ³*J*₆ₐ₇ₐ = 11.8 Hz,³*J*₆ₐ₅ₑ = 3.5 Hz, Hₐ⁶, **5a**), 1H total; 1.96-2.52 (m, total 4H, Hₑ⁵ + Hₑ⁶ + H_{e,a}⁹, **5a + 5b**), 2.59 (br.t, ³*J* = 7.3 Hz, CH₂S, **5a**), 2.61 (br.t, ³*J* = 7.3 Hz, C¹³H₂S, **5b**), total 2H; 2.60 (br.d, ²*J* = 12.2 Hz, H¹², **5b**), 2.80 (br.d, ²*J* = 12.2 Hz, H'¹², **5b**), 2.96 (br.d, ²*J* = 13.2 Hz, H¹², **5a**), 3.20 (br.d, ²*J* = 13.2 Hz, H'¹², **5a**), total 2H; 3.97 (br.s, 1H, Hₑ¹, **5a + 5b**), 5.76 (m, 1H, Hₐ⁷, **5a + 5b**), 7.46 (m, 2H, H^{m-benz.}, **5a + 5b)**, 7.59 (m, 1H, H^{p-benz.}, **5a + 5b**), 8.03 (m, 2H, H^{o-benz.}, **5a + 5b**); ¹³C NMR (100 MHz, CDCl₃, δ): Isomer 5a: 13.67 (Me¹⁶), 21.89 (C¹⁵H₂), 22.09 (Me¹¹), 23.79 (C⁹H₂), 24.44 (Me¹⁰), 29.33 (C⁶H₂), 31.58 (C⁵H), 31.82 (C¹⁴H₂), 33.47 (C¹³H₂),38.16 (C¹²H₂), 72.81 (C⁸), 74.79 (C⁷H), 82.73 (C¹H), 83.81 (C⁴), 128.50 (2C^{m-benz.}H), 129.46 (2C^{o-benz.}H), 130.05 (C^{1-benz.}), 133.20 (C^{p-benz.}H), 165.58 (C=O) ; Isomer **5b**: 13.62 (Me¹⁶), 20.66 (Me¹¹), 21.85 (C¹⁵H₂), 23.89 (C⁹H₂), 24.44 (Me¹⁰), 28.99 (C⁶H₂), 31.54 (C¹⁴H₂), 32.59 (C⁵H), 33.67 (C¹³H₂),38.88 (C¹²H₂), 72.80 (C⁸), 74.96 (C⁷H), 83.15 (C¹H), 83.92 (C⁴), 128.50 (2C^{m-benz.}H), 129.46 (2C^{o-benz.}H), 130.09 (C^{l-benz.}), 133.17 (C^{p-benz.}H), 165.58 (C=O).

### Example 5: Preparation of (1S,4S,5S,7R,8S)-7-benzoyloxy-4,8-dimethyl-4-phenylthiomethyl-2,3-dioxabicyclo[3.3.1]nonan-8-ol (6a) and (1S,4R,5S,7R,8S)-7-benzoyloxy-4,8-dimethyl-4-phenylthiomethyl-2,3-dioxabicyclo[3.3.1]nonan-8-ol (6b) (Scheme 2).

Oxygen was bubbled through a solution of the α-benzoate **3b** (690 mg, 2.70 mmol) and DBPO (8.5 mg, 0.036 mmol) in hexane-benzene (50 mL, 7:3) with simultaneous addition (syringe pump) of a solution of PhSH (99 mg, 2.0 mmol) in hexane (20 mL) during 10 h. After completion of the thiol addition, the mixture was kept under oxygen for an additional 14 h, cooled to 4°C, and Ph₃P (236 mg, 0.90 mmol) was added. The reaction mixture was stirred for 2h at 5°C and at rt for additional 1h. The solvents were then evaporated, and flash chromatography (hexane-EtOAc, 9:1 → 1:1) afforded a mixture of diastereomeric (1*S*,4*S*,5*S*,7*R*,8*S*)-7-benzoyloxy-4,8-dimethyl-4-phenylthiomethyl-2,3-dioxabicyclo-[3.3.1]nonan-8-ol 6a and (1*S*,4*R*,5*S*,7*R*,8*S*)-7-benzoyloxy-4,8-dimethyl-4-phenylthiomethyl-2,3-dioxabicyclo[3.3.1]nonan-8-ol **6b** (24 mg of *ca*. 94% purity, 6% yield, 6a/6b *ca*. 65:35), R_{f} 0.42 (EtOAc-hexane, 35:65) as a pale yellow oil. ¹H NMR (400 MHz, CDCl₃, δ): 1.23 (br.s, Me¹¹, **6a**), 1.59 (br.s, Me¹¹, **6b**), total 3H; 1.41 (s, Me¹⁰, **6b**), 1.42 (s, Me¹⁰, **6a**), total 3H; 1.91-2.37 (m, total 5H, Hₑ⁵ + H_{a,e}⁶ + H_{a,e}⁹, **6a+6b**); 3.00 (d, ²*J* = 12.1 Hz, H¹², **6b**), 3.14 (br.d, ²*J* = 12.1 Hz, H'¹², **6b**), 3.39 (d, ²*J* = 12.8 Hz, H¹², **6a**), 3.80(br.d, ²*J* = 12.8 Hz, H'¹², **6a**), total 2H; 3.82 (m, Hₑ¹, **6a**), 3.88 (dd, ³*J*₁ₑ₉ₑ = 4.1 Hz, ³*J*₁ₑ₉ₐ = 1.5 Hz, Hₑ¹, 6b), total 1H; 5.26 (dd, 1H, ³*J*₇ₑ₆ₑ = 5.0 Hz, ³*J*₇ₑ₆ₐ = 1.7 Hz, Hₑ⁷, **6b**), 5.27 (dd, 1H, ³*J*₇ₑ₆ₑ = 5.0 Hz, ³*J*₇ₑ₆ₐ = 1.5 Hz, Hₑ⁷, **6a**), total 1H; 7.09 (m, 1H), 7.22 (m, 6b), 7.29 (m, 6a), total 2H; 7.41-7.47 (m,), 7.54-7.58 (m,), total 5H; 8.13 (m, 2H). ¹³C NMR (100 MHz, CDCl₃, δ): Isomer **6a**: 22.30 (Me¹¹), 23.42 (C⁹H₂), 23.47 (Me¹⁰), 28.81 (C⁶H₂), 29.36 (C⁵H), 40.78 (C¹²H₂), 73.83 (C⁸), 74.36 (C⁷H), 80.85 (C¹H), 82.74 (C⁴), 126.36 (C¹⁶H), 128.48 (2CH), 129.00 (2CH), 129.81 (2CH), 129.90 (2CH), 130.15 (C^{1-benz.}), 133.11 (C^{p-benz.}H), 136.71 (C¹³), 166.33 (C=O) ; Isomer **6b**: 21.68 (Me¹¹), 23.35 (Me¹⁰), 23.56 (C⁹H₂), 28.68 (C⁶H₂), 30.86 (C⁵H), 40.37 (C¹²H₂), 73.68 (C⁸), 74.51 (C⁷H), 81.38 (C¹H), 82.83 (C⁴), 126.12 (C¹⁶H), 128.52 (2CH), 128.88 (2CH), 129.06 (2CH), 129.81 (C^{1-benz.}), 129.90 (2CH), 133.18 (C^{p-benz.}H), 136.25 (C¹³), 166.25 (C=O).

### Example 6: Preparation of (1S,4S,5S,7R,8S)-4,8-dimethyl-4-phenylthiomethyl-2,3-dioxabicyclo [3.3.1]nonan-7,8-diol monohydrate (7a) (Scheme 2).

Oxygen was bubbled through a solution of α-carveol **3c** (2.284 g, 15 mmol) and DBPO (47 mg, 0.2 mmol) in benzene-*n*-hexane (250 mL, 1:1) with simultaneous addition (syringe pump) of a solution of PhSH (550 mg, 5.0 mmol) in hexane (20 mL) during 12 h. After completion of the thiol addition, the mixture was kept under oxygen for an additional 12h, cooled to 5°C and Ph₃P (1.31 g, 5.0 mmol) was added in 3 portions. The mixture was stirred at 5°C for 2 h and for an additional 2 hr at rt , the solvents were evaporated, and flash chromatography (hexane-EtOAc, 3:1 → 1:1) afforded (1*S*,4*S*,5*S*,7*R*,8*S*)-4,8-dimethyl-4-phenylthiomethyl-2,3-dioxabicyclo[3.3.1]nonan-7,8-diol monohydrate **7a** (194 mg, 11.8% yield) as colorless needles, mp 108-109°C (hexane-EtOAc, 5:1), R_{f} 0.20 (EtOAc-hexane, 50:50). ¹H NMR (400 MHz, CDCl₃, δ): 1.36 (br.s, 3H, Me¹¹), 1.45 (s, 3H, Me¹⁰), 1.96-2.06 (m, total 3H, Hₑ⁵ + H_{a,e}⁹), 2.09 (ddd, 1H, ²*J* = 16.0 Hz, ³*J*₆ₐ₅ₑ = 3.7 Hz, ³*J*₆ₐ₇ₑ = 1.4 Hz, Hₐ⁶), 2.18 (ddd, 1H, ²*J* = 16.0 Hz, ³*J*₆ₑ₇ₑ = 5.5 Hz, ³*J*₆ₑ₅ₑ = 4.4 Hz Hₑ⁶), 3.37 (d, 1H, ²*J* = 12.9 Hz, H¹²), 3.55 (br.dd, 1H, ³*J*_{7e,OH} = 13.2 Hz, ³*J*₇ₑ₆ₑ = 5.5 Hz, Hₑ⁷), 3.76 (dd, 1H, ²*J* = 12.9 Hz, ⁴*J*_{12',11}= 0.6 Hz, H'¹²), 3.76 (m, 1H, Hₑ¹), 4.09 (d, 1H, ³*J*_{HO,7e} = 13.2 Hz, OH, exch. with CD₃COOD), 7.22 (m, 1H, H¹⁶), 7.32 (m, 2H, 2H¹⁵),7.42 (m, 2H, 2H¹⁴). ¹³C NMR (100 MHz, CDCl₃, δ): 22.22 (Me¹¹), 22.91 (C⁹H₂), 24.57 (Me¹⁰), 29.95 (C⁵H), 30.92 (C⁶H₂), 40.55 (C¹²H₂), 72.98 (C⁷H), 74.21 (C⁸), 82.19 (C¹H), 83.86 (C⁴), 126.56 (C¹⁶H), 129.03 (2C¹⁵H), 130.07 (2C¹⁴H), 136.35 (C¹³). Anal. Calcd for C₁₆H₂₂O₄S x H₂O : C, 58.51; H 7.36; S, 9.76. Found: C, 58.48; H 7.34; S, 9.27.

### Example 7: Preparation of (1R,4R,5R)-4,8-dimethyl-4-phenylthiomethyl-2,3-dioxabicyclo[3.3.1]non-7-ene (9a), (1R,4S,5R)-4,8-dimethyl-4-phenylthiomethyl-2,3-dioxabicyclo[3.3.1]non-7-ene (9b), (1R,4R,5R)-4-methyl-8-methylidene-4-phenylthiomethyl-2,3-dioxabicyclo[3.3.1]nonane (10a) and (1R,4S,5R)-4-methyl-8-methylidene-4-phenylthiomethyl-2,3-dioxabicyclo [3.3.1]nonane (10b) (Scheme 3).

To a mixture of SOCl₂ (1.90 g, 16.0 mmol) and pyridine (3.165 g, 40.0 mmol) in CH₂Cl₂ (150 mL) at -30 ö -40°C was added during 1.5 h a solution of hydroxysulfides **2a,b** (1.10 g, 3.74 mmol, ratio **2a/2b** *ca*. 55:45) in CH₂Cl₂ (30 mL). The temperature raised to 25°C and the mixture was stirred for additional 4 h. The resulting mixture was poured into ice-cold 0.1 M HCl (100 mL), extracted with hexane-EtOAc (9:1, 2 x 300 mL), the organic layer was washed with saturated NaHCO₃ (2 x 80 mL), dried(Na₂SO₄ + NaHCO₃) and evaporated. The residue was purified by flash chromatography (hexane-EtOAc, 95:5) to give a mixture of unsaturated sulfides **9a,b** and **10a,b** (798 mg, total 80%, consisting of **9a**, *ca*. 47%;, 9b, 39%; **10a**, 8%; and **10b**, 6% according the integration of the C¹H peaks in 400 MHz ¹H NMR spectrum). ¹H NMR (400 MHz, CDCl₃, δ): 1.20 (d, ⁴*J* = 0.7 Hz, Me¹¹ **9a**), 1.29 (d, ⁴*J* = 0.6 Hz, Me¹¹ **10a**), 1.57 (d, ⁴*J* = 0.5 Hz, Me¹¹ **10b**), 1.58 (d, ⁴*J* = 0.8 Hz, Me¹¹ **9b**), total 3H; 1.55 (ddd, ²*J* = 13.1 Hz, ³*J*₉ₐ₅ₑ = 3.0 Hz, ³*J*₉ₐ₁ₑ = 2.0 Hz, Hₐ⁹ **9a**), 1.63 (dddd, ²*J* = 13.0 Hz, ³*J*₉ₐ₅ₑ = 2.9 Hz, ³*J*₉ₐ₁ₑ = 2.4 Hz, ⁴*J* = 0.5 Hz, Hₐ⁹ **9b**), total *ca*. 1H; 1.79-1.81 (m, *ca*. 3H, Me¹⁰ **9 a,b**), 1.93 (m, Hₑ⁵ **9b**), 2.04 (m, Hₑ⁵ **9a**), total *ca*. 1H; 2.15-2.37 (m), 2.48 (dddd, ²*J* = 13.0 Hz, ³*J*₉ₑ₅ₑ = ³*J*₉ₑ₁ₑ = 3.6 Hz, ⁴*J*₉ₑ₆ₑ = 1.6 Hz, Hₑ⁹ **9b**), total 3H; 2.90 (d, ²*J* = 11.8 Hz, H¹² 9b) and 2.99 (dd, ²*J* = 11.8 Hz, ⁴*J*_{12,11} = 0.8 Hz, H'¹² **9b**), 3.00 (d, ²*J* = 12.0 Hz, H¹² **10b**) and 3.10 (dd, ²*J* = 12.0 Hz, ⁴*J*_{12,11} = 0.5 Hz, H'¹² **10b**), 3.30 (d, ²*J* = 12.2 Hz, H¹² **10a**), 3.33 (d, ²*J* = 12.7 Hz, H¹² **9a**) and 3.79 (dd, ²*J* = 12.7 Hz,⁴*J*_{12,11} = 0.7 Hz, H¹² **9a**), total 2H; 4.10 (m, ³*J*₁ₑ₉ₑ = 3.6 Hz, ³*J*₁ₑ₉ₐ = 2.0 Hz, Hₑ¹, **9a**), 4.13 (br.dd, ³*J*₁ₑ₉ₑ = 3.6 Hz, ³*J*₁ₑ₉ₐ = 2.4 Hz, Hₑ¹ **9b**), 4.34 (br.dd, ³*J*₁ₑ₉ₑ = 4.0 Hz, ³*J*₁ₑ₉ₐ = 1.4 Hz, Hₑ¹, **10a**), 4.40 (br.dd, ³*J*₁ₑ₉ₑ = 4.3 Hz, ³*J*₁ₑ₉ₐ = 1.7 Hz, Hₑ¹ **10b**), total 1H; 4.89-4.93 (m, HH'C¹⁰ = **10a,b**), 5.73 (m, H⁷ **9b**), 5.75 (m, H⁷ **9a**), total *ca*. 1H; 7.18-7.46 (m, 5H).

By an additional preparative RP HPLC (column LiChrosorb® RP-8 (7 µm) 250-25; eluent-MeCN-H₂O, 60:40) single diastereomer (1*R*,4*R*,5*R*)-4,8-dimethyl-4-phenylthiomethyl-2,3-dioxabicyclo[3.3.1]non-7-ene **9a** (*ca*. 97% purity) was isolated: a colorless waxy substance, mp 60-63°C. ¹H NMR (400 MHz, CDCl₃, δ): 1.20 (d, 3H, ⁴*J* = 0.6 Hz, Me¹¹), 1.55 (ddd, 1H, ²*J* = 13.1 Hz, ³*J*₉ₐ₅ₑ = 3.0 Hz, ³*J*₉ₐ₁ₑ = 2.0 Hz, Hₐ⁹), 1.80 (ddd, 3H, ⁵*J*_{10,6a} = 2.7 Hz, ⁵*J*_{10,6e} = ⁴*J*_{10,7} = 1.6 Hz, Me¹⁰⁾, 2.04 (m, 1H, Hₑ⁵), 2.21 (dddq, 1H, ²*J* = 19.0 Hz, ³*J*₆ₐ₇ = 5.4 Hz, ³*J*₆ₐ₅ₑ = ⁵*J*₆ₐ₁₀ = 2.7 Hz, Hₐ⁶), 2.30 (dddd, 1H, ²*J* = 13.1 Hz, ³*J*₉ₑ₅ₑ = ³*J*₉ₑ₁ₑ = 3.6 Hz, ⁴*J*₉ₑ₆ₑ = 1.6 Hz, Hₑ⁹), 2.33 (dddq, 1H, ²*J* = 19.0 Hz, ³*J*₆ₑ₇ = 6.4 Hz, ³*J*₆ₑ₅ₑ = ⁵*J*₆ₑ₁₀ = 1.6 Hz, Hₑ⁶), 3.34 (d, 1H, ²*J* = 12.7 Hz, H¹²), 3.79 (br.dd, ²*J* = 12.7 Hz, ⁴*J*_{12',11} = 0.7 Hz, H¹²), 4.10 (m, 1H, ³*J*₁ₑ₉ₑ = 3.6 Hz, ³*J*₁ₑ₉ₐ = 2.0 Hz, Hₑ¹), 5.76 (m, 1H, H⁷), 7.19 (m, 1H, H¹⁶), 7.29 (m, 2H, H^{15, 15'}), 7.42 (m, 2H, H^{14,} ^{14'}). ¹³C NMR (100 MHz, CDCl₃, δ): 21.17 (Me¹⁰), 22.72 (Me¹¹), 26.38 (C⁹H₂), 27.56 (C⁶H₂), 28.52 (C⁵H), 40.73 (C¹²H₂), 76.21 (C¹H), 83.37 (C⁴), 126.15 (C¹⁶H), 126.82 (C⁷H), 128.90 (2C¹⁵H), 129.69 (2C¹⁴H), 131.26(C⁸ = ), 136.96 (C¹³).

### Example 8: Preparation of (1R,4R,5R,8S)-4,8-dimethyl-4-phenylthiomethyl-2,3-dioxabicyclo [3.3.1]nonane (11a), (1R,4S,5R,8S)-4,8-dimethyl-4-phenylthiomethyl-2,3-dioxabicyclo[3.3.1]nonane (11b), (1R,4R,5R,8R)-4,8-dimethyl-4-phenylthiomethyl-2,3-dioxabicyclo[3.3.1]nonane (12a) and (1R,4S,5R,8R)-4,8-dimethyl-4-phenylthiomethyl-2,3-dioxabicyclo[3.3.1]nonane (12b) (Scheme 4).

To a mixture of unsaturated sulfides **9a,b** and **10a,b** (total 120 mg, 0.43 mmol; ratio **9a : 9b : 10a : 10b** *ca*. 54 : 34: 6.5 : 5.5) and potassium azodicarboxilate (1.00 g, 5.15 mmol) in MeOH (3 mL) and CH₂Cl₂ (2 mL) at 0°C for 45 min was added a solution of AcOH (620 mg, 10.32 mmol) in CH₂Cl₂ (2 mL). Temperature was slowly raised to 25°C (2 h) and the reaction mixture was stirred for an additional 48 h. The mixture was diluted with ether (50 mL), filtered through Celite® and evaporated. The residue was separated by several consecutive MPLC (hexane-EtOAc, 98:2) to give starting material **9a,b** (70 mg, 0.25 mmol; **9a/9b** *ca.* 61: 39), sulfides **12a** (4 mg), **12b** (3 mg), and two fractions consisted of mixtures of **11a,b** with different ratio of isomers : (4 mg, **11a/11b** 83:17) and (19 mg, **11a/11b** 53:47). **11a,b**: ¹H NMR (CDCl₃, 400 MHz) (δ): 1.09 (d, ³*J* = 6.7 Hz, Me¹⁰, **11b**), 1.10 (d, ³*J* = 6.7 Hz, Me¹⁰, **11a**), total 3H; 1.24 (br.s, Me¹¹, **11a**), 1.55 (d, ⁴*J*_{11,12} = 0.7 Hz, Me¹¹, **11b**), total 3H; 1.41 (ddd, ²*J* = 13.3 Hz, ³*J*₉ₐ₅ₑ = 3.0 Hz, ³*J*₉ₐ₁ₑ = 1.7 Hz, Hₐ⁹ **11a**), 1.48 (ddd, ²*J* = 13.1 Hz, ³*J*₉ₐ₅ₑ = 3.2 Hz, ³*J*₉ₐ₁ₑ = 1.7 Hz, Hₐ⁹ **11b**), total 1H; 1.58-1.69 (m, 2H, Hₑ⁶ + Hₐ⁶, **11a+11b**); 1.75-1.85 (m, 1H, Hₐ⁸, **11a+11b**); 1.90-1.94 (m, 1H, Hₑ⁵, **11a+11b**); 1.94-2.05 (m, 2H, Hₑ⁷ + Hₐ⁷, *s***11a+11b**); 2.28 (dddd, ²*J*₉ₑ₉ₐ = 13.3 Hz, ³*J*₉ₑ₁ₑ = ³*J*₉ₑ₅ₑ = 4.0 Hz, ⁴*J*₉ₑ₆ₑ = 2.6 Hz, Hₑ⁹, **11a**), 2.45 (dddd, ²*J*₉ₑ₉ₐ = 13.1 Hz, ³*J*₉ₑ₁ₑ = ³*J*₉ₑ₅ₑ = 3.8 Hz, ⁴*J*₉ₑ₆ₑ = 2.9 Hz, Hₑ⁹, **11b**), total 1H; 2.94 (d, ²*J* = 11.9 Hz, H¹², **11b**) and 3.05 (dd, ²*J* = 11.9 Hz, ⁴*J*_{12,11} = 0.7 Hz, H'¹², **11b**), 3.31 (d, ²*J* = 12.7 Hz, H¹², **11a**), and 3.73 (dd, ²*J* = 12.7 Hz,⁴*J*_{12,11} = 0.7 Hz, H¹², **11a**), total 2H; 3.83 (m, ³*J*₁ₑ₉ₑ = 4.0 Hz, ³*J*₁ₑ₉ₐ = 1.7 Hz, Hₑ¹, **11a**), 3.86 (m, ³*J*₁ₑ₉ₑ = 3.8 Hz, ³*J*₁ₑ₉ₐ = 1.7 Hz, Hₑ¹, **11b**), total 1H; 7.17-7.43 (m, 5H, **11a** + **11b**). Isomer **11a**: ¹³C NMR (CDCl₃, 100 MHz) (δ): 18.56 (Me¹⁰), 22.05 (Me¹¹), 27.06 (CH₂), 29.19 (C⁵H), 29.56 (CH₂), 29.59 (CH₂), 35.62 (C⁸H), 40.91 (C¹²H₂), 79.23 (C¹H), 83.36 (C⁴), 126.07(C¹⁶H), 128.90 (2C¹⁵H), 129.59 (2C¹⁴H), 136.90(C¹³). Isomer 12a: ¹H NMR (CDCl₃, 400 MHz) (δ): 1.02 (d, 3H, ³*J* = 7.4 Hz, Me¹⁰), 1.25 (d, 3H, ⁴*J*_{11,12} = 0.6 Hz, Me¹¹), 1.29 (br.dd, 1H, ²*J* = 14.0 Hz, ³*J*₇ₑ₆ₑ = 5.8 Hz, Hₑ⁷), 1.62 (ddd, 1H, ²*J* = 13.6 Hz, ³*J*₉ₐ₅ₑ = 3.1 Hz, ³*J*₉ₐ₁ₑ = 1.8 Hz, Hₐ⁹), 1.74 (dddd, ²*J* = ³*J*₆ₐ₇ₐ = 14.0 Hz, ³*J*₆ₐ₅ₑ = 6.2 Hz, ³*J*₆ₐ₇ₑ = 3.4 Hz, Hₐ⁶), 1.84 (m, 1H, ²*J* = 14.0 Hz, Hₑ⁶), 1.87 (m, 1H, Hₑ⁵); 2.02 (dddd, 1H, ²*J*₉ₑ₉ₐ = 13.6 Hz, ³*J*₉ₑ₅ₑ = 6.2 Hz, ³*J*₉ₑ₁ₑ = 3.6 Hz, ⁴*J*₉ₑ₆ₑ = 1.3 Hz, Hₑ⁹), 2.39 (br.dq, 1H, ³*J*_{8e,10} = 7.4 Hz, ³*J*₈ₑ₇ₐ = 7.2Hz, Hₑ⁸), 2.55 (dddd, 1H, ²*J* = ³*J*₇ₐ₆ₐ = 14.0 Hz, ³*J*₇ₐ₈ₑ = ³*J*₇ₐ₆ₑ = 7.2 Hz, Hₐ⁷),3.29 (d, 1H, ²*J* = 12.8 Hz, H¹²), 3.75 (br.dd, ²*J* = 12.8 Hz,⁴*J*_{12',11} = 0.7 Hz, H¹²), 3.79 (m, 1H, ³*J*₁ₑ₉ₑ = 3.6 Hz, ³*J*₁ₑ₉ₐ = ³*J*₁ₑ₈ₑ = 1.8 Hz, Hₑ¹), 7.18 (m, 1H, H¹⁶), 7.28 (m, 2H, H^{15, 15'}), 7.41 (m, 2H, H^{14, 14'}); ¹³C NMR (CDCl₃, 100 MHz) (δ): 19.18 (Me¹⁰), 22.06 (Me¹¹), 23.38 (C⁶H₂), 24.02 (C⁹H₂), 27.17 (C⁷H₂), 29.61 (C⁵H), 32.98 (C⁸H), 40.83 (C¹²H₂), 80.32 (C¹H), 83.66 (C⁴), 126.11(C¹⁶H), 128.90 (2C¹⁵H), 129.66 (2C¹⁴H), 136.92(C¹³). Isomer **12b**: ¹H NMR (CDCl₃, 400 MHz) (δ): 1.025 (d, 3H, ³*J* = 7.4 Hz, Me¹⁰), 1.29 (br.dd, 1H, ²*J* = 13.6 Hz, ³*J*₇ₑ₆ₑ = 6.0 Hz, Hₑ⁷), 1.54 (d, 3H, ⁴*J*_{11,12} = 0.7 Hz, Me¹¹), 1.64-1.82 (m, 4H, Hₑ⁵ + Hₑ⁶ + Hₐ⁶ + Hₐ⁹), 2.20 (dddd, 1H, ²*J*₉ₑ₉ₐ = 13.4 Hz, ³*J*₉ₑ₅ₑ = 6.6 Hz, ³*J*₉ₑ₁ₑ = 3.9 Hz, ⁴*J*₉ₑ₆ₑ = 1.3 Hz, Hₑ⁹), 2.37 (br.dq, 1H, ³*J*_{8e,10} = 7.4 Hz, ³*J*₈ₑ₇ₐ = 6.8 Hz, Hₑ⁸), 2.53 (dddd, 1H, ²*J* = ³*J*₇ₐ₆ₐ = 13.6 Hz, ³*J*₇ₐ₈ₑ = ³*J*₇ₐ₆ₑ = 6.8 Hz, Hₐ⁷), 2.97 (d, 1H, ²*J* = 12.0 Hz, H¹²), 3.09 (br.dd, ²*J* = 12.0 Hz,⁴*J*_{12',11} = 0.7 Hz, H¹²), 3.84 (ddd, 1H, ³*J*₁ₑ₉ₑ = 3.9 Hz, ³*J*₁ₑ₉ₐ = ³*J*₁ₑ₈ₑ = 1.8 Hz, Hₑ¹), 7.19 (m, 1H, H¹⁶), 7.28 (m, 2H, H^{15, 15'}), 7.37 (m, 2H, H^{14, 14'}). ¹³C NMR (CDCl₃, 100 MHz) (δ): 19.02 (Me¹⁰), 21.96 (Me¹¹), 23.05 (C⁶H₂), 24.05 (C⁹H₂), 27.51 (C⁷H₂), 31.14 (C⁵H), 32.89 (C⁸H), 40.78 (C¹²H₂), 80.76 (C¹H), 83.65 (C⁴), 126.29(C¹⁶H), 128.97 (2C¹⁵H), 129.62 (2C¹⁴H), 136.86(C¹³).

### Example 9: Synthesis of (1R,4R,5R,8R)-8-acetoxy-4,8-dimethyl-4-phenylthiomethyl-2,3-dioxabicyclo[3.3.1]nonane (13a), (1R,4S,5R,8R)-8-acetoxy-4,8-dimethyl-4-phenylthio-methyl-2,3-dioxabicyclo[3.3.1]nonane (13b), (1R,4R,5R,8R)-8-acetylacetoxy-4,8-dimethyl-4-phenylthiomethyl-2,3-dioxabicyclo[3.3.1]nonane (14a) and (1R,4S,5R,8R)-8-acetylacetoxy-4,8-dimethyl-4-phenylthiomethyl-2,3-dioxabicyclo[3.3.1]nonane (14b) (Scheme 5).

To a solution of sulfides **2a** and **2b** (1.15 g, 3.91 mmol, **2a/2b** 55:45), pyridine (1.54 g, 19.5 mmol) and DMAP (49 mg, 0.4 mmol) in dry CH₂Cl₂ (30 mL) at 0°C was added a solution of AcCl (1.22 g, 15.6 mmol) in CH₂Cl₂ (5 mL). The mixture was stirred for 1 h at 0°C and for 12 h at rt The dark reaction mixture was poured into water (100 mL), extracted with hexane-EtOAc (7:3 mixture, 3 x 100 mL), dried (Na₂SO₄ + NaHCO₃) and evaporated. The residue was separated by flash chromatography (gradient elution, hexane-EtOAc, 9:1 → 7:3) to give: (i) unreacted hydroxysulfides **2a** and **2b** (292 mg, 0.99 mmol, 25.4%); (ii) a mixture of diastereomers (1*R*,4*R*,5*R*,8*R*)-8-acetoxy-4,8-dimethyl-4-phenylthiomethyl-2,3-dioxabicyclo[3.3.1]nonane **13a** and (1*R*,4*S*,5*R*,8*R*)-8-acetoxy-4,8-dimethyl-4-phenylthiomethyl-2,3-dioxabicyclo[3.3.1]nonane **13b** (518 mg, 1.54 mmol, 52.9% yield, **13a/13b** *ca*. 55:45) as a colorless viscous liquid, R_{f} 0.67 (hexane-EtOAc 7:3); (iii) a mixture of diastereomers (1*R*,4*R*,5*R* 8*R*)-8-acetylacetoxy-4,8-dimethyl-4 phenylthiomethyl-2,3-dioxabicyclo[3.3.1]nonane **14a** and (1*R*,4*S*,5*R*,8*R*)-8-acetylacetoxy-4,8-dimethyl-4 phenylthiomethyl-2,3-dioxabicyclo [3.3.1]nonane **14b** (152 mg, 12.6% yield, **14a/14b** *ca*. 54:46; keto/enol *ca*. 91:9) as a colorless oil, R_{f} 0.40 (hexane-EtOAc 7:3); (iv) a mixture of diastereomers of *E*-enolacetates ***E*****-15a,b** (48 mg, 0.105 mmol, 3.6% yield, ***E*****-15a/*****E*****-15b** *ca*. 54:46) as a yellowish viscous oil, R_{f} 0.61 (hexane-EtOAc. 7:3); (v) a mixture of diastereomers of Z-enolacetates **Z-15a,b** (41 mg, 0.090 mmol, 3.1% yield, **Z-15a/Z-15b** *ca*. 52:48) as a yellowish viscous oil, R_{f} 0.50 (hexane-EtOAc 7:3). Spectral data for **13a,b**: IR (neat): 692, 742, 1022 v.s, 1054, 1204, 1223, 1254 v.s, 1371 v.s, 1453, 1732 v.s, 2935 cm⁻¹. ¹H NMR (CDCl₃, 400 MHz) (δ): 1.24 (br.s, Me¹¹, **13a**), 1.56 (br.s, Me¹¹, **13b**), total 3H; 1.65 (s, Me¹⁰, **13b**), 1.66 (s, Me¹⁰, **13a**), total 3H; 1.67-1.89 (m, 3H, Hₑ⁶ + Hₐ⁶ + Hₐ⁹, **13a+13b**); 1.74 (m, Hₑ⁵, **13b**), 1.90 (dddd, ³*J*₅ₑ₆ₑ = ³*J*₅ₑ₉ₑ = 6.6 Hz, ³*J*₅ₑ₆ₐ = ³*J*₅ₑ₉ₐ = 3.3 Hz, Hₑ⁵ , **13a**), total 1H ; 2.07 (ddd, ²*J*₉ₑ₉ₐ = 14.1 Hz, ³*J*₉ₑ₅ₑ = 6.6 Hz,³*J*₉ₑ₁ₑ = 3.4 Hz, Hₑ⁹, **13a**), 2.27 (m, Hₑ⁹, **13b**), total 1H, 2.12-2.20 (m, 1H, Hₑ⁷, **13a+13b**) ; 2.25 (ddd, ²*J*₇ₐ₇ₑ = 15.0 Hz, *J*₇ₐ₆ₐ = 13.2 Hz, ³*J*₇ₐ₆ₑ = 6.0 Hz, Hₐ⁷, **13b**), 2.28 (ddd, ²*J*₇ₐ₇ₑ = 14.3 Hz, *J*₇ₐ₆ₐ = 13.3 Hz,³*J*₇ₐ₆ₑ = 6.0 Hz, Hₐ⁷, **13a**), total 1H; 2.95 (H¹²,**13b**)) and 3.02 (⁴*J*_{12,11} = 0.4 Hz, H'¹², **13b**), (ABq, ²*J* = 12.0 Hz), 3.30 (d, ²*J* = 12.8 Hz, H¹², **13a**) and 3.72 (dd, ²*J* = 12.8 Hz, ⁴*J*_{12,11} = 0.5 Hz, H¹², **13a**), total 2H; 4.40 (dd, ³*J*₁ₑ₉ₑ = 3.4 Hz, ³*J*₁ₑ₉ₐ = 1.0 Hz, H¹, **13a**), 4.44 (dd, ³*J*₁ₑ₉ₑ = 3.4 Hz, ³*J*₁ₑ₉ₐ = 0.8 Hz, H¹, **13b**), total 1H; 7.17-7.23 (m, 1H, H¹⁶, **13a+13b**); 7.29 (m, 2H, H¹⁵, **13a+13b**); 7.37 (m, 2H, H¹⁴, **13a+13b**). ¹³C NMR (CDCl₃, 100 MHz) (δ): Isomer **13a**: 22.03 (Me¹¹), 22.42 (Me¹⁰), 22.55 (Me¹⁷C = O), 23.50 (C⁶H₂), 23.97 (C⁹H₂), 28.52 (C⁵H), 32.88 (C⁷H₂), 40.71 (C¹²H₂), 77.46 (C¹H), 82.76 (C⁸), 83.85 (C⁴), 126.24 (C¹⁶H), 128.91 (2C¹⁴H), 129.76 (2C¹⁵H), 136.73 (C¹³), 170.13 (C=O); Isomer **13b**: 21.77 (Me¹¹),22.53 (Me¹⁰), 22.55 (Me¹⁷C=O) 23.22 (C⁶H₂), 24.09 (C⁹H₂), 29.89(C⁵H), 33.20 (C⁷H₂), 40.81 (C¹²H₂), 77.91 (C¹H), 82.67 (C⁸), 83.73 (C⁴), 126.43 (C¹⁶H), 128.96 (2C¹⁴H), 129.73 (2C¹⁵H), 136.29 (C¹³), 170.13 (C=O). Spectral data for **14a,b**: ¹H NMR (CDCl₃, 400 MHz) (δ): 1.23 (br.s, Me¹¹, **14a**), 1.55 (br.s, Me¹¹, **14b**), total 3H; 1.68 (s, Me¹⁰, **14b**), 1.69 (s, Me¹⁰, **14a**), total 3H; 1.70-1.93 (m, 4H); 2.08 (ddd, ²*J*₉ₑ₉ₐ = 14.0 Hz, ³*J*₉ₑ₅ₑ = 6.5 Hz,³*J*₉ₑ₁ₑ = 3.5 Hz, Hₑ⁹, **14a**), 2.15-2.33 (m), total 3H; 2.25 (s, Me¹⁸C=O, **14a**), 2.26 (s, Me¹⁸C=O, **14b**), total 3H; 2.95 (H¹²,**14b**) and 3.00 (br., H'¹², **14b**), (ABq, ²*J* = 12.6 Hz), 3.31 (d, ²*J* = 12.8 Hz, H¹², **14a**) and 3.69 (br.d, ²*J* = 12.8 Hz, H¹², **14a**), total 2H; 3.40 (s, C¹⁷H₂C=O, **14a**), 3.41 (s, C¹⁷H₂C=O, **14b**), total 2H; 4.37 (br.d, ³*J*₁ₑ₉ₑ = 3.5 Hz, Hₑ¹, **14a** ₖₑₜₒ), 4.41 (br.d, ³*J*₁ₑ₉ₑ = 3.6 Hz, Hₑ¹,**14b** ₖₑₜₒ), 4.45 (br.d, ³*J*₁ₑ₉ₑ = 3.5 Hz, Hₑ¹, **14a** ₑₙₒₗ), 4.49 (br.d, ³*J*₁ₑ₉ₑ = 3.5 Hz, Hₑ¹**,14b** ₑₙₒₗ), total 1H; 4.92 (br.s, HC= , **14a** ₑₙₒₗ), 4.94 (br.s, HC= , **14b** ₑₙₒₗ); 7.26-7.42 (m, 5H _{arom.,} **14a + 14b**); 12.05 (br.s, HO_{enol,} **14a** + **14b**). ¹³C NMR (CDCl₃, 100 MHz) (δ): Isomer **14a**: 21.89 (Me¹¹), 22.48 (Me¹⁰), 23.28 (C⁶H₂), 23.74 (C⁹H₂), 28.43 (C⁵H), 30.15 (Me¹⁸C = O),32.67 (C⁷H₂), 40.59 (C¹²H₂), 51.18 (C¹⁷H₂), 77.30 (C¹H), 83.76 (C⁴), 84.25 (C⁸), 90.60 (HC¹⁷ = enol), 126.16 (C¹⁶H), 128.83 (2C¹⁴H), 129.65 (2C¹⁵H), 136.62 (C¹³), 165.83 (OC²⁰=O), 200.57 (C¹⁹=O) ; Isomer **14b:** 21.64 (Me¹¹),22.49 (Me¹⁰), 22.98 (C⁶H₂), 23.85 (C⁹H₂), 29.74 (C⁵H), 30.15 (Me¹⁸C = O), 33.02 (C⁷H₂), 40.75 (C¹²H₂), 51.18 (C¹⁷H₂), 77.72 (C¹H), 83.82 (C4), 84.17 (C⁸), 90.60 (HC¹⁷ = ₑₙₒₗ), 126.37 (C¹⁶H), 128.88 (2C¹⁴H), 129.68 (2C¹⁵H), 136.18 (C¹³), 165.84 (OC²⁰=O), 200.57 (C¹⁹=O).

### Example 10: Preparation of (1R,4R,5R,8R)-8-ethoxyoxalyloxy-4,8-dimethyl-4-phenylthiomethyl-2,3- dioxabicyclo [3.3.1]nonane (18a) and (1R,4S,5R,8R)-8-ethoxyoxalyloxy-4,8-dimethyl-4-phenylthiomethyl-2,3-dioxabicyclo [3.3.1]nonane (18b) (Scheme 6).

(a) To a solution of hydroxysulfides **2a,b** (153 mg, 0.52 mmol) and 2,6-lutidine (155 mg, 1.45 mmol) in CH₂Cl₂ (3 mL) at 0°C was added neat TfOTMS (260 mg, 1.10 mmol) and the reaction mixture was stirred at 0°C for 30 min, the temperature was raised to 20°C. After 30 min the reaction mixture was poured into the cold water (4°C, 30 mL) and extracted with EtOAc-hexane (1:4, 2 x 30 mL), washed with cold saturated NaHCO₃ (15 mL), dried (Na₂SO₄), and evaporated to give the crude TMS-derivative **16a,b** (**16a/ 16b** *ca*. 56:44) (204 mg, *ca*. quant. yield) as a mobile pale yellow liquid. IR (neat): 2954, 2928, 1453, 1446, 1372, 1250 v.s, 1125, 1060 v.s, 1040 v.s, 858 v.s, 840 v.s, 748, 739, 690 cm⁻¹. ¹H NMR (CDCl₃, 400 MHz): 0.108 and 0.111 (s, Me₃Si), total 9H; 1.22 (br.s, Me¹¹ **16a**) and 1.53 (d, Me¹¹, ⁴*J* = 0.5 Hz, **16b**), total 3H; 1.38 (s, Me¹⁰ **16b**) and 1.39 (s, Me¹⁰ **16a**), total 3H; 1.60-1.65 (m, 1H, Hₑ⁷ **16a + 16b**), 1.67-1.82 (m, 3H), 1.84 (dddd, ³*J*₅ₑ₉ₑ = ³*J*₅ₑ₆ₑ = 6.0 Hz, ³*J*₅ₑ₉ₐ = ³*J*₅ₑ₆ₐ = 3.0 Hz, Hₑ⁵ **16a**), 1.97-1.99 (m, 1H), 2.06 (ddd, ²*J* = 13.0 Hz, ³*J*₉ₐ₅ₑ = 3.0 Hz, ³*J*₉ₐ₁ₑ = 2.5 Hz, Hₐ⁹ **16a**), 2.13-2.25 (m), total 2H; 2.945 and 3.015 (⁴*J*_{12',11} = 0.5 Hz) (ABq, ²*J* = 12.0 Hz, H¹² + H'¹² **16b**), 3.32 (d, ²*J* = 12.0 Hz, H¹² **16a**) and 3.68 (dd, ²*J* = 12.7 Hz, ⁴*J*_{12',11} = 0.6 Hz, H'¹² **16a**), total **2H**; 3.59 (br.dd, ³*J*₁ₑ₉ₑ = ³*J*₁ₑ₉ₐ = 2.5 Hz, Hₑ¹ **16a**), 3.63 (m, Hₑ¹ **16b**), total 1H; 7.26-7.42 (m, 5H).

(b) The mixture of TMS-derivative **16a,b** (202 mg, *ca*. 0.52 mmol) and oxalyl chloride (3 mL) was stirred at rt for 18 h, evaporated under vacuum for 3 h (0.2 mm Hg) to afford the crude chloride **17a,b** (**17a/17b***ca*. 56:44) as a viscous pale brown oil. IR (neat): 2965, 2937, 1796 v.s, 1754 v.s, 1481, 1452, 1441, 1376, 1275 v.s, 1224, 1118, 1053, 1015 v.s, 977 v.s, 954, 823 v.s, 808, 741 v.s, and 692 cm⁻¹. ¹H NMR (CDCl₃, 250 MHz, δ): 1.26 (br.s, Me¹¹ **17a**) and 1.58 (br.s, Me¹¹ **17b**), total 3H; 1.78 (s, Me¹⁰ **17b**) and 1.79 (s, Me¹⁰ **17a**), total 3H; 1.68-2.02 (m, 4H), 2.15 (ddd, ²*J* = 13.8 Hz, ³*J*₉ₑ₅ₑ = 6.6 Hz, ³*J*₉ₑ₁ₑ = 3.4 Hz, Hₑ⁹ **17a**), 2.30-2.44 (m), total 3H; 2.965 and 3.00 (br.) (ABq, ²*J* = 12.1 Hz, H¹² + H'¹² **17b**), 3.33 (d, ²*J* = 13.0 Hz, H¹² **17a**) and 3.70 (dd, ²*J* = 13.0 Hz, ⁴*J*_{12',11} = 0.5 Hz, H'¹² **17a**), total 2H; 4.28 (br.dd, ³*J*₁ₑ₉ₑ = 3.4Hz, ³*J*₁ₑ₉ₐ = 1.0 Hz, Hₑ¹ **17a**), 4.32 (br.dd, ³*J*₁ₑ₉ₑ = 3.4Hz, ³*J*₁ₑ₉ₐ = 1.0 Hz, Hₑ¹ **17b**), total 1H; 7.18-7.45 (m, 5H).

(c) To a solution of crude chloride **17a,b** in CH₂Cl₂ (3 mL) at 0°C was added a solution of EtOH (79 mg, 1.7 mmol) and 2,6-lutidine (214 mg, 2.0 mmol) in CH₂Cl₂ (2 mL) and the reaction mixture was stirred overnight at 4°C. The mixture was poured into cold water (50 mL), extracted with EtOAc-hexane (1:4, 2 x 50 mL), dried (Na₂SO₄) and evaporated. Flash chromatography (hexane-EtOAc, 85:15) of the residue gave the esters **18a,b** (**18a /18b** *ca*. 57:43) (140 mg, 0.355 mmol, 68% yield) as a viscous pale yellow liquid, R_{f} 0.41 (hexane-EtOAc, 4:1). IR (neat): 2984, 2938, 1765 v.s, 1742 v.s, 1451, 1441, 1374, 1320 v.s, 1185 v.s, 1090, 1053, 1013 v.s, 741 v.s, 692 cm⁻¹. ¹H NMR (CDCl₃, 250 MHz, δ): 1.26 (br.s, Me¹¹ **18a**) and 1.57 (br.s, Me¹¹ **18b**), total 3H; 1.373 (t, ³*J* = 7.1 Hz, Me¹⁸CH₂ **18a**), 1.378 (t, ³*J* = 7.1 Hz, Me¹⁸CH₂ **18b**), total 3H; 1.75 (s, Me¹⁰ **18b**) and 1.76 (s, Me¹⁰ **18a**), total 3H; 1.68-1.96 (m, 4H), 2.11 (ddd, ²*J* = 13.6 Hz, ³*J*₉ₑ₅ₑ = 6.2 Hz, ³*J*₉ₑ₁ₑ = 3.4 Hz, Hₑ⁹ **18a**), 2.27-2.39 (m), total 3H; 2.96 and 3.015 (br.) (ABq, ²*J* = 12.1 Hz, H¹² + H'¹² **18b**), 3.32 (d, ²*J* = 12.9 Hz, H¹² **18a**) and 3.71 (br.d, ²*J* = 12.9 Hz, H'¹² **18a**), total 2H; 4.325 (q, ³*J* = 7.1 Hz, C¹⁷H₂O **18a**), 4.331 (q, ³*J* = 7.1 Hz, C¹⁷H₂O **18b**), total 2H; 4.34 (m, Hₑ¹ **18a**), 4.38 (m, Hₑ¹ **18b**), total 1H; 7.17-7.44 (m, 5H).

### Example 11: Preparation of (1R,4R,5R,8R)-8-dibenzylaminooxalyloxy-4,8-dimethyl-4-phenylthiomethyl-2,3-dioxabicyclo[3.3.1]nonane (19a) and (1R,4S,5R,8R)-8-dibenzylaminooxalyloxy-4,8-dimethyl-4-phenylthiomethyl-2,3-dioxabicyclo[3.3.1]nonane (19b) (Scheme 6).

To a solution of chloride **17 a,b,** prepared from hydroxysulfide **2a,b** (112 mg, 0.38 mmol) as described above, in CH₂Cl₂ (3 mL) at 0°C was added a solution of dibenzylamine (156 mg, 0.8 mmol) and 2,6-lutidine (160 mg, 1.5 mmol) in CH₂Cl₂ (2 mL). The mixture was stirred for 8 h at 0°C , poured into cold water (50 mL), extracted with hexane-EtOAc (3:1, 2 x 50 mL), organic layer was washed with saturated KHSO₄ (15 mL) and NaHCO₃ (15 mL), dried (Na₂SO₄) and evaporated. Flash chromatography (hexane-EtOAc, 85:15) of the residue gave the amides **19a,b** (**19a/19b***ca*. 59:41) (160 mg, 0.293 mmol, 77% yield based on the alcohols **2a,b**) as a colorless oil, R_{f} 0.38 (hexane-EtOAc, 4:1). IR (neat): 1728 v.s, 1661 v.s, 1452 v.s, 1440 v.s, 1374, 1287, 1265, 1170 v.s, 1081, 1053, 1014 v.s, 738 v.s, 694 v.s. cm⁻¹. ¹H NMR (CDCl₃, 250 MHz, δ): 1.24 (br.s, Me¹¹ **19a**) and 1.57 (br.s, Me¹¹ **19b**), total 3H; 1.71 (br.s, 3H, Me¹⁰ **19a** + **19b**), 1.62-1.90 (m, 4H), 2.06 (ddd, ²*J* = 13.4 Hz, ³*J*₉ₑ₅ₑ = 6.2 Hz, ³*J*₉ₑ₁ₑ = 3.5 Hz, Hₑ⁹ **19a**), 2.18-2.42 (m), total 3H; 2.95 and 3.00 (br.) (ABq, ²*J* = 12.1 Hz, H¹² + H'¹² 19b), 3.32 (d, ²*J* = 13.0 Hz, H¹² **19a**) and 3.68 (br.d, ²*J* = 13.0 Hz, H'¹² **19a**), total 2H; 4.33 (br.d, ³*J*₁ₑ₉ₑ = 3.4 Hz, Hₑ¹ **19a**) and 4.39 (m, Hₑ¹ **19b**), total 1H; 4.38 (br.s, 2H, NC¹⁷H₂), 4.52 (br.s, 2H, NC¹⁸H₂), 7.22-7.41 (m, 15H).

### Example 12: Preparation of (1S,4S,5S,8S)-8-acetoxy-4,8-dimethyl-4 phenylthiomethyl-2,3-dioxabicyclo [3.3.1]nonane (13'a) and (1S,4R,5S,8S)-8-acetoxy-4,8-dimethyl-4-phenylthiomethyl-2,3-dioxabicyclo[3.3.1]nonane (13'b) (Scheme 7).

To a neat TMS-derivative **16'a,b,** prepared from **2'a,b** (0.854 mmol, 252 mg) and TMSOTf as described in Example 9, was added AcCl (2.0 mL) and the mixture was stirred at rt for 50 h. The mixture was evaporated to give the crude acetoxysulfide **13' a,b** (283 mg, 0.841 mmol, practically quantitative yield) as a pale yellow oil. ¹H NMR spectrum of **13'a,b** is consistent with that of **13a,b**.

### Example 13: Preparation of (1R,4R,5R,8R)-4,8-dimethyl-4-phenylsulfonylmethyl-2,3-dioxabicyclo [3.3.1]nonan-8-ol (21a) and (1R,4S,5R, 8R)-4,8-dimethyl-4-phenylsulfonylmethyl-2,3-dioxabicyclo[3.3.1]nonan-8-ol (21b) (Scheme 8).

To a solution of sulfides **2a** and **2b** (55/45) (2.10 g, 7.13 mmol) in EtOAc (40 mL) at rt was added a solution of MCPBA (5.12 g of *ca*. 60%, 17.8 mmol) in EtOAc (100 mL) and the reaction mixture was stirred for 8 h. Then it was diluted with hexane (140 mL), washed with saturated NaHCO₃ (2 x 100 mL), the combined water washings were extracted with EtOAc-hexane (1:1, 200 mL). The organic extract was dried (Na₂SO₄ and NaHCO₃), evaporated and the residue was chromatographed (hexane-EtOAc, 1:1) to afford a mixture of diastereomers **21a** and **21b** (2.128 g, 91% yield; **21a/ 21b** *ca*. 55:45). The diastereomers were separated by MPLC (hexane-EtOAc, 1:1). Less polar isomer **21a**, mp 112-113°C (hexane-EtOAc, 5 : 1), R_{f} 0.38 (EtOAc-hexane, 1:1). ¹H NMR (400 MHz, CDCl₃, δ): 1.33 (s, 3H, Me¹⁰), 1.49 (br.s, 3H, Me¹¹), 1.58 (ddd, 1H, ²*J* = 14.2 Hz, ³*J*₇ₑ₆ₑ = 5.4 Hz, ³*J*₇ₑ₆ₐ = 0.9 Hz, Hₑ⁷), 1.80-1.93 (m, 2H, Hₑ⁶ + Hₐ⁶), 2.10 (ddd, 1H, ²*J* = 13.9 Hz, ³*J*₉ₐ₅ₑ = 3.0Hz, ³*J*₉ₐ₁ₑ = 2.0 Hz, Hₐ⁹), 2.21 (ddd, 1H,²*J* = 13.9 Hz, ³*J*₉ₑ₅ₑ = 6.1 Hz, ³*J*₉ₑ₁ₑ = 3.5 Hz, Hₑ⁹), 2.27 (m, 1H, Hₑ⁵), 2.27 (ddd, 1H, ²*J* = 14.2 Hz, ³*J*₇ₐ₆ₐ = 13.8 Hz, ³*J*₇ₐ₆ₑ = 6.6 Hz, Hₐ⁷), 3.26 (d, 1H, ²*J* = 14.3 Hz, H¹²), 3.66 (dd, 1H, ³*J*₁ₑ₉ₑ = 3.5 Hz, ³*J*₁ₑ₉ₐ = 2.0 Hz, Hₑ¹), 4.21 (br.d, 1H, ²*J* = 14.3 Hz, H'¹²), 7.57 (dddd, 2H, ³*J*_{15,14} = ³*J*_{15,16} = 7.4 Hz, ⁴*J*_{1514'} = ⁴*J*_{15'14} = 1.3 Hz, H^{15, 15'}), 7.65 (dddd, 1H, ³*J*_{16,15} = ³*J*_{16,15'} = 7.4 Hz, ⁴*J*₁₆₁₄ = ⁴*J*_{1614'} = 1.3 Hz, H¹⁶), 7.93 (ddd, 2H, ³*J*_{14,15} = ³*J*_{14',15'} = 7.4 Hz, ⁴*J*_{14,16} = 1.3 Hz, H^{14, 14'}); ¹³C NMR (100 MHz, CDCl₃, δ): 22.87 (Me¹¹), 23.45 (C⁶H₂), 24.64 (C⁹H₂), 27.97 (Me¹⁰), 30.05 (C⁵H), 35.70 (C⁷H₂), 61.02 (C¹²H₂), 71.30 (C⁸), 81.96 (C¹H), 82.71 (C⁴), 127.51 (2C^{14'}H), 129.33 (2C¹⁵H), 133.71 (C¹⁶H), 141.08 (C¹³). Anal. Calcd for C₁₆H₂₂O₅S: C, 58.87; H, 6.79; S, 9.82. Found: C, 58.87; H, 6.84; S, 9.73. More polar isomer **21b**: a colorless oil, R_{f} 0.34 (EtOAc-hexane, 1:1). ¹H NMR (400 MHz, CDCl₃, δ): 1.28 (s, 3H, Me¹⁰), 1.58 (br.dd, 1H, ²*J* = 14.0 Hz, ³*J*₇ₑ₆ₑ = 5.2 Hz, Hₑ⁷), 1.70 (br.s, 1H, OH), 1.79 (br.s, 3H, Me¹¹), 1.85-1.99 (m, 2H, Hₑ⁶ + Hₐ⁶), 2.03 (ddd, 1H, ²*J* = 13.4 Hz, ³*J*₉ₐ₅ₑ = 3.2 Hz, ³*J*₉ₐ₁ₑ = 1.7 Hz, Hₐ⁹), 2.08 (dddd, 1H, ³*J*₅ₑ₆ₑ = ³*J*₅ₑ₉ₑ = 6.2 Hz, ³*J*₅ₑ₆ₐ = ³*J*₅ₑ₉ₐ = 3.2 Hz, Hₑ⁵), 2.12 (ddd, 1H, ²*J* = ³*J*₇ₐ₆ₐ = 14.0 Hz, ³*J*₇ₐ₆ₑ = 6.7 Hz, Hₐ⁷), 2.26 (ddd, 1H, ²*J* = 13.4 Hz, ³*J*₉ₑ₅ₑ = 6.2 Hz, ³*J*₉ₑ₁ₑ = 3.3 Hz, Hₑ⁹), 3.14 (d, 1H, ²*J* = 14.0 Hz, H¹²), 3.33 (br.d, 1H, ²*J* = 14.0 Hz, H'¹²), 3.68 (dd, 1H, ³*J*₁ₑ₉ₑ = 3.3 Hz, ³*J*₁ₑ₉ₐ = 1.7 Hz, Hₑ¹), 7.56 (dddd, 2H, ³*J*_{15,14} = ³*J*_{15,16} = 7.4 Hz, ⁴*J*_{1514'} = ⁴*J*_{15'14} = 1.4 Hz, H^{15, 15'}), 7.66 (dddd, 1H, ³*J*_{16,15} = ³*J*_{16,15'} = 7.4 Hz, ⁴*J*₁₆₁₄ = ⁴*J*_{1614'} = 1.4 Hz, H¹⁶), 7.92 (ddd, 2H, ³*J*_{14,15} = ³*J*_{14',15'} = 7.4 Hz, ⁴*J*_{14,16} = 1.3 Hz, H^{14, 14'}); ¹³C NMR (100 MHz, CDCl₃, δ): 21.84 (Me¹¹), 23.57 (C⁹H₂), 23.85 (C⁶H₂), 28.01 (Me¹⁰), 31.53 (C⁵H), 35.54 (C⁷H₂), 60.55 (C¹²H₂), 71.19 (C⁸), 82.45 (C¹H), 82.94 (C⁴), 127.62 (2C¹⁴H), 129.33 (2C¹⁵H), 133.83 (C¹⁶H), 141.21 (C¹³).

### Example 14: Preparation of (1R,4R,5R,8S)-4,8-dimethyl-4-phenylsulfonylmethyl-2,3-dioxabicyclo [3.3.1]nonane (22a) and (1R,4S,5R,8S)-4,8-dimethyl-4-phenylsulfonylmethyl-2,3-dioxabicyclo[3.3.1]nonane (22b) (Scheme 8).

To a solution of sulfides **11 a,b** (18.0 mg, 0.065 mmol, **11a/11b** 54:46) in EtOAc (0.5 mL) was added MCPBA (52 mg of *ca*. 60%, 0.18 mmol) and the mixture was stirred for 8 h at rt The mixture was poured into saturated NaHCO₃ (10 mL), extracted with hexane-EtOAc (7:3, 40 mL), organic extract was washed with saturated NaHCO₃ (10 mL) and the water washings were extracted with hexane-EtOAc (7:3, 30 mL). The combined organic layer was dried (Na₂SO₄ + NaHCO₃) and evaporated. Flash chromatography (hexane-EtOAc, 80:20) afforded a mixture of diastereomeric sulfones **22 a,b** (18.0 mg, 90% yield, ratio **22a/22b** *ca*. 55:45). The sulfones were separated by semi-preparative DP HPLC (column - LiChrospher® Si 60 (10 µm) 250-10; eluent *i*-PrOH-hexane 2:98). Less polar isomer **22 a**, colorless semisolid, τ_{R} 16.7 min (column - LiChrospher® Si 60 (5 µm) 250-4; eluent EtOAc-hexane 12:88). ¹H NMR (CDCl₃, 250 MHz) (δ): 1.09 (d, 3H, ³*J* = 6.4 Hz, Me¹⁰), 1.53 (br.s, 3H, Me¹¹),1.58 (ddd, 1H, ²*J* = 13.4 Hz, ³*J*₉ₐ₅ₑ = 3.0 Hz, ³*J*₉ₐ₁ₑ = 1.7 Hz, Hₐ⁹),1.64-2.13 (m, 5H, Hₑ⁶ + Hₐ⁶ +Hₑ⁷ + Hₐ⁷ + Hₐ⁸), 2.36 (br.dddd, 1H, ³*J*₅ₑ₉ₑ = ³*J*₅ₑ₆ₑ = 6.0 Hz, ³*J*₅ₑ₉ₐ = ³*J*₅ₑ₆ₐ = 3.0 Hz, Hₑ⁵), 2.48 (br.ddd, 1H, ²*J*₉ₑ₉ₐ = 13.4 Hz, ³*J*₉ₑ₅ₑ = 6.0 Hz, ³*J*₉ₑ₁ₑ = 3.6 Hz, Hₑ⁹), 3.25 (d, 1H, ²*J* = 14.3 Hz, H¹²), 3.86 (br.dd, 1H, ³*J*₁ₑ₉ₑ = 3.6 Hz, ³*J*₁ₑ₉ₐ = 1.7 Hz, Hₑ¹), 4.31 (br.dd, 1H, ²*J* = 14.3 Hz, ⁴*J* = 0.6 Hz, H'¹²), 7.60 (m, 2H, H^{15, 15'}), 7.66 (m, 1H, H¹⁶), 7.97 (m, 2H, H^{14, 14'}); DCI HRMS obsd 311.12940, calcd for C₁₆H₂₃O₄S (M + H) 311.13171. More polar isomer **22b**, colorless solid, mp 129-130°C, τ_{R} 17.7 min. (column - LiChrospher® Si 60 (5 µm) 250-4; eluent EtOAc-hexane 12:88). ¹H NMR (CDCl₃, 400 MHz) (δ): 1.01 (d, 3H, ³*J* = 6.4 Hz, Me¹⁰), 1.46 (ddd, 1H, ²*J* = 13.3 Hz, ³*J*₉ₐ₅ₑ = 3.1 Hz, ³*J*₉ₐ₁ₑ = 2.0 Hz, Hₐ⁹), 1.65 (m, 1H), 1.68-1.82 (m, 3H, Hₑ⁶ + Hₐ⁶ +Hₑ⁷ + Hₐ⁷); 1.80 (d, 3H, ⁴*J*_{11,12'} = 0.5 Hz, Me¹¹), 2.07-2.14 (m, 2H, Hₑ⁵ + Hₐ⁸), 2.47 (dddd, 1H, ²*J*₉ₑ₉ₐ = 13.3 Hz, ³*J*₉ₑ₁ₑ = 4.4 Hz, ³*J*₉ₑ₅ₑ = ⁴*J*₉ₑ₆ₑ = 3.2 Hz, Hₑ⁹), 3.14 (d, 1H, ²*J* = 14.0 Hz, H¹²), 3.37 (br.dd, 1H, ²*J* = 14.0 Hz, ⁴*J*_{12'11} = 0.5 Hz, H'¹²), 3.85 (br.dd, 1H, ³*J*₁ₑ₉ₑ = 4.4 Hz, ³*J*₁ₑ₉ₐ = 2.0 Hz, Hₑ¹), 7.57 (m, 2H, H¹⁵, 15'), 7.66 (m, 1H, H¹⁶), 7.93 (m, 2H, H^{14, 14'}); ¹³C NMR (100 MHz, CDCl₃, δ): 18.49 (Me¹⁰), 22.06(Me¹¹), 27.33 (CH₂), 28.96 (CH₂), 29.61 (C⁷H₂), 31.56 (C⁵H), 35.53 (C⁸H), 60.60 (C¹²H₂), 80.06 (C¹H), 82.53 (C⁴), 127.64 (2C¹⁴H), 129.32 (2C¹⁵H), 133.76 (C¹⁶H), 141.37 (C¹³). CI HRMS (CH₄) obsd 311.12650, calcd for C₁₆H₂₃O₄S (M + H) 311.13171.

### Example 15: Preparation of (1R,4R,5R,8R)-8-acetoxy-4,8-dimethyl-4-phenylsulfonylmethyl-2,3-dioxabicyclo [3.3.1]nonane (23a) and (1R,4S,5R,8R)-8-acetoxy-4,8-dimethyl-4-phenylsulfonylmethyl-2,3-dioxabicyclo[3.3.1]nonane (23b) (Scheme 8).

To a solution of sulfides **13a** and **13b** (55/45) (55 mg, 0.163 mmol) in EtOAc (1 mL) at 0°C was added a solution of MCPBA (103 mg of ∼60%, 0.35 mmol) in EtOAc (1.5 ml) and the reaction mixture was stirred for 4 h at rt The reaction mixture was poured into saturated NaHCO₃ (10 mL), extracted with EtOAc-hexane (1:2, 2 x 30 mL). The organic extract was dried (Na₂SO₄ and NaHCO₃), evaporated and the residue was separated by MPLC (hexane-EtOAc, 7:3) to give the sulfones **23a** (31 mg) and **23b** (26 mg) (total yield 97%). Less polar isomer **23a,** mp 96-98°C, R_{f} 0.37 (hexane-benzene-EtOAc 11:6:3). ¹H NMR (400 MHz, CDCl₃, δ): 1.51 (br.s, 3H, Me¹¹), 1.62 (s, 3H, Me¹⁰), 1.78 (dddd, 1H, ²*J* = 14.5 Hz, ³*J*₆ₐ₇ₐ = 13.4 Hz, ³*J*₆ₐ₇ₑ = 6.8 Hz, ³*J*₆ₐ₅ₑ = 3.4 Hz, Hₐ⁶), 1.88 (ddd, 1H, ²*J* = 14.0 Hz, ³*J*₉ₐ₅ₑ = 3.4 Hz, ³*J*₉ₐ₁ₑ = 1.2 Hz, Hₐ⁹), 1.90 (dddd, 1H, ²*J* = 14.5 Hz, ³*J*₆ₑ₅ₑ = 6.8 Hz, ³*J*₆ₑ₇ₑ = ³*J*₆ₑ₇ₐ = 2.8 Hz, Hₑ⁶), 2.01 (s, 3H, Me¹⁷CO), 2.14 (m, 1H, Hₑ⁷), 2.19 (m, 1H, Hₐ⁷), 2.26 (ddd, 1H, ²*J* = 14.0 Hz, ³*J*₉ₑ₅ₑ = 6.5 Hz, ³*J*₉ₑ₁ₑ = 3.2 Hz, Hₑ⁹), 2.30 (br.dddd, 1H, ³*J*₅ₑ₉ₑ = ³*J*₅ₑ₆ₑ = 6.8 Hz, ³*J*₅ₑ₉ₐ = ³*J*₅ₑ₆ₐ = 3.4 Hz, Hₑ⁵), 3.25 (d, 1H, ²*J* = 14.3 Hz, H¹²), 4.22 (dd, 1H, ²*J* = 14.3 Hz, ⁴*J*_{12',11} = 0.4 Hz, H'¹²), 4.45 (br.dd, ³*J*₁ₑ₉ₑ = 3.2 Hz, *J*₁ₑ₉ₐ = 1.2 Hz, Hₑ¹), 7.58 (m, 2H, H^{15, 15'}), 7.66 (m, 1H, H¹⁶), 7.94 (m, 2H, H^{14, 14'}). ¹³C NMR (100 MHz, CDCl₃, δ): 22.42 (Me¹⁷CO), 22.49 (Me¹⁰), 23.09 (Me¹¹), 23.39 (C⁶H₂), 24.52 (C⁹H₂), 29.42 (C⁵H), 33.26 (C⁷H₂), 60.98 (C¹²H₂), 77.80 (C¹H), 82.54 (C⁸), 82.72 (C⁴), 127.52 (2C¹⁴H), 129.35 (2C¹⁵H), 133.75 (C¹⁶H), 141.08 (C¹³), 170.20 (C=O). HRMS (Cl, CH₄): obsd 369.13300, calcd for C₁₈H₂₅O₆S (M + 1) 369.13719. Anal. Calcd for C₁₈H₂₄O₆S: C, 58.68; H, 6.56; S, 8.70. Found: C, 58.64; H, 6.55; S, 8.34. More polar isomer **23b,** a colorless solid, mp 101-102°C, R_{f} 0.31 (hexane-benzene-EtOAc 11:6:3). ¹H NMR (400 MHz, CDCl₃, δ): 1.56 (s, 3H, Me¹⁰), 1.79 (ddd, 1H, ²*J* = 13.6 Hz, ³*J*₉ₐ₅ₑ = 3.4 Hz, ³*J*₉ₐ₁ₑ = 1.4 Hz, Hₐ⁹), 1.80 (br.s, 3H, Me¹¹), 1.84 (dddd, 1H, ²*J* = 14.0 Hz, ³*J*₆ₐ₇ₐ = 14.0 Hz, ³*J*₆ₐ₇ₑ = 6.0 Hz, ³*J*₆ₐ₅ₑ = 3.4 Hz, Hₐ⁶), 1.96 (m, 1H, Hₑ⁶), 2.00 (s, 3H, Me¹⁷CO), 2.04 (m, 1H, Hₐ⁷), 2.10 (dddd, 1H, ³*J*₅ₑ₉ₑ = ³*J*₅ₑ₆ₑ = 6.8 Hz, ³*J*₅ₑ₉ₐ = ³*J*₅ₑ₆ₐ = 3.4 Hz, Hₑ⁵), 2.16 (br.dd, 1H, ²*J* = 14.5 Hz, ³*J*₇ₑ₆ₑ = 6.0 Hz, Hₑ⁷), 2.29 (ddd, 1H, ²*J* = 13.6 Hz, ³*J*₉ₑ₅ₑ = 6.8 Hz, ³*J*₉ₑ₁ₑ = 3.6 Hz, Hₑ⁹), 3.12 (d, 1H, ²*J* = 14.0 Hz, H¹²), 3.32 (br.d, 1H, ²*J* = 14.0 Hz, H'¹²), 4.42 (br.d, ³*J*₁ₑ₉ₑ = 3.6 Hz, *J*₁ₑ₉ₐ = 1.4 Hz, Hₑ¹), 7.57 (m, 2H, H^{15, 15'}), 7.66 (m, 1H, H¹⁶), 7.92 (m, 2H, H^{14, 14'}); ¹³C NMR (100 MHz, CDCl₃, δ): 21.94 (Me¹¹), 22.43 (Me¹⁷CO), 22.53 (Me¹⁰), 23.40 (C⁹H₂), 23.78 (C⁶H₂), 30.92 (C⁵H), 32.92 (C⁷H₂), 60.50 (C¹²H₂), 78.45 (C¹H), 82.35 (C⁸), 82.97 (C⁴), 127.61 (2C¹⁴H), 129.37 (2C¹⁵H), 133.89 (C¹⁶H), 141.15 (C¹³), 170.16 (C=O). Anal. Calcd for C₁₈H₂₄O₆S: C, 58.68; H, 6.56; S, 8.70. Found: C, 58.72; H, 6.57; S, 8.51.

### Example 16: Preparation of (1R,4R,5R,8R)-8-acetylacetoxy-4,8-dimethyl-4-phenylsulfonylmethyl-2,3-dioxabicyclo[3.3.1]nonane (24a) and (1R,4S,5R,8R)-8-acetylacetoxy-4,8-dimethyl-4-phenylsulfonylmethyl-2,3-dioxabicyclo [3.3.1]nonane (24b) (Scheme 8).

To a solution of sulfides **14a** and **14b** (55/45) (130 mg, 0.34 mmol) in EtOAc (6 mL) at 0°C was added MCPBA (276 mg of *ca*. 60%, 0.96 mmol) and the mixture was stirred for 6 hr at rt The reaction mixture was poured into saturated NaHCO₃ (20 mL), extracted with EtOAc-hexane (2:3, 3 x 25 mL). The organic extract was dried (Na₂SO₄ and NaHCO₃), evaporated and the residue was purified by flash chromatography (hexane-EtOAc, 3:2) to afford a mixture of diastereomeric **24a** and **24b** (94 mg, total yield 66.7%, **24a/24b** *ca* .55:45). The sulfones were separated by MPLC (hexane-EtOAc, 3:2). Less polar isomer **24a,** colorless oil. ¹H NMR (400 MHz, CDCl₃, δ): 1.52 (br.s, 3H, Me¹¹), 1.66 (s, 3H, Me¹⁰), 1.80 (dddd, 1H, ²*J* = 14.0 Hz, ³*J*₆ₐ₇ₐ = 13.7 Hz, ³*J*₆ₐ₇ₑ = 6.5 Hz, ³*J*₆ₐ₅ₑ = 3.4 Hz, Hₐ⁶), 1.91 (m, 1H, Hₐ⁹), 1.93 (m, 1H, Hₑ⁶), 2.15-2.25 (m, 2H, Hₑ⁷ + Hₐ⁷), 2.26 (s, 3H, Me¹⁸C=O), 2.28 (ddd, 1H, ²*J* = 13.0 Hz, ³*J*₉ₑ₅ₑ = 6.4 Hz, ³*J*₉ₑ₁ₑ = 3.6 Hz, Hₑ⁹), 2.31 (dddd, 1H, ³*J*₅ₑ₉ₑ = ³*J*₅ₑ₆ₑ = 6.4 Hz,³*J*₅ₑ₉ₐ = ³*J*₅ₑ₆ₐ = 3.2 Hz, Hₑ⁵), 3.27 (d, 1H, ²*J* = 14.4 Hz, H¹²), 3.42 (s, *ca*. 2H, C¹⁷H₂CO _{keto form}), 4.20 (br.d, *ca*. 1H, ²*J* = 14.4 Hz, H'¹² ₖₑₜₒ), 4.23 (br.d, ²*J* = 14.0 Hz, H'¹² ₑₙₒₗ), total 1H; 4.43 (br.d, *ca*. 1H, ³*J*₁ₑ₉ₑ = 3.6 Hz, Hₑ⁹ ₖₑₜₒ), 4.50 (br.d, ³*J*₁ₑ₉ₑ = 4.0 Hz, Hₑ⁹ ₑₙₒₗ), total 1H; 4.94 (br.s, HC = ₑₙₒₗ), 7.58 (m, 2H, H^{15, 15'}), 7.67 (m, 1H, H¹⁶), 7.94 (m, 2H, H¹⁴, ¹⁴'), 12.05 (br.s, HO ₑₙₒₗ) (ratio keto/enol *ca*. 93:7 in *ca*. 2% solution in CDCl₃). ¹³C NMR (100 MHz, CDCl₃, δ): 22.53 (Me¹⁰), 23.01 (Me¹¹), 23.27 (C⁶H₂), 24.39 (C⁹H₂), 29.39 (C⁵H), 30.28 (Me¹⁸CO), 33.13 (C⁷H₂), 51.29 (C¹⁷H₂), 60.95 (C¹²H₂), 77.73 (C¹H), 82.75 (C⁴), 84.17 (C⁸), 127.53 (2C¹⁴H), 129.36 (2C¹⁵H), 133.76 (C¹⁶H), 141.05 (C¹³), 165.99 (OC²⁰ = O), 200.48 (MeC¹⁹ = O). HRMS (CI, CH₄): obsd 411.15100, calcd for C₂₀H₂₇O₇S (M + 1) 411.14775; obsd 439.17800, calcd for C₂₂H₃₁O₇S (M + 29) 439.17905. More polar isomer **24b,** colorless oil. ¹H NMR (400 MHz, CDCl₃, δ): 1.60 (s, 3H, Me¹⁰), 1.80 (br.s, 3H, Me¹¹),1.81-1.88 (m, 2H, Hₐ⁶ + Hₐ⁹), 1.93 (d, ⁴*J* = 0.4 Hz, Me¹⁸C = ₑₙₒₗ), 1.95 (m, 1H, Hₑ⁶), 2.07 (ddd, 1H, ²*J* = ³*J*₇ₐ₆ₐ = 14.5 Hz, ³*J*₇ₐ₆ₑ = 6.0 Hz, Hₐ⁷), 2.10 (dddd, 1H, ³*J*₅ₑ₉ₑ = ³*J*₅ₑ₆ₑ = 6.8 Hz,³*J*₅ₑ₉ₐ = ³*J*₅ₑ₆ₐ = 3.4 Hz, Hₑ⁵), 2.18 (ddd, 1H, ²*J* = 14.5 Hz, ³*J*₇ₑ₆ₑ = 6.0 Hz, Hₑ⁷), 2.25 (s, 3H, Me¹⁸C = O), 2.30 (br.ddd, 1H, ²*J* = 13.8 Hz, ³*J*₉ₑ₅ₑ = 6.8 Hz, ³*J*₉ₑ₁ₑ = 3.6 Hz, Hₑ⁹), 3.13 (d, 1H, ²*J* = 14.0 Hz, H¹²), 3.32 (dd, *ca*. 1H, ²*J* = 14.0 Hz, ⁴*J*_{12', 11} = 0.3 Hz, H'¹² ₖₑₜₒ), 3.33 (br.d, ²*J* = 14.0 Hz, H'¹² ₑₙₒₗ), total 1H; 3.41 (s, *ca*. 2H, C¹⁷H₂CO _{keto form}), 4.39 (br.d, *ca*. 1H, ³*J*₁ₑ₉ₑ = 3.6 Hz, Hₑ⁹ ₖₑₜₒ), 4.49 (br.d, ³*J*₁ₑ₉ₑ = 3.8 Hz, Hₑ⁹ ₑₙₒₗ), total 1H; 4.93 (br.s, HC = ₑₙₒₗ), 7.58 (m, 2H, 2H¹⁵), 7.66 (m, 1H, H¹⁶), 7.92 (m, 2H, 2H¹⁴), 12.04 (br.s, HO ₑₙₒₗ) (ratio keto/enol *ca*. 95:5 in *ca*. 2% solution in CDCl₃), ¹³C NMR (63 MHz, CDCl₃, δ): 22.89 (Me¹¹), 22.56 (Me¹⁰), 23.25 (C⁹H₂), 23.65 (C⁶H₂), 30.30 (Me¹⁸CO), 30.84 (C⁵H), 32.82 (C⁷H₂), 51.29 (C¹⁷H₂), 60.51 (C¹²H₂), 78.35 (C¹H), 83.03 (C⁴), 83.97 (C⁸), 127.62 (2C¹⁴H), 129.38 (2C¹⁵H), 133.91 (C¹⁶H), 141.12 (C¹³), 165.95 (OC²⁰=O), 200.58 (MeC¹⁹=O). DCI HRMS: obsd 411.14210, calcd for C₂₀H₂₇O₇S (M + 1) 411.14775.

### Example 17: Preparation of (1R,4R,5R,8R)-8-ethoxyoxalyloxy-4,8-dimethyl-4-phenylsulfonylmethyl-2,3-dioxabicyclo [3.3.1]nonane (25a) and (1R,4S,5R,8R)-8-ethoxyoxalyloxy-4,8-dimethyl-4-phenylsulfonylmethyl-2,3-dioxabicyclo [3.3.1]nonane (25b) (Scheme 8).

To a solution of sulfides **18a,b** (118 mg, 0.30 mmol) in EtOAc (4 mL) at 5°C was added a solution of MCPBA (216 mg of *ca*. 60%, 0.75 mmol) in EtOAc (3 mL). The mixture was stirred for 5 h at rt, poured into saturated NaHCO₃ (30 mL) and extracted with hexane-EtOAc (3:1, 3 x 40 mL). Organic extract was dried (Na₂SO₄) and evaporated. Flash chromatography (hexane-EtOAc, 75:25) of the residue gave a mixture of diastereomeric sulfones **25a,b** (120 mg, 94% yield, **25a /25b** *ca*. 57:43) as a colorless oil. The sulfones were separated by MPLC (hexane-EtOAc 75:25). Less polar isomer **25a**, a colorless oil. IR (neat): 2987, 2940, 1767 v.s, 1737 v.s, 1448 v.s, 1376 v.s, 1310, 1187 v.s, 1152 v.s, 1095 v.s, 1086 v.s, 1013 v.s, 751, 724 v.s, 690 cm⁻¹. ¹H NMR (CDCl₃, 400 MHz, δ): 1.37 (t, 3H, ³*J* = 7.1 Hz, Me¹⁸CH₂), 1.53 (br.s, 3H, Me¹¹), 1.72 (s, 3H, Me¹⁰), 1.82 (dddd, 1H, ²*J* = 14.2 Hz, ³*J*₆ₐ₇ₐ = 13.2 Hz, ³*J*₆ₐ₇ₑ = 6.8 Hz, ³*J*₆ₐ₅ₑ = 3.5 Hz, Hₐ⁶), 1.94 (ddddd, 1H, ²*J* = 14.2 Hz, ³*J*₆ₑ₇ₑ = 5.2 Hz, ³*J*₆ₑ₇ₐ = ³*J*₆ₑ₅ₑ = ⁴*J*₆ₑ₉ₑ = 2.6 Hz, Hₑ⁶), 1.97 (ddd, 1H, ²*J* = 14.0 Hz, ³*J*₉ₐ₅ₑ = 2.6 Hz, ³*J*₉ₐ₁ₑ = 1.4 Hz, Hₐ⁹), 2.24-2.37 (m, 4H, Hₑ⁵ +Hₑ⁷ + Hₐ⁷ +Hₑ⁹), 3.27 (d, 1H, ²*J* = 14.3 Hz, H¹²), 4.21 (br.d, 1H, ²*J* = 14.3 Hz, H'¹²), 4.33 (q, 2H, ³*J* = 7.1 Hz, C¹⁷H₂O), 4.36 (br.dd, 1H, ³*J*₁ₑ₉ₑ = 3.6 Hz, ³*J*₁ₑ₉ₐ = 1.4 Hz, Hₑ¹), 7.59 (m, 2H, 2H¹⁵), 7.67 (m, 1H, H¹⁶), 7.94 (m, 2H, 2H¹⁴). ¹³C NMR (100 MHz, CDCl₃, δ): 13.92 (Me¹⁸CH₂), 22.34 (Me¹⁰), 23.05 (Me¹¹), 23.23 (C⁶H₂), 24.46 (C⁹H₂), 29.32 (C⁵H), 32.49 (C⁷H₂), 60.96 (C¹²H₂), 63.03 (C¹⁷H₂), 77.74 (C¹H), 82.91 (C⁴), 86.58 (C⁸), 127.57 (2C¹⁴H), 129.41 (2C¹⁵H), 133.83 (C¹⁶H), 141.03(C¹³), 156.66 (C=O), 158.08 (C=O). Anal. Calcd for C₂₀H₂₆O₈S: S, 7.52. Found: S, 7.13. More polar isomer **25b**, a colorless oil. IR (neat): 1763 v.s, 1740 v.s, 1448, 1376, 1322 v.s, 1197 v.s, 1181 v.s, 1154 v.s, 1087, 1014 v.s, 734 v.s, 690 cm⁻¹. ¹H NMR (400 MHz, CDCl₃, δ): 1.36 (t, 3H, ³*J* = 7.1 Hz, Me¹⁸CH₂), 1.66 (s, 3H, Me¹⁰), 1.80-1.89 (m, 1H, Hₐ⁶), 1.82 (br.s, 3H, Me¹¹), 1.88 (ddd, 1H, ²*J* = 14.2 Hz, ³*J*₉ₐ₅ₑ = 3.4 Hz, ³*J*₉ₐ₁ₑ = 1.8 Hz, Hₐ⁹), 1.99 (m, 1H, Hₑ⁶), 2.13 (m, 1H, Hₑ⁵), 2.14 (ddd, 1H, ²*J* = ³*J*₇ₐ₆ₐ = 14.6 Hz, ³*J*₇ₐ₆ₑ = 2.0 Hz, Hₐ⁷), 2.33 (br.dd, 1H, ²*J* = 14.6 Hz, ³*J*₇ₑ₆ₑ = 6.2 Hz, Hₑ⁷), 2.34 (ddd, 1H, ²*J* = 14.2 Hz, ³*J*₉ₑ₅ₑ = 6.4 Hz, ³*J*₉ₑ₁ₑ = 3.6 Hz, Hₑ⁹), 3.12 (d, 1H, ²*J* = 14.0 Hz, H¹²), 3.32 (br.d, 1H, ²*J* = 14.0 Hz, H'¹²), 4.32 (q, 2H, ³*J* = 7.1 Hz, C¹⁷H₂O), 4.34 (m, 1H, Hₑ¹), 7.57 (m, 2H, H^{15, 15'}), 7.67 (m, 1H, H¹⁶), 7.92 (m, 2H, H^{14, 14'}); ¹³C NMR (63 MHz, CDCl₃, δ): 13.87 (Me¹⁸), 21.87 (Me¹¹), 22.29 (Me¹⁰), 23.30 (C⁹H₂), 23.53 (C⁶H₂), 30.70 (C⁵H), 32.14 (C⁷H₂), 60.41 (C¹²H₂), 63.01 (C¹⁷H₂O), 78.26 (C¹H), 83.12 (C⁴), 86.31 (C⁸), 127.58 (2C¹⁴H), 129.37 (2C¹⁵H), 133.92 (C¹⁶H), 141.02 (C¹³), 156.56 (C=O), 158.00 (C=O).

### Example 18: Preparation of (1R,4R,5R,8R)-8-dibenzylaminooxalyloxy-4,8-dimethyl-4-phenylsulfonylmethyl-2,3-dioxabicyclo[3.3.1]nonane (26a) and (1R,4S,5R,8R)-dibenzylaminooxalyloxy-4,8-dimethyl-4-phenylsulfonylmethyl-2,3-dioxabicyclo[3.3.1]nonane (26b) (Scheme 8).

To a solution of sulfides **19 a,b** (137 mg, 0.25 mmol) in EtOAc (5mL) was added a solution of MCPBA (201 mg of *ca*. 60%, 0.7 mmol) in EtOAc (2mL). The mixture was stirred for 5 h at rt, poured into saturated NaHCO₃ (30 mL) and extracted with hexane-EtOAc (1:1, 3 x 50 mL). The organic extract was dried (Na₂SO₄) and evaporated. Flash chromatography (hexane-EtOAc, 75:25) of the residue afforded the diastereomers **26a,b** (143.5 mg, 99% yield, **26a/26b***ca*. 60:40) as a colorless oil. The sulfones were separated by MPLC (hexane-EtOAc, 70:30). Less polar isomer **26a,** a colorless glass; IR (neat): 1736 v.s, 1670 v.s, 1653 v.s, 1496, 1453 v.s, 1377, 1318 v.s, 1272, 1181 v.s, 1171 v.s, 1150 v.s, 1083 v.s, 1013 , 912, 723 v.s, 700 v.s, 690 v.s. cm⁻¹. ¹H NMR (CDCl₃, 400 MHz, δ): 1.50 (br.s, 3H, Me¹¹), 1.67 (s, 3H, Me¹⁰), 1.72 (m, 1H, Hₐ⁶), 1.82-1.92 (m, 2H, Hₑ⁶ + Hₐ⁹), 2.18-2.28 (m, 4H, Hₑ⁵ +Hₑ⁷ +Hₐ⁷ +Hₑ⁹), 3.27 (d, 1H, ²*J* = 14.3 Hz, H¹²), 4.17 (br.d, 1H, ²*J* = 14.3 Hz, H'¹²), 4.35 (br.s, 2H, NC¹⁷H₂), 4.36 (m, 1H, Hₑ¹), 4.50 (br.s, 2H, NC¹⁸H₂), 7.19-7.26 (m, 4H), 7.31-7.41 (m, 6H), 7.59 (m, 2H, 2H¹⁵), 7.67 (m, 1H, H¹⁶), 7.94 (m, 2H, 2H¹⁴). ¹³C NMR (100 MHz, CDCl₃, δ): 22.46 (Me¹⁰), 22.93 (Me¹¹), 23.12 (C⁶H₂), 24.33 (C⁹H₂), 29.25 (C⁵H), 32.90 (C⁷H₂), 46.22 (NC¹⁷H₂), 50.02 (NC¹⁸H₂), 60.89 (C¹²H₂), 77.60 (C¹H), 82.78 (C⁴), 86.51 (C⁸), 127.38 (2CH), 127.53 (2C¹⁴H), 127.87 (CH), 128.25 (CH), 128.46 (2CH), 128.78 (2CH), 128.93 (2CH), 129.36 (2C¹⁵H), 133.79 (C¹⁶H), 134.81 (Cq), 135.34 (Cq), 140.98(C¹³), 162.14 (C = O), 162.18 (C = O). Anal. Calcd for C₃₂H₃₅NO₇S: C, 66.53; H, 6.11; N, 2.42; S, 5.55. Found: C, 65.36; H, 6.09; N, 2.10, S, 5.28. More polar isomer **26b**, a colorless glass. IR (neat): 2982, 2935 v.s, 1733 v.s, 1663 v.s, 1654 v.s, 1450 v.s, 1379, 1322 v.s, 1312 v.s, 1176 v.s, 1153 v.s, 1084, 1015 , 913, 734 v.s, 700 cm⁻¹. ¹H NMR (400 MHz, CDCl₃, δ): 1.61 (s, 3H, Me¹⁰), 1.76 (m, 1H, Hₐ⁹), 1.77 (m, 1H, Hₐ⁹), 1.79 (s, 3H, Me¹¹), 1.92 (m, 1H, Hₑ⁶), 2.03 (dddd, 1H, ³*J*₅ₑ₆ₑ = ³*J*₅ₑ₉ₑ = 6.4 Hz, ³*J*₅ₑ₆ₐ = ³*J*₅ₑ₉ₐ = 3.2 Hz, Hₑ⁵), 2.09 (ddd, 1H, ²*J* = ³*J*₇ₐ₆ₐ = 14.2 Hz, ³*J*₇ₐ₆ₑ = 6.0 Hz, Hₐ⁷), 2.23 (br.dd, 1H, ²*J* = 14.6 Hz, ³*J*₇ₑ₆ₑ = 5.8 Hz, Hₑ⁷), 2.27 (ddd, 1H, ²*J* = 13.8 Hz, ³*J*₉ₑ₅ₑ = 6.4 Hz, ³*J*₉ₑ₁ₑ = 3.3 Hz, Hₑ⁹), 3.11 (d, 1H, ²*J* = 14.0 Hz, H¹²), 3.29 (br.d, 1H, ²*J* = 14.0 Hz, H'¹²), 4.34 (m, 1H, Hₑ¹), 4.35 (br.s, 2H, NC¹⁷H₂), 4.485 and 4.51 (ABq, 2H, ²*J* = 14.7 Hz, NC¹⁸H₂), 7.19-7.24 (m, 4H), 7.30-7.40 (m, 6H), 7.57 (m, 2H, 2H¹⁵), 7.66 (m, 1H, H¹⁶), 7.92 (m, 2H, 2H¹⁴). ¹³C NMR (100 MHz, CDCl₃, δ): 21.84 (Me¹¹), 22.49 (Me¹⁰), 23.24 (C⁹H₂), 23.50 (C⁶H₂), 30.71 (C⁵H), 32.71 (C⁷H₂), 46.27 (NC¹⁷H₂), 50.04 (NC¹⁸H₂),60.49 (C¹²H₂), 78.19 (C¹H), 83.11 (C⁴), 86.30 (C⁸), 127.36 (2CH), 127.63 (2C¹⁵H), 127.90 (CH), 128.26 (CH), 128.48 (2CH), 128.80 (2CH), 128.95 (2CH), 129.38 (2C¹⁴H), 133.93 (C¹⁶H), 134.84 (Cq), 135.35 (Cq), 141.09(C¹³), 162.10. (C=O), 162.21 (C=O).

### Example 19: Preparation of (1R,4R,5R,8R)-4,8-dimethyl-8-phenoxycarbonyloxy-4-phenylsulfonylmethyl-2,3-dioxabicyclo[3.3.1]nonane (27a) and (1R,4S, 5R,8R)-4,8-dimethyl-8-phenoxycarbonyloxy-4-phenylsulfonylmethyl-2,3-dioxabicyclo[3.3.1]-nonane (27b) (Scheme 8).

The mixture of diastereomeric (1*R*,4*R*, 5*R*,8*R*)-4,8-dimethyl-8-phenoxycarbonyloxy-4-phenylthiomethyl-2,3-dioxabicyclo [3.3.1]nonane **20a** and (1*R*,4*S*,5*R*,8*R*)-4,8-dimethyl-8-phenoxycarbonyloxy-4-phenylthiomethyl-2,3-dioxabicyclo[3.3.1]nonane **20b** (75 mg, 0.181 mmol, 25% yield, ratio **20a/20b** *ca*. 56:44) was prepared by phenoxycarbonylation of hydroxysulfides **2a,b.** The sulfides **20a,b,** a colorless solid, mp 64-67°C. ¹H NMR (CDCl₃, 250 MHz, δ): 1.29 (br.s, Me¹¹ **20a**) and 1.61 (br.s, Me¹¹ **20b**), total 3H; 1.777 (br.s, Me¹⁰ **20b**) and 1.784 (br.s, Me¹⁰ **20a**), total 3H; 1.80-2.05 (m, 4H); 2.18 (ddd, ²*J* = 13.8 Hz, ³*J*₉ₑ₅ₑ = 5.8 Hz, ³*J*₉ₑ₁ₑ = 3.5 Hz, Hₑ⁹ 20a), 2.29-2.48 (m), total 3H; 3.00 and 3.06 (br.) (ABq, ²*J* = 12.1 Hz, H¹² + H'¹² **20b**), 3.36 (d, ²*J* = 12.9 Hz, H¹² **20a**) and 3.75 (br.d, ²*J* = 12.9 Hz, H'¹² **20a**), total 2H; 4.41 (dd, ³*J*₁ₑ₉ₑ = 3.5 Hz, ³*J*₁ₑ₉ₐ = 1.1 Hz, Hₑ¹ **20a**) and 4.44 (dd, ³*J*₁ₑ₉ₑ = 3.9 Hz, ³*J*₁ₑ₉ₐ = 1.4 Hz, Hₑ¹ **20b**), total 1H; 7.16-7.48 (m, 10H). ¹³C NMR (CDCl₃, 63 MHz) (δ): Isomer **20a:** 22.01 (Me¹¹), 22.28 (Me¹⁰), 23.37 (C⁶H₂), 23.95 (C⁹H₂), 28.47 (C⁵H), 32.38 (C⁷H₂), 40.73 (C¹²H₂), 77.50 (C¹H), 84.03 (C⁴), 85.36 (C⁸), 121.07 (2C^{*o*-phen.}H), 125.90 (C^{*p*-phen.}H), 126.33 (C¹⁶H), 128.95 (2C¹⁴H), 129.39 (2C^{*m*-phen.}H), 129.84 (2C¹⁵H), 136.66 (C¹³), 150.88 (C^{1-phen.}), 151.37 (C = O) ; Isomer **20b**: 21.78 (Me¹¹), 22.28 (Me¹⁰), 23.08 (C⁶H₂), 24.08 (C⁹H₂), 29.80(C⁵H), 32.70 (C⁷H₂), 40.83 (C¹²H₂), 77.95 (C¹H), 84.03 (C⁴), 85.29 (C⁸), 121.07 (2C^{*o*-phen.}H), 125.90 (C^{*p*-phen.}H), 126.52 (C¹⁶H), 129.00 (2C¹⁴H), 129.39 (2C^{*m*-phen.}H), 129.84 (2C¹⁵H), 136.22 (C¹³), 150.88 (C^{1-phen.}), 151.37 (C=O).

To a solution of sulfides **20a,b** (60 mg, 0.145 mmol) in EtOAc (1.2 mL) was added MCPBA (108 mg of *ca.* 60%, 0.38 mmol). The mixture was stirred for 6 h at rt, diluted with hexane-EtOAc (3:1, 50 mL), washed with saturated NaHCO₃ (2 x 10 mL) and the water washings were extracted with hexane-EtOAc (3:1, 50 mL). The combined organic extract was dried (Na₂SO₄ + NaHCO₃) and evaporated. MPLC (hexane-EtOAc, 80:20) of the residue afforded the diastereomeric sulfones **27a** (31.7 mg) and **27b** (24.5 mg) (total 60.2 mg, 93% yield). Less polar isomer **27a,** a colorless solid, mp 99-100°C, R_{f} = 0.30 (hexane-EtOAc, 75:25). ¹H NMR (CDCl₃, 400 MHz, δ): 1.54 (br.s, 3H, Me¹¹), 1.72 (s, 3H, Me¹⁰), 1.83-1.99 (m, 2H, Hₑ⁶ + Hₐ⁶), 2.02 (ddd, 1H, ²*J* = 13.8 Hz, ³*J*₉ₐ₅ₑ = 2.8 Hz, ³*J*₉ₐ₁ₑ = 1.6 Hz, Hₐ⁹), 2.28-2.39 (m, 4H, Hₑ5 +Hₑ⁷ +Hₐ⁷ +Hₑ⁹), 3.28 (d, 1H, ²*J* = 14.3 Hz, H¹²), 4.23 (br.d, 1H, ²*J* = 14.3 Hz, H'¹²), 4.42 (br.dd, 1H, ³*J*₁ₑ₉ₑ = 4.0 Hz, ³*J*₁ₑ₉ₐ = 1.6 Hz, Hₑ¹), 7.15 (m, 2H, 2H^{*o*-phen.}), 7.24 (m, 1H, H^{*p*-phen.}), 7.39 (m, 2H, 2H^{*m*-phen.}), 7.59 (m, 2H, 2H¹⁵), 7.67 (m, 1H, H¹⁶), 7.94 (m, 2H, 2H¹⁴). ¹³C NMR (100 MHz, CDCl₃, δ): 22.25 (Me¹⁰), 23.08 (Me¹¹), 23.27 (C⁶H₂), 24.51 (C⁹H₂), 29.38 (C⁵H), 32.75 (C⁷H₂), 60.99 (C¹²H₂), 77.86 (C¹H), 82.84 (C⁴), 85.12 (C⁸), 121.10 (2C^{*o*-phen.}H), 126.00 (C^{*p*-phen.}H), 127.57 (2C¹⁴H), 129.40 (2C¹⁵H), 129.39 (2C^{*m*-phen.}H), 133.81 (C¹⁶H), 141.05(C¹³), 150.89 (C^{1-phen.}), 151.43(C = O). Anal. Calcd for C₂₃H₂₆O₇S: C, 61.87; H, 5.87; S, 7.18. Found: C, 62.21; H,5.76; S,6.95. More polar isomer **27b**, a colorless solid, mp 109-111°C, R_{f} = 0.26 (hexane-EtOAc, 75:25). ¹H NMR (CDCl₃, 400 MHz, δ): 1.66 (s, 3H, Me¹⁰), 1.84 (br.s, 3H, Me¹¹), 1.87-1.97 (m, 2H, Hₑ⁶ + Hₐ⁹), 2.02 (m, 1H, Hₑ⁶), 2.15 (ddd, 1H, ²*J* = ³*J*₇ₐ₆ₐ = 15.0 Hz, ³*J*₇ₐ₆ₑ = 6.0 Hz, Hₐ⁷), 2.18-2.39 (m, 1H, Hₑ⁵), 2.31 (br.dd, 1H, ²*J* = 15.0 Hz, ³*J*₇ₑ₆ₑ = 6.0 Hz, Hₑ⁷), 2.37 (ddd, 1H, ²*J* = 13.8 Hz, ³*J*₉ₑ₅ₑ = 6.4 Hz, ³*J*₉ₑ₁ₑ = 3.5 Hz,Hₑ⁹), 3.14 (d, 1H, ²*J* = 14.0 Hz, H¹²), 3.34 (br.d, 1H, ²*J* = 14.0 Hz, H'¹²), 4.40 (br.dd, 1H, ³*J*₁ₑ₉ₑ = 3.5 Hz, Hₑ¹), 7.15 (m, 2H, 2H^{*o*-phen.}), 7.24 (m, 1H, H^{*p*-phen.}), 7.38 (m, 2H, 2H^{*m*-phen.}), 7.59 (m, 2H, 2H¹⁵), 7.68 (m, 1H, H¹⁶), 7.94 (m, 2H, 2H¹⁴). ¹³C NMR (63 MHz, CDCl₃, δ): 21.94 (Me¹¹), 22.28 (Me¹⁰), 23.41 (C⁹H₂), 23.67 (C⁶H₂), 30.83 (C⁵H), 32.49 (C⁷H₂), 60.50 (C¹²H₂), 78.47 (C¹H), 83.17 (C⁴), 84.93 (C⁸), 121.09 (2C^{*o*-phen.}H), 126.01 (C^{*p*-phen.}H), 127.65 (2C¹⁴H), 129.42 (2C¹⁵H), 129.47 (2C^{*m*-phen.}H), 133.95 (C¹⁶H), 141.15(C¹³), 150.89 (C^{1-phen.}), 151.42(C=O). Anal. Calcd. for C₂₃H₂₆O₇S: C, 61.87; H, 5.87; S, 7.18. Found: C, 61.78; H, 5.98; S, 7.19.

### Example 20: Preparation of (1S,4S,5S,8S)-4,8-dimethyl-4-phenylsulfonylmethyl-2,3-dioxabicyclo [3.3.1]nonan-8-ol (21'a) and (1S,4R,5S,8S)-4,8-dimethyl-4-phenylsulfonylmethyl-2,3-dioxabicyclo[3.3.1]nonan-8-ol (21'b) (Scheme 9).

The title compounds were prepared by oxidation of sulfides **2'a,b** as described in example 13 for the corresponding (1*R*, 5*R*, 8*R*)-epimers **21a,b**. ¹H NMR spectra in CDCl₃ of individual diastereomers **21'a,b** and **21a,b** (example 13) are identical. **21'a** is a colorless solid, mp 112-113°C (hexane-EtOAc), **21'b** is a colorless oil, total yield of **21'a,b** 90%.

### Example 21: Preparation of 1S,4S,5S,8S)-8-acetoxy-4,8-dimethyl-4 phenylsulfonylmethyl-2,3-dioxabicyclo[3.3.1]nonane (23'a) and (1S,4R,5S,8S)-8-acetoxy-4,8-dimethyl-4-phenylsulfonylmethyl-2,3-dioxabicyclo[3.3.1]nonane (23'b) (Scheme 9).

To TMS-derivative **16'a,b**, prepared from **2'a,b** (0.854 mmol, 252 mg) and TMSOTf as described in example 10, was added AcCl (2.0 mL) and the mixture was stirred at rt for 50 h. The mixture was evaporated and the crude acetyloxysulfide **13'a,b** was dissolved in EtOAc (8 mL) and oxidized by MCPBA (526 mg of *ca*. 70%, 2.135 mmol) at rt for 6 h. Workup as in example 15 followed by flash chromatography (silica gel, hexane-EtOAc 3:1) afforded a mixture of sulfones **23'a,b** (265 mg, 84%). The mixture was separated by MPLC (hexane-EtOAc 3:1) to give the individual epimers **23'a** as a colorless solid, mp 96-98°C and **23'b**, as a colorless solid, mp 101-102°C. NMR Spectra are consistent with those described above for (1*R*, 5*R*, 8*R*)-epimers **23a** and **23b** (example 15).

### Example 22: Preparation of (1S,4S,5S,7S,8S)-7-benzoyloxy-4,8-dimethyl-4-phenylsulfonylmethyl-2,3-dioxabicyclo[3.3.1]nonan-8-ol (29a) and (1S,4R,5S,7S,8S)-7-benzoyloxy-4,8-dimethyl-4-phenylsulfonylmethyl-2,3-dioxabicyclo[3.3.1]nonan-8-ol (29b) (Scheme 9).

To a solution of sulfides **4a** and **4b** (80 mg, 0.193 mmol) in EtOAc (2 mL) at 0°C was added a solution of MCPBA (94 mg of *ca*. 85%, 0.46 mmol) in EtOAc (2 mL). The mixture was stirred for 2 h at 0-5°C and for 4 h at rt, diluted with EtOAc (10 mL) and washed with saturated NaHCO₃ (3 x 5 mL).Water washings were extracted with EtOAc (2 x 10 mL), the combined organic extract was dried (Na₂SO₄) and evaporated. Flash chromatography (hexane-EtOAc, 65:35) of the residue gave a mixture of diastereomeric sulfones **29a** and **29b** (80.5 mg, 93% yield) as colorless oil. The diastereomers were separated by MPLC (hexane-EtOAc, 60:40). Less polar isomer **29b,** a colorless solid, mp 132-133°C, R_{f} 0.42 (hexane-EtOAc, 60:40). ¹H NMR (400 MHz, CDCl₃, δ): 1.36 (s, 3H, Me¹⁰), 1.90 (br.s, 3H, Me¹¹), 1.93 (ddd, 1H, ²*J* = 13.8 Hz, ³*J*₆ₐ₇ₐ = 12.0 Hz, ³*J*₆ₐ₅ₑ = 3.5 Hz, Hₐ⁶), 2.16 (ddd, 1H, ²*J* = 13.8 Hz, ³*J*₉ₐ₅ₑ = 3.3 Hz, ³*J*₉ₐ₁ₑ = 2.0 Hz, Hₐ⁹), 2.30 (dddd, 1H, ²*J* = 13.8 Hz, ³*J*₉ₑ₁ₑ = ³*J*₉ₑ₅ₑ = 3.6 Hz, ⁴*J*₉ₑ₆ₑ = 2.9 Hz, Hₑ⁹), 2.34 (br.s., 1H, OH), 2.43 (ddddd, 1H, ³*J*₅ₑ₉ₑ = 3.6 Hz, ³*J*₅ₑ₆ₐ = 3.5 Hz, ³*J*₅ₑ₉ₐ = 3.3 Hz, ³*J*₅ₑ₆ₑ = 2.9 Hz, ⁴*J*₅ₑ₁ₑ = 1.5 Hz, Hₑ⁵), 2.57 (dddd, 1H, ²*J* = 13.8 Hz, ³*J*₆ₑ₇ₐ = 6.1 Hz, ³*J*₆ₑ₅ₑ = ⁴*J*₆ₑ₉ₑ = 2.9 Hz, Hₑ⁶), 3.16 (d, 1H, ²*J* = 13.9 Hz, H¹²), 3.58 (br.d, 1H, ²*J* = 13.9 Hz, H'¹²), 3.97 (ddd, 1H, ³*J*₁ₑ₉ₑ = 3.6 Hz, ³*J*₁ₑ₉ₐ = 2.0 Hz, ⁴*J*₁ₑ₅ₑ = 1.5 Hz, Hₑ¹), 5.57 (dd, 1H, ³*J*₇ₐ₆ₐ = 12.0 Hz, ³*J*₇ₐ₆ₑ = 6.1 Hz, Hₐ⁷), 7.50 (m, 2H), 7.58-7.63 (m, 3H), 7.67 (m, 1H), 7.97-8.05 (m, 4H). ¹³C NMR (100 MHz, CDCl₃, δ): 21.71 (Me¹¹), 23.27 (C⁹H₂), 24.31 (Me¹⁰), 29.49 (C⁶H₂), 33.61 (C⁵H), 60.24 (C¹²H₂), 72.70 (C⁸), 74.58 (C⁷H), 82.91 (C⁴), 83.80 (C¹H), 127.79 (2C¹⁴H), 128.55 (2CH^{m-benz.}), 129.39 (2C¹⁵H), 129.49 (2CH^{o-benz.}), 129.97 (C^{1-benz.}), 133.30 (CH^{p-benz.}), 133.91 (C¹⁶H), 141.02 (C¹³), 165.69 (C = O). Anal. Calcd. for C₂₃H₂₆O₇S: C, 61.87; H, 5.87; S 7.18. Found: C, 61.60; H 5.91; S 7.43. More polar isomer **29a**, a colorless solid, mp 182-183°C, R_{f} 0.36 (hexane-EtOAc, 60:40). ¹H NMR (400 MHz, CDCl₃, δ): 1.41 (s, 3H, Me¹⁰), 1.64 (br.s, 3H, Me¹¹), 1.88 (ddd, 1H, ²*J* = 13.3 Hz, ³*J*₆ₐ₇ₐ = 11.8 Hz, ³*J*₆ₐ₅ₑ = 3.6 Hz, Hₐ⁶), 2.25 (ddd, 1H, ²*J* = 14.0 Hz, ³*J*₉ₐ₁ₑ = ³*J*₉ₐ₅ₑ = 2.8 Hz, Hₐ⁹), 2.28 (dddd, 1H, ²*J* = 14.0 Hz, ³*J*₉ₑ₅ₑ = 3.2 Hz, ³*J*₉ₑ₁ₑ = 2.8 Hz, ⁴*J*₉ₑ₆ₑ = 2.2 Hz, Hₑ⁹), 2.38 (br.s., 1H, OH), 2.39 (dddd, 1H, ²*J* = 13.3 Hz, ³*J*₆ₑ₇ₐ = 6.2 Hz, ³*J*₆ₑ₅ₑ = 3.2 Hz, ⁴*J*₆ₑ₉ₑ = 2.2 Hz, Hₑ⁶), 2.61 (br.dddd, 1H, ³*J*₅ₑ₆ₐ = 3.6 Hz, ³*J*₅ₑ₉ₑ = ³*J*₅ₑ₉ₐ = ³*J*₅ₑ₆ₑ = 3.2 Hz, Hₑ⁵), 3.31 (d, 1H, ²*J* = 14.3 Hz, H¹²), 4.23 (br.d, 1H, ²*J* = 14.3 Hz, H'¹²), 3.97 (br.dd, 1H, ³*J*₁ₑ₉ₑ = ³*J*₁ₑ₉ₐ = 2.8 Hz, Hₑ¹), 5.71 (dd, 1H, ³*J*₇ₐ₆ₐ = 11.8 Hz, ³*J*₇ₐ₆ₑ = 6.2 Hz, Hₐ⁷), 7.48 (m, 2H), 7.58-7.62 (m, 3H), 7.68 (m, 1H), 7.96(m, 2H), 8.04 (m, 2H). ¹³C NMR (100 MHz, CDCl₃, δ): 23.28 (Me¹¹), 24.19 (C⁹H₂), 24.41 (Me¹⁰), 29.13 (C⁶H₂), 32.10 (C⁵H), 60.87(C¹²H₂), 72.71 (C⁸), 74.51 (C⁷H), 82.51 (C⁴), 83.14 (C¹H), 127.59 (2C¹⁴H), 128.56 (2CH^{m-benz.}), 129.42 (2C¹⁵H), 129.51 (2CH^{o-benz.}), 129.93 (C^{1-benz.}), 133.31(CH^{p-benz.}), 133.87 (C¹⁶H), 140.96 (C¹³), 165.61 (C=O). Anal. Calcd for C₂₃H₂₆O₇S: C, 61.87; H, 5.87; S 7.18. Found: C, 61.60; H 5.81; S 6.87.

### Example 23: Preparation of (1S,4S,5S,7S,8S)-7-benzoyloxy-4-n-butylsulfonylmethyl-4,8-dimethyl-2,3-dioxabicyclo[3.3.1]nonan-8-ol (30a) and (1S,4R,5S,7S,8S)-7-benzoyloxy-4-n-butylsulfonylmethyl-4,8-dimethyl-2,3-dioxabicyclo [3.3.1]nonan-8-ol (30b) (Scheme 9).

To a solution of sulfides **5a** and **5b** (37.5 mg, 0.095 mmol, **5a/5b** ∼ 55:45) in EtOAc (1 mL) at 0°C was added a solution of MCPBA (49 mg of *ca*. 85%, 0.24 mmol) in EtOAc (1.5 mL). The mixture was stirred for 3 h at 0-5°C and for additional 3 h at rt, diluted with EtOAc (10 mL) and washed with saturated NaHCO₃ (2 x 10 mL). Water washings were extracted with EtOAc (15 mL), organic extract was dried (Na₂SO₄, NaHCO₃) and evaporated. Flash chromatography (hexane-EtOAc, 70:30) of the residue afforded the separated diastereomeric sulfones **30a** (19.5 mg) and **30b** (17.2 mg) (total 36.7 mg, 91% yield). Less polar isomer **30b**, a colorless oil, R_{f} 0.36 (hexane-EtOAc, 60:40). ¹H NMR (400 MHz, CDCl₃, δ): 0.99 (t, 3H, ³*J* = 7.4 Hz, Me¹⁶CH₂), 1.44 (s, 3H, Me¹⁰), 1.50 (tq, 2H, ³*J* = ³*J* = 7.4 Hz, MeC¹⁵H₂CH₂),1.86-1.95 (m, 3H, Hₐ⁶ + CH₂C¹⁴H₂CH₂S), 1.88 (s, 3H, Me¹¹), 2.16 (ddd, 1H, ²*J* = 13.0 Hz, ³*J*₉ₐ₁ₑ = 2.5 Hz, ³*J*₉ₐ₅ₑ = 1.9 Hz, Hₐ⁹), 2.28 (ddddd, 1H, ³*J*₅ₑ₉ₑ = 3.5 Hz, ³*J*₅ₑ₆ₑ = 2.9 Hz, ³*J*₅ₑ₆ₐ = 2.5 Hz, ³*J*₅ₑ₉ₐ = 1.9 Hz, ⁴*J*₅ₑ₁ₑ = 1.8 Hz, Hₑ⁵), 2.31 (dddd, 1H, ²*J* = 13.0 Hz, ³*J*₉ₑ₅ₑ = 3.5 Hz, ³*J*₉ₑ₁ₑ = 3.3 Hz, ⁴*J*₉ₑ₆ₑ = 2.9 Hz, Hₑ⁹), 2.32 (br.s., 1H, OH), 2.42 (dddd, 1H, ²*J* = 13.8 Hz, ³*J*₆ₑ₇ₐ = 6.1 Hz, ³*J*₆ₑ₅ₑ = ⁴*J*₆ₑ₉ₑ = 2.9 Hz, Hₑ⁶), 3.08 (m, 2H, CH₂C¹³H₂S), 3.17 (d, 1H, ²*J* = 13.8 Hz, H¹²), 3.37 (br.d, 1H, ²*J* = 13.8 Hz, H'¹²), 4.00 (ddd, 1H, ³*J*₁ₑ₉ₑ = 3.5 Hz, ³*J*₁ₑ₉ₐ = 2.5 Hz, ⁴*J*₁ₑ₅ₑ = 1.8 Hz, Hₑ¹), 5.68 (dd, 1H, ³*J*₇ₐ₆ₐ = 12.0 Hz, ³*J*₇ₐ₆ₑ = 6.1 Hz, Hₐ⁷), 7.48 (m, 2H, 2HC^{m-benz.}), 7.61 (m, 1H, HC^{p-benz.}), 8.02 (m, 2H, 2HC^{o-benz.}). ¹³C NMR (63 MHz, CDCl₃, δ): 13.52 (Me¹⁶), 21.16 (Me¹¹), 21.68 (C¹⁵H₂), 23.23 (C⁹H₂), 24.13 (C¹⁴H₂), 24.36 (Me¹⁰), 29.33 (C⁶H₂), 33.79 (C⁵H), 56.19 (C¹³H₂), 56.57 (C¹²H₂), 72.71 (C⁸), 74.44 (C⁷H), 82.53 (C⁴), 83.73 (C¹H), 128.60 (2CH^{m-benz.}), 129.49 (2CH^{o-benz.}), 129.87 (C^{1--benz.}), 133.36 (CH^{p-benz.}), 165.79 (C = O). More polar isomer **30a**, a colorless solid, mp 140-142°C, R_{f} 0.25 (hexane-EtOAc, 60:40). ¹H NMR (400 MHz, CDCl₃, δ): 0.98 (t, 3H, ³*J* = 7.4 Hz, Me¹⁶CH₂), 1.43 (s, 3H, Me¹⁰), 1.49 (tq, 2H, ³*J* = ³*J* = 7.4 Hz, MeC¹⁵H₂CH₂), 1.64 (s, 3H, Me¹¹), 1.83-1.91 (m, 2H, CH₂C¹⁴H₂CH₂S), 1.85 (ddd, ²*J* = 13.5 Hz, ³*J*₆ₐ₇ₐ = 11.7 Hz, ³*J*₆ₐ₅ₑ = 3.5 Hz, Hₐ⁶), 2.25 (m, 2H, Hₐ⁹ + Hₑ⁹), 2.31 (m, 1H, ³*J*₅ₑ₆ₐ = ³*J*₅ₑ₉ₑ = 3.5 Hz, ³*J*₅ₑ₆ₑ = ³*J*₅ₑ₉ₐ = 2.5 Hz, Hₑ⁵), 2.40 (br.s., 1H, OH), 2.38 (ddddd, 1H, ²*J* = 13.5 Hz, ³*J*₆ₑ₇ₐ = 6.1 Hz, ³*J*₆ₑ₅ₑ = 2.5 Hz, ⁴*J*₆ₑ₉ₑ = *J* = 1.6 Hz, Hₑ⁶), 3.08 (m, 2H, CH₂C¹³H₂S), 3.50 (d, 1H, ²*J* = 14.3 Hz, H¹²), 3.76 (br.d, 1H, ²*J* = 13.8 Hz, H'¹²), 4.01 (br.dd, 1H, ³*J*₁ₑ₉ₑ = ³*J*₁ₑ₉ₐ = 2.5 Hz, Hₑ¹), 5.72 (dd, 1H, ³*J*₇ₐ₆ₐ = 11.7 Hz, ³*J*₇ₐ₆ₑ = 6.2 Hz, Hₐ⁷), 7.48 (m, 2H, 2HC^{m-benz.}), 7.60 (m, 1H, HC^{p-benz.}), 8.03 (m, 2H, 2HC^{o-benz.}). ¹³C NMR (63 MHz, CDCl₃, δ): 13.55 (Me¹⁶), 21.70 (C¹⁵H₂), 22.46 (Me¹¹), 23.86 (C¹⁴H₂), 24.12 (C⁹H₂), 24.40 (Me¹⁰), 29.06 (C⁶H₂), 32.84 (C⁵H), 55.41 (C¹³H₂), 56.64 (C¹²H₂), 72.71 (C⁸), 74.36 (C⁷H), 81.95 (C⁴), 83.12 (C¹H), 128.57 (2CH^{m-benz.}), 129.51 (2CH^{o-benz.}), 129.90 (C^{1-benz.}), 133.33 (CH^{p-benz.}) 165.58 (C=O). Anal. Calcd for C₂₁H₃₀O₇S: C, 59.14; H, 7.09; S, 7.52. Found: C, 59.34; H, 7.17; S, 7.22.

### Example 24: Preparation of (1S,4S,5S,7R,8S)-7-benzoyloxy-4,8-dimethyl-4- phenylsulfonylmethyl-2,3-dioxabicyclo[3.3.1]nonan-8-ol (31a) and (1S,4R,5S,7R,8S)-7-benzoyloxy-4,8-dimethyl-4-phenylsulfonylmethyl-2,3-dioxabicyclo[3.3.1]nonan-8-ol (31b) (Scheme 9).

To a solution of sulfides **6a** and **6b** (19.5 mg of *ca*. 94% purity, 0.0435 mmol, *ca*. 65:35) in EtOAc (1.2 mL) at rt was added MCPBA (32 mg of *ca*. 60%, 0.108 mmol) in EtOAc (2 mL). The mixture was stirred for 5 h at rt, poured into saturated NaHCO₃ (10 mL), extracted with hexane-EtOAc (30 mL, 3:2). The organic extract was dried (Na₂SO₄, NaHCO₃₎, evaporated and MPLC (hexane-EtOAc, 60:40) of the residue afforded the diastereomeric sulfones **31a** (11.0 mg) and **31b** (6.3 mg) (total 89% yield). Less polar isomer **31b,** a colorless solid, mp 160-162°C (dec.). ¹H NMR (400 MHz, CDCl₃, δ): 1.37 (s, 3H, Me¹⁰), 1.86 (s, 3H, Me¹¹), 1.97 (br.s., 1H, OH), 2.08 (ddd, 1H, ²*J* = 13.6 Hz, ³*J*₉ₐ₅ₑ = 3.0 Hz, ³*J*₉ₐ₁ₑ = 1.6 Hz, Hₐ⁹), 2.21 (ddd, 1H, ²*J* = 16.0 Hz, ³*J*₆ₐ₅ₑ = 4.4 Hz, ³*J*₆ₐ₇ₑ = 2.0 Hz, Hₐ⁶), 2.32 (m, 1H, Hₑ⁵), 2.34-2.42 (m, 2H, Hₑ⁶ + Hₑ⁹), 3.11 (d, 1H, ²*J* = 14.0 Hz, H¹²), 3.58 (br.d, 1H, ²*J* = 14.0 Hz, H'¹²), 3.94 (dd, 1H, ³*J*₁ₑ₉ₑ = 3.6 Hz, ³*J*₁ₑ₉ₐ = 1.6 Hz, Hₑ¹), 5.27 (dd, 1H, ³*J*₇ₑ₆ₑ = 6.3 Hz, ³*J*₇ₑ₆ₐ = 2.0 Hz, Hₑ⁷), 7.34 (m, 4H), 7.49 (m, 2H), 7.68 (m, 1H), 7.64 (m, 2H), 7.97 (m, 2H). ¹³C NMR (100 MHz, CDCl₃, δ): 21.89 (Me¹¹), 22.83 (C⁹H₂), 23.33 (Me¹⁰), 29.32 (C⁶H₂), 32.20 (C⁵H), 60.85 (C¹²H₂), 73.33 (C⁸), 74.80 (C⁷H), 81.87 (C¹H), 82.04 (C⁴), 127.02 (2C¹⁴H), 128.48 (2CH^{m-benz.}), 129.17 (2C¹⁵H), 129.65 (2CH^{o-benz.}), 129.83 (C^{1-benz.}), 133.18(CH^{p-benz.}), 133.55 (C¹⁶H), 140.88 (C¹³),166.08 (C = O). More polar isomer **31a**, a colorless solid, mp 71-73°C. ¹H NMR (400 MHz, CDCl₃, δ): 1.39 (s, 3H, Me¹⁰), 1.51 (s, 3H, Me¹¹), 1.94 (br.s., 1H, OH), 2.14 (ddd, 1H, ²*J* = 14.0 Hz, ³*J*₉ₐ₅ₑ = 5.0 Hz, ³*J*₉ₐ₁ₑ = 2.4 Hz, Hₐ⁹), 2.15 (ddd, 1H, ²*J* = 16.0 Hz, ³*J*₆ₐ₅ₑ = 4.7 Hz, ³*J*₆ₐ₇ₑ = 1.6 Hz, Hₐ⁶), 2.35 (ddd, 1H, ²*J* = 16.0 Hz, ³*J*₆ₑ₅ₑ = 6.5 Hz, ³*J*₆ₐ₇ₑ = 5.0 Hz, Hₑ⁶), 2.36 (m, 1H, Hₑ⁹), 2.50 (m, 1H, Hₑ⁵), 3.36 (d, 1H, ²*J* = 14.3 Hz, H¹²), 3.83 (dd, 1H, ³*J*₁ₑ₉ₑ = 3.6 Hz, ³*J*₁ₑ₉ₐ = 1.6 Hz, Hₑ¹), 4.34 (br.d, 1H, ²*J* = 14.3 Hz, H'¹²), 5.29 (dd, 1H, ³*J*₇ₑ₆ₑ = 6.5 Hz, ³*J*₇ₑ₆ₐ = 1.6 Hz, Hₑ⁷), 7.44 (m, 2H), 7.55-7.62 (m, 3H), 7.68 (m, 1H), 7.97 (m, 2H), 8.10 (m, 2H). ¹³C NMR (100 MHz, CDCl₃, δ): 23.23 (Me¹¹), 23.41 (Me¹⁰), 23.97 (C⁹H₂), 28.71 (C⁶H₂), 30.15 (C⁵H), 60.89 (C¹²H₂), 73.75 (C⁸), 74.14 (C⁷H), 81.21 (C¹H), 81.71 (C⁴), 127.58 (2C¹⁴H), 128.48 (2CH^{m-benz.}), 129.42 (2C¹⁵H), 129.90 (2CH^{o-benz.}), 130.11 (C^{1-benz.}), 133.15(CH^{p-benz.}), 133.83 (C¹⁶H), 141.10 (C¹³), 166.20 (C=O). DCI HRMS : obsd 447.15100, calcd for C₂₃H₂₇O₇S (M + 1) 447.14775.

### Example 25: Preparation of (1R,4R,5R,8R)-8-acetoxy-4,8-dimethyl-4-phenylsulfonylmethyl-2,3-dioxabicyclo[3.3.1]nonane (23a) (Scheme 10).

To a solution of hydroxysulfone **21a** (230 mg, 0.705 mmol) and 2,6-lutidine (193 mg, 1.80 mmol) in CH₂Cl₂ (5 mL) at -30°C was added TfOTMS (354 mg, 1.50 mmol). Temperature was slowly raised to 0°C (30 min) and the mixture was stirred at 0°C for 1.5 h. The resulting solution was poured into ice-cold saturated NaHCO₃ (30 mL), extracted with hexane-EtOAc (9:1, 150 mL), dried (Na₂SO₄) and evaporated to give the crude TMS-derivative **32a**, containing 2,6-lutidine as an impurity (305 mg, 1: 0.35), a colorless oil, R_{f} 0.36 (hexane-EtOAc 85:15). ¹H NMR (250 MHz, CDCl₃, δ): 0.12 (s, 9H, Me₃Si), 1.37 (s, 3H, Me¹⁰), 1.51 (br.s, 3H, Me¹¹), 1.66 (ddd, 1H, ²*J* = 13.6 Hz, ³*J*₉ₐ₅ₑ = ³*J*₉ₐ₁ₑ = 3.4 Hz, Hₐ⁹), 1.79-1.90 (m, 1H, Hₐ⁶ + Hₑ⁶), 2.11-2.22 (m, 3H, Hₑ⁷ + Hₐ⁷ + Hₑ⁹), 2.25 (m, 1H, Hₑ⁵), 3.27 (d, 1H, ²*J* = 14.2 Hz, H¹²), 3.61 (dd, ³*J*₁ₑ₉ₑ = *J*₁ₑ₉ₐ = 3.4 Hz, Hₑ¹), 4.24 (br.d, 1H, ²*J* = 14.2 Hz, H'¹²), 7.59 (m, 2H, H^{15, 15'}), 7.68 (m, 1H, H¹⁶), 7.96 (m, 2H, H^{14, 14'}); ¹³C NMR (63 MHz, CDCl₃, δ): 2.46 (Me₃Si), 22.93 (Me¹¹), 23.49 (C⁶H₂), 24.33 (C⁹H₂), 27.15 (Me¹⁰), 30.03 (C⁵H), 35.97 (C⁷H₂), 61.09 (C¹²H₂), 74.40 (C⁸), 82.45 (C¹H), 82.65 (C⁴), 127.54 (2C¹⁴H), 129.31 (2C¹⁵H), 133.65 (C¹⁶H), 141.22 (C¹³).

The mixture of crude TMS-derivative **32a** (285 mg) and AcCl (3 mL) was stirred at rt for 45 h, evaporated *in vacuo*, the residue was dissolved in EtOAc (20 mL) and poured into saturated NaHCO₃ (30 mL). After addition of hexane (40 mL) the organic layer was separated and the water layer was extracted with hexane-EtOAc (7:3, 60 mL). The combined organic extract was dried (Na₂SO₄), evaporated and purified by flash chromatography (hexane-EtOAc, 80:20) to give the sulfone **23a** (230 mg, 95%), mp 98-99°C. The NMR spectrum of the sulfone **23a** is identical to that of sulfone **23a** prepared as described in Example 15.

### Example 26: Preparation of (1R,4R,5R,8R)-4,8-dimethyl-8-phenoxyacetoxy-4-phenylsulfonylmethyl-2,3-dioxabicyclo[3.3.1]nonane (33a) (Scheme 10).

A mixture of TMS-derivative **32a** (prepared from 0.22 mmol of hydroxysulfone **21a** as described in Example 25), phenoxyacetyl chloride (1.1 mL) and freshly oven-dried CsF (152 mg, 1.0 mmol) in acetonitrile (2.4 mL) was stirred at rt for a week. The mixture was poured into saturated NaHCO₃ (40 mL) and stirred for 1 h at rt. Hexane-EtOAc (75:25, 100 mL) was added, the organic layer was separated and washed with saturated NaHCO₃ (25 mL). The combined water washings were extracted with hexane-EtOAc (2:1, 75 mL). The combined organic extract was dried (Na₂SO₄ + NaHCO₃) and evaporated. MPLC (hexane-EtOAc, 80:20) of the residue gave the sulfone **33a** (57 mg, 56% yield) as an oil. ¹H NMR (CDCl₃, 400 MHz, δ): 1.50 (br.s, 3H, Me¹¹),1.60 (m, 1H, Hₐ⁶), 1.67 (s, 3H, Me¹⁰), 1.74 (m, 1H, Hₐ⁹), 1.86 (m, 1H, Hₑ⁶), 2.16-2.27 (m, 4H, Hₑ⁵ +Hₑ⁷ +Hₐ⁷ +Hₑ⁹), 3.24 (d, 1H, ²*J* = 14.4 Hz, H¹²), 4.19 (br.d, 1H, ²*J* = 14.4 Hz, H'¹²), 4.36 (br.d, 1H, ³*J*₁ₑ₉ₑ = 3.2 Hz, Hₑ¹), 4.57 (s, 2H, C¹⁷H₂O), 6.89 (m, 2H, 2H^{*o*-phen.}), 7.00 (m, 1H, H^{*p*-phen.}), 7.30 (m, 2H, 2H^{*m*-phen.}), 7.58 (m, 2H, 2H¹⁵), 7.66 (m, 1H, H¹⁶), 7.94 (m, 2H, 2H¹⁴). ¹³C NMR (100 MHz, CDCl₃, δ): 22.60 (Me¹⁰), 23.02 (Me¹¹), 23.18 (C⁶H₂), 24.36 (C⁹H₂), 29.29 (C⁵H), 32.97 (C⁷H₂), 60.93 (C¹²H₂), 65.52 (C¹⁷H₂O), 77.84 (C¹H), 82.76 (C⁴), 84.47 (C⁸), 114.36 (2C^{*o*-phen.}H), 121.73 (C^{*p*-phen.}H), 127.53 (2C¹⁴H), 129.37 (2C¹⁵H), 129.58 (2C^{*m*-phen.}H), 133.78 (C¹⁶H), 141.02 (C¹³), 157.65 (C^{1-phen.}), 167.93(C=O). DCI HRMS : obsd 461.16000, calcd for C₂₄H₂₉O₇S (M + 1) 461.16340.

### Example 27: Preparation of (1R,4R,5R,8R)-4,8-dimethyl-8-(p-methoxybenzyloxy)-4-phenylsulfonylmethyl-2,3-dioxabicyclo[3.3.1]nonane (34a) (Scheme 11).

To a suspension of hydroxysulfone **21a** (280 mg, 0.858 mmol) in ether (3 mL) at 0°C was added a solution of O-(*p*-methoxybenzyl) trichloroacetimidate **44** (1.287 g of *ca*. 93%, 4.19 mmol) in CH₂Cl₂ (1.5 mL), the mixture was stirred for 10 min until homogeneous solution was formed. Then at 0°C a solution of TfOH in ether (0.43 mL of 0.1M TfOH solution in abs. ether, 0.043 mmol) was added and the green-yellow mixture was stirred for 1.5 h at 0°C and for 10 h at rt A second portion of TfOH solution (0.25 mL, 0.025 mmol) was added and the mixture was stirred for 12 h and then additional portions of imidate **44** (772 mg, 2.51 mmol) and TfOH solution (0.25 mL, 0.025 mmol) were added and the mixture was stirred for additional 8 h until consumption of **21a** (TLC monitoring). The mixture was diluted with ether (15 mL) and hexane (15 mL), NaHCO₃ (0.6 g) and Na₂SO₄ (2 g) were added. After being stirred overnight the mixture was filtered, evaporated, fractionated by flash chromatography (hexane-EtOAc 75:25) and purified by a number of MPLC (hexane-EtOAc 78:22) to afford **34a** (175 mg, 45.7%) as a pale yellowish oil, R_{f} 0.40 (hexane-EtOAc 75:25). ¹H NMR (400 MHz, CDCl₃, δ): 1.38 (s, 3H, Me¹⁰), 1.53 (br.s, 3H, Me¹¹), 1.81 (dddd, 1H, ²*J* = ³*J*₆ₐ₇ₐ 14.0 Hz, ³*J*₆ₐ₅ₑ = 6.4 Hz, ³*J*₆ₐ₇ₑ = 3.3 Hz, Hₐ⁶), 1.85 (m, 1H, Hₑ⁶), 1.94 (br.dd, 1H, ²*J* = 14.6 Hz, ³*J*₇ₑ₆ₑ = 5.0 Hz, Hₑ⁷), 2.11 (ddd, 1H, ²*J* = ³*J*₇ₐ₆ₐ = 14.0 Hz, ³*J*₇ₐ₆ₑ = 6.4 Hz, Hₐ⁷), 2.17 (m, 2H, Hₑ⁹ + Ha⁹), 2.27 (br.dddd, 1H, ³*J*₅ₑ₆ₑ = ³*J*₅ₑ₆ₐ = 6.4 Hz, ³*J*₅ₑ₉ₑ = ³*J*₅ₑ₉ₐ = 3.2 Hz, Hₑ⁵), 3.29 (d, 1H, ²*J* = 14.3 Hz, H¹²), 3.81 (s, 3H, MeO), 3.825 (br.dd, 1H, ³*J*₁ₑ₉ₑ = ³*J*₁ₑ₉ₐ = 3.0 Hz, Hₑ¹), 4.24 (br.d, 1H, ²*J* = 14.3 Hz, H'¹²), 4.29 and 4.41 (ABq, 2H, ²*J* = 10.7 Hz, CH¹⁷H^{17'}O), 6.88 (ddd, 2H, ³*J* = 8.7 Hz, ⁴*J*₁ = ⁴*J*₂ = 2.0 Hz, 2H^{*m*-benz.}), 7.24 (br.d, ³*J* = 8.7 Hz, 2H^{*o*-benz.}), 7.58 (dddd, 2H, ³*J*_{15,14} = ³*J*_{15,16} = 7.3 Hz, ⁴*J*_{1514'} = ⁴*J*_{15'14} = 1.4 Hz, H¹⁵, ^{15'}), 7.66 (dddd, 1H, ³*J*_{16,15} = ³*J*_{16,15'} = 7.3 Hz, ⁴*J*₁₆₁₄ = ⁴*J*_{1614'} = 1.4 Hz, H¹⁶), 7.95 (br.d, 2H, ³*J*_{14,15} = 7.3 Hz, H^{14, 14'}); ¹³C NMR (100 MHz, CDCl₃, δ): 22.16 (Me¹⁰), 22.92 (Me¹¹), 23.53 (C⁶H₂), 24.57 (C⁹H₂), 29.86 (C⁵H), 30.76 (C⁷H₂), 61.10 (C¹²H₂), 62.91 (C¹⁷H₂O), 75.94 (C⁸), 80.84 (C¹H), 82.71 (C⁴), 113.78 (2C^{*m*-benz.}H), 127.55 (2C¹⁴H), 128.72 (2C^{*o*-benz.}H), 129.33 (2C¹⁵H), 131.26 (C^{1-benz.}), 133.68 (C¹⁶H), 141.17 (C¹³), 158.92 (C^{*p*-benz.}). DCI HRMS: obsd 447.18690, calcd for C₂₄H₃₁O₆S (M + 1) 447.18414.

### Example 28: Preparation of (1R,4R,5R,8R)-4,8-dimethyl-8-(γ,γ-dimethylallyloxy)-4-phenylsulfonylmethyl-2,3-dioxabicyclo [3.3.1]nonane (35a) (Scheme 11).

To a suspension of hydroxysulfone **21a** (95 mg, 0.32 mmol) in ether (1 mL) at 0°C was added a solution of O-(γ,γ-dimethylallyl)trichloroacetimidate **45** (240 mg of *ca*. 96%, 1.0 mmol) in CH₂Cl₂ (0.5 mL), the mixture was stirred for 10 min to form a clear solution. A solution of TfOH in ether (0.1 mL of 0.1M TfOH solution in abs. ether, 0.01 mmol) at 0°C was added and the green-yellow mixture was stirred for 4 h at 0°C. Subsequently additional portions of imidate **45** (240 mg, 1.0 mmol) and TfOH solution (0.1 mL, 0.01 mmol) were added and the mixture was stirred for 2 h, and then additional imidate **45** (240 mg, 1.0 mmol) and TfOH solution (0.1 mL, 0.01 mmol) were added. After being stirred for additional 2 h at 0°C the mixture was diluted with EtOAc (15 mL) and hexane (60 mL), NaHCO₃ (2 g) and Na₂SO₄ (10 g) were added and the mixture was stirred overnight. The mixture was filtered, evaporated, the residue was fractionated by flash chromatography (hexane-EtOAc 84:16) and purified by MPLC (hexane-EtOAc 84:16) to give **35a** (58 mg, 50.5%) as a colorless oil, R_{f} 0.42 (hexane-EtOAc, 80:20). ¹H NMR (400 MHz, CDCl₃, δ): 1.29 (s, 3H, Me¹⁰), 1.50 (br.s, 3H, Me¹¹), 1.65 (m, 3H, Me²⁰), 1.73 (m, 3H, Me²¹), 1.78 (dddd, 1H, ²*J* = ³*J*₆ₐ₇ₐ 14.0 Hz, ³*J*₆ₐ₅ₑ = 6.0 Hz, ³*J*₆ₐ₇ₑ = 3.5 Hz, Hₐ⁶), 1.82 (m, 1H, Hₑ⁶), 1.85 (br.dd, 1H, ²*J* = 14.0 Hz, ³*J*₇ₑ₆ₑ = 5.5 Hz, Hₑ⁷), 2.06 (ddd, 1H, ²*J* ≈ ³*J*₇ₐ₆ₐ = 14.0 Hz, ³*J*₇ₐ₆ₑ = 6.1 Hz, Hₐ⁷), 2.10-2.18 (m, 2H, Hₑ⁹ + Ha⁹), 2.27 (dddd, 1H, ³*J*₅ₑ₆ₑ ≈ ³*J*₅ₑ₉ₑ = 6.0 Hz, ³*J*₅ₑ₆ₐ ≈ ³*J*₅ₑ₉ₐ = 3.0 Hz, Hₑ⁵), 3.27 (d, 1H, ²*J* = 14.3 Hz, H¹²), 3.77 (br.s, 1H, Hₑ¹), 3.80 and 3.90 (dABq, 2H, ²*J* = 10.8 Hz, ³*J*_{17,18} = ³*J*_{17',18} = 6.8 Hz, H¹⁷ and H'¹⁷), 4.22(d.d, 1H, ²*J* = 14.3 Hz, ⁴*J* = 0.3 Hz, H'¹²), 5.27 (ddqq, ³*J*_{18,17} = ³*J*_{18,17'} = 6.8 Hz, ³*J*_{18,20} = ³*J*_{18,21} = 1.4 Hz, HC¹⁸ = ), 7.56 (dddd, 2H, ³*J*_{15,14} = ³*J*_{15,16} = 7.5 Hz, ⁴*J*_{1514'} = ⁴*J*_{15'14} = 1.4 Hz, H^{15, 15'}), 7.65 (dddd, 1H, ³*J*_{16,15} = ³*J*_{16,15'} = 7.5 Hz, ⁴*J*₁₆₁₄ = ⁴*J*_{1614'} = 1.4 Hz, H¹⁶), 7.94 (ddd, 2H, ³*J*_{14,15} = 7.4 Hz, ⁴*J*_{14,16} = ⁴*J*_{14'16} = 1.4 Hz, H^{14, 14'}); ¹³C NMR (100 MHz, CDCl₃, δ): 17.94 (Me²⁰C=), 22.04 (Me¹⁰), 22.87 (Me¹¹), 23.49 (C⁶H₂), 24.48 (C⁹H₂), 29.84 (C⁵H), 30.97 (C⁷H₂), 57.78 (C¹⁷H₂O), 61.09 (C¹²H₂), 75.47 (C⁸), 80.70 (C¹H), 82.63 (C⁴), 121.78 (HC¹⁸=), 127.53 (2C¹⁴H), 129.29 (2C¹⁵H), 133.64 (C¹⁶H), 135.90 (C¹⁹=), 141.17 (C¹³). DCI HRMS: obsd 395.18590, calcd for C₂₁H₃₁O₅S (M + 1) 395.18922; obsd 377.17500, calcd for C₂₁H₂₉O₄S (M + 1 - H₂O) 377.17866.

### Example 29: Preparation of (1R,4R,5R,8R)-8-(trans-cinnamyloxy)-4,8-dimethyl-4-phenylsulfonylmethyl-2,3-dioxabicyclo [3.3.1]nonane (36a) (Scheme 11).

To a suspension of hydroxysulfone **21a** (113 mg, 0.345 mmol) in ether (1.2 mL) at 0°C a solution of iminoester **46** (406 mg of *ca*. 97%, 1.41 mmol) in CH₂Cl₂ (0.7 mL) was added, the mixture was stirred for 10 min and a clear solution was formed. Then at 0°C a solution of TfOH in ether (0.15 mL of 0.1M TfOH solution in abs. ether, 0.015 mmol) was added and the green-yellow mixture was stirred for 1 h at 0°C and for 6 h at rt A second portion of iminoester **46** (406 mg, 1.41 mmol, neat) and a solution of TfOH (0.15 mL, 0.015 mmol) were added and the mixture was stirred for 12 h. A third portion of imidate **46** (406 mg, 1.41 mmol) and TfOH solution (0.15 mL, 0.015 mmol) were added and the mixture was stirred for 12 h followed by a fourth addition of TfOH solution (0.15 ml, 0.015 mmol) and stirring for additional 30 h. The mixture was diluted with hexane-EtOAc (3:1, 100 mL), NaHCO₃ (2 g) and Na₂SO₄ (5 g) were added. After being stirred overnight the mixture was filtered, evaporated, fractionated by flash chromatography (hexane-EtOAc, 75:25) and further purified by MPLC (hexane-EtOAc 80:20) to give **36a** (49 mg, 32%) as a pale yellowish gum, R_{f} 0.46 (hexane-EtOAc 75:25). ¹H NMR (400 MHz, CDCl₃, δ): 1.34 (s, 3H, Me¹⁰), 1.53 (br.s, 3H, Me¹¹), 1.84 (dddd, 1H, ²*J* = ³*J*₆ₐ₇ₐ 14.0 Hz, ³*J*₆ₐ₅ₑ = 5.6 Hz, ³*J*₆ₐ₇ₑ = 3.6 Hz, Hₐ⁶), 1.84-1.93 (m, 2H, Hₑ⁶ + Hₑ⁷), 2.10 (ddd, 1H, ²*J* = ³*J*₇ₐ₆ₐ = 14.0 Hz, ³*J*₇ₐ₆ₑ = 6.4 Hz, Hₐ⁷), 2.15-2.22 (m, 2H, Hₑ⁹ + Ha⁹), 2.27 (dddd, 1H, ³*J*₅ₑ₆ₑ *=* ³*J*₅ₑ₆ₐ = 5.6 Hz, ³*J*₅ₑ₉ₑ = ³*J*₅ₑ₉ₐ = 3.0 Hz, Hₑ⁵), 3.29 (d, 1H, ²*J* = 14.3 Hz, H¹²), 3.82 (br.s, 1H, Hₑ¹), 3.80 and 3.90 (ddABq, 2H, ²*J* = 12.6 Hz, ³*J*_{17,18} = ³*J*_{17',18} = 5.8 Hz, ⁴*J*_{17,19} = ⁴*J*_{17',19} = 1.5 Hz, H¹⁷ and H'¹⁷), 4.24(br.d, 1H, ²*J* = 14.3 Hz, H'¹²), 6.25 (ddd, ³*J*_{18,19} = 15.9 Hz, ³*J*_{18,17} = ³*J*_{18,17'} = 5.8 Hz, HC¹⁸=), 6.60 (br.d, ³*J*_{19,18} = 15.9 Hz, HC¹⁹=), 7.22-7.40 (m, 5H, PhCH=), 7.58 (m, 2H, H^{15, 15'}), 7.66 (m, 1H, H¹⁶), 7.96 (m, 2H, H^{14, 14'}); ¹³C NMR (100 MHz, CDCl₃, δ): 22.11 (Me¹⁰), 22.93 (Me¹¹), 23.53 (C⁶H₂), 24.58 (C⁹H₂), 29.90 (C⁵H), 31.00 (C⁷H₂), 61.15 (C¹²H₂), 61.94 (C¹⁷H₂O), 75.96 (C⁸), 80.78 (C¹H), 82.75 (C⁴), 126.40 (2C^{*o*-phen.}H), 127.12 (HC¹⁸=), 127.52 (C^{*p*-phen.}H),127.59 (2C¹⁴H), 128.52 (2C^{*m*-phen.}H), 129.34 (2C¹⁵H), 130.99 (HC¹⁹=), 133.69 (C¹⁶H), 136.85 (C^{1-phen.}), 141.24 (C¹³). DCI HRMS : obsd 443.19150, calcd for C₂₅H₃₁O₅S (M + 1) 443.18922.

### Example 30: Preparation of (1R,4R,5R)-4,8-dimethyl-4-phenylsulfonylmethyl-2,3-dioxabicyclo [3.3.1]non-7-ene (37a) and (1R,4R,5R)-4-methyl-8-methylidene-4-phenylsulfonylmethyl-2,3-dioxabicyclo[3.3.1]nonane (38a) (Scheme 12).

To a mixture of SOCl₂ (395 mg, 3.32 mmol) and pyridine (656 mg, 8.30 mmol) in CH₂Cl₂ (40 mL) at 0°C for 30 min a solution of hydroxysulfone **21a** (270 mg, 0.827 mmol) in CH₂Cl₂ (15 mL) was added. The mixture was stirred at 0°C for 2 h and for additional 4 h at rt, poured into ice-cold 0.2 N HCl (100 mL) and extracted with hexane-EtOAc (3:1, 2 x 200 mL). The combined organic extract was washed with saturated NaHCO₃ (30 mL), dried (Na₂SO₄), filtered and evaporated. Flash chromatogaphy (hexane-EtOAc, 80:20) afforded a colorless solid mixture of **37a** and **38a** (243 mg, 95.5%, **37a/38a** *ca*. 86:14). More polar isomer **37a** of *ca*. 97.5% purity was obtained by several sequential MPLC (hexane-EtOAc, 85:15) as a colorless solid, mp 111-112°C. ¹H NMR (400 MHz, CDCl₃, δ): 1.47 (d, 3H, ⁴*J* = 0.6 Hz, Me¹¹), 1.72 (ddd, 1H, ²*J* = 13.3 Hz, ³*J*₉ₐ₅ₑ = 2.8 Hz, ³*J*₉ₐ₁ₑ = 2.0 Hz, Hₐ⁹), 1.78 (ddd, 3H, ⁴*J*_{10,7} = 2.7 Hz, ⁵*J*_{10,6a} ≈ ⁵*J*_{10,6e} = 1.7 Hz, Me¹⁰), 2.26 (dddq, 1H, ²*J* = 19.1 Hz, ³*J*₆ₐ₇ = 5.4 Hz, ³*J*₆ₐ₅ₑ = ⁵*J*₆ₐ₁₀ = 1.7 Hz, Hₐ⁶), 2.39 (dddq, 1H, ²*J* = 19.1 Hz, ³*J*₆ₑ₇ = 3.2 Hz, ³*J*₆ₑ₅ₑ = ⁵*J*₆ₑ₁₀ = 1.7 Hz, Hₑ⁶), 2.46 (m, 1H, Hₑ⁵), 2.50 (dddd, 1H, ²*J* = 13.3 Hz, ³*J*₉ₑ₅ₑ = ³*J*₉ₑ₁ₑ = 3.5 Hz, ^{4 or 5}*J* = 1.6 Hz, Hₑ⁹), 3.29 (d, 1H, ²*J* = 14.3 Hz, H¹²), 4.12 (m, 1H, Hₑ¹), 4.31 (dd, 1H, ²*J*= 14.3 Hz, ⁴*J* = 0.6 Hz, H'¹²), 5.77 (m, 1H, H⁷), 7.58 (m, 2H, H^{15, 15'}), 7.66 (m, 1H, H¹⁶), 7.95 (m, 2H, H^{14, 14'}); ¹³C NMR (100 MHz, CDCl₃, δ): 21.16 (Me¹⁰), 23.62 (Me¹¹), 26.83 (C⁹H₂), 27.48 (C⁶H₂), 29.54 (C⁵H), 61.23 (C¹²H₂), 76.70 (C¹H), 82.35 (C⁴), 127.28 (C⁷H), 127.55 (2C¹⁴H), 129.35 (2C¹⁵H), 131.15(C⁸=), 133.71 (C¹⁶H), 141.20 (C¹³). Anal. Calcd for C₁₆H₂₀O₄S: C, 62.31; H, 6.54; S, 10.40. Found: C, 62.17; H, 6.57; S, 10.47. Less polar isomer **38a** (*ca*. 95% purity) was isolated as a colorless semi-solid by semi-preparative DP HPLS (column - LiChrospher® Si 60 (10 µm) 250-10; eluent *i*-PrOH-hexane 2:98). ¹H NMR (400 MHz, CDCl₃, δ): 1.57 (d, 3H, ⁴*J* = 0.6 Hz, Me¹¹), 1.65 (ddd, 1H, ²*J* = 13.7 Hz, ³*J*₉ₐ₅ₑ = 3.2 Hz, ³*J*₉ₐ₁ₑ = 1.7 Hz, Hₐ⁹), 1.73 (dddd, 1H, ²*J* = ³*J*₆ₐ₇ₐ = 14.2 Hz, ³*J*₆ₐ₅ₑ = 6.4 Hz, ³*J*₆ₐ₇ₑ = 3.8 Hz, Hₐ⁶), 2.15 (br.ddd, 1H, ²*J* = 14.2 Hz, ³*J*₆ₑ₇ₑ = 6.6 Hz, ³*J*₆ₑ₅ₑ = 3.2 Hz, Hₑ⁶), 2.39 (br.dd, 1H, ²*J* = 14.2 Hz, ³*J*₇ₑ₆ₑ = 6.6 Hz, Hₑ⁷), 2.46 (dddd, 1H, ³*J*₅ₑ₆ₐ = ³*J*₅ₑ₉ₑ = 6.4 Hz, ³*J*₅ₑ₆ₑ = ³*J*₅ₑ₉ₐ = 3.2 Hz, Hₑ⁵), 2.57 (ddd, 1H, ²*J* = 13.7 Hz, ³*J*₉ₑ₅ₑ = 6.4 Hz, ³*J*₉ₑ₁ₑ = 3.6 Hz, Hₑ⁹), 2.99 (m, 1H, ²*J* = ³*J*₆ₐ₇ₐ = 14.2 Hz, Hₑ⁷), 3.29 (d, 1H, ²*J* = 14.3 Hz, H¹²), 4.30 (br.dd, 1H, ²*J* = 14.3 Hz, ⁴*J* = 0.6 Hz, H'¹²), 4.36 (br.dd, 1H, ³*J*₁ₑ₉ₑ = 3.6 Hz, ³*J*₁ₑ₉ₐ = 1.7 Hz, Hₑ¹), 4.92 (br.dd, 1H, ²*J =* ⁴*J*_{10,7a} = 2.2 Hz, H¹⁰), 4.95 (br.ddd, 1H, ²*J* = ⁴*J*_{10',7a} = 2.2 Hz, ⁴*J* = 0.6 Hz, H'¹⁰),7.59 (m, 2H, H^{15, 15'}), 7.67 (m, 1H, H¹⁶), 7.96 (m, 2H, H^{14, 14'}); ¹³C NMR (100 MHz, CDCl₃, δ): 23.32 (Me¹¹), 27.02 (C⁶H₂), 30.39 (C⁵H), 30.50 (C⁹H₂), 30.52 (C⁷H₂), 61.27 (C¹²H₂), 80.77 (C¹H), 82.96 (C⁴), 113.59 (=C¹⁰H₂), 127.57 (2C¹⁴H), 129.36 (2C¹⁵H), 131.15(C⁸=), 133.74 (C¹⁶H), 141.18 (C¹³), 146.21 (C⁸). DCI HRMS obsd 309.11930, calcd for C₁₆H₂₁O₄S (M + H) 309.11606.

### Example 31: Preparation of (1R,4R,5R,7R,8R)-7,8-epoxy-4,8-dimethyl-4-phenylsulfonylmethyl-2,3-dioxabicyclo[3.3.1]nonane (39a) (Scheme 13).

To a solution of unsaturated sulfone **37a** (36.0 mg of *ca*. 97.5% purity , 0.114 mmol) in CH₂Cl₂ (0.5 mL) at 0°C a suspension of MCPBA (38.0 mg of *ca*. 60%, 0.13 mmol) in CH₂Cl₂ (1.5 mL) was added. The mixture was stirred at rt for 12 h, diluted with hexane-EtOAc (40 mL, 3:1), washed with saturated NaHCO₃ (2 x 5 mL), the water washings were extracted with hexane-EtOAc (20 mL, 3:1). The combined organic extract was dried (Na₂SO₄ + NaHCO₃) and evaporated. The residue was purified by flash chromatography (hexane-EtOAc, 4:1) to afford the epoxide **39a** (35.5 mg, 96%) as a colorless solid, mp 130-131°C. ¹H NMR (400 MHz, CDCl₃, δ): 1.44 (s, 3H, Me¹⁰), 1.50 (d, 3H, ⁴*J* = 0.5 Hz, Me¹¹), 1.91 (ddd, 1H, ²*J* = 14.0 Hz, ³*J*₉ₐ₅ₑ = 2.3 Hz, ³*J*₉ₐ₁ₑ = 1.8 Hz, Hₐ⁹), 2.01(dd, 1H, ²*J* = 16.5 Hz, ³*J*₆ₐ₅ₑ = 2.3 Hz, Hₐ⁶), 2.15 (ddd, 1H, ²*J* = 14.0 Hz, ³*J*₉ₑ₅ₑ = 6.6 Hz, ³*J*₉ₑ₁ₑ = 3.8 Hz, Hₑ⁹), 2.21-2.32 (m, 2H, Hₑ⁵ + Hₑ⁶), 3.19 (br.d, 1H, ⁴*J*₇ₑ₆ₑ = 5.5 Hz, Hₑ⁷), 3.29 (d, 1H, ²*J* = 14.3 Hz, H¹²), 4.18 (br.dd, 1H, ³*J*₁ₑ₉ₑ = 3.8 Hz, ³*J*₁ₑ₉ₐ = 1.8 Hz, Hₑ¹), 4.22(d.d, 1H, ²*J* = 14.3 Hz, ⁴*J* = 0.5 Hz, H'¹²), 7.59 (m, 2H, H^{15, 15'}), 7.68 (m, 1H, H¹⁶), 7.94 (m, 2H, H^{14, 14'}); ¹³C NMR (63 MHz, CDCl₃, δ): 18.64 (Me¹⁰), 22.57 (C⁹H₂), 23.38 (Me¹¹), 25.27 (C⁶H₂), 28.74 (C⁵H), 57.10 (C⁸), 59.00 (C⁷H), 60.93 (C¹²H₂), 78.29 (C¹H), 82.15 (C⁴), 127.50 (2C¹⁴H), 129.36 (2C¹⁵H), 133.79 (C¹⁶H), 140.99 (C¹³). Anal. Calcd for C₁₆H₂₀O₅S: C, 59.24; H, 6.21; S, 9.88. Found: C, 59.54; H, 6.21; S, 9.46. **Example 32**: **Preparation of 1*****R*****,4*****R*****,5*****R*****,8*****R*****)-4,8-dimethyl-4-phenylsulfinylmethyl-2,3-dioxabicyclo [3.3.1]nonan-8-ols (40 a, a') and (1*****R*****,4*****S*****,5*****R*****,8*****R*****)-4,8-dimethyl-4-phenylsulfinylmethyl-2,3-dioxabicyclo[3.3.1]nonan-8-ols (40 b, b') (Scheme 14).**

To a solution of sulfides **2a,b** (468 mg, 1.59 mmol, ratio **2a/2b** *ca*. 55:45) in EtOAc (25 mL) at -50°C was added a solution of MCPBA (478 mg of *ca*. 60%, *ca*. 1.65 mmol) in EtOAc (20 mL). The mixture was stirred at -30 ö -40°C for 1 h, poured into saturated NaHCO₃ (75 mL), extracted with EtOAc (3 x 60 mL), dried (Na₂SO₄ + NaHCO₃) and evaporated. Flash chromatography on silica gel (eluent hexane-EtOAc, 1:4) afforded the mixture of diastereomeric sulfoxides **40 a, a', b, b'** (438 mg, total yield 89%, ratio **a:a':b:b'** ∼ 21: 31.5: 20: 27.5 according to the integration in 400 MHz ¹H NMR spectrum). The sulfoxides were separated by MPLC (hexane-EtOAc, 22:78) to obtain the individual diastereomers (listed below according the order of polarity). Isomer **40b,** colorless solid, R_{f} 0.38 (hexane-EtOAc 1:4). ¹H NMR (400 MHz, CDCl₃, δ): 1.36 (s, 3H, Me¹⁰), 1.62 (br.dd, 1H, ²*J* = 13.9 Hz, ³*J*₇ₑ₆ₑ = 5.8 Hz, Hₑ⁷), 1.79 (br.s, 3H, Me¹¹), 1.81 (m, 1H, Hₑ⁶), 1.96 (dddd , 1H, ²*J* ≈ ³*J*₆ₐ₇ₐ = 13.9 Hz, ³*J*₆ₐ₅ₑ = 6.0 Hz, ³*J*₆ₐ₇ₑ = 3.5 Hz, Hₐ⁶), 2.00 (m, 1H, Hₑ⁵), 2.13 (ddd, 1H, ²*J* = 13.5 Hz, ³*J*₉ₐ₅ₑ = 2.9 Hz, ³*J*₉ₐ₁ₑ = 2.0 Hz, Hₐ⁹), 2.29 (ddd, 1H, ²*J* = ³*J*₇ₐ₆ₐ = 13.9 Hz, ³*J*₇ₐ₆ₑ = 6.2 Hz, Hₐ⁷), 2.35 (br.ddd, 1H, ²*J* = 13.5 Hz, ³*J*₉ₑ₅ₑ = 6.4 Hz, ³*J*₉ₑ₁ₑ = 3.6 Hz, Hₑ⁹), 2.77 (dd, 1H, ²*J* = 13.5 Hz, ⁴*J* = 0.6 Hz, H'¹²), 2.91 (d, 1H, ²*J* = 13.5 Hz, H¹²), 3.73 (m, ³*J*₁ₑ₉ₑ = 3.6 Hz, *J*₁ₑ₉ₐ = 2.0 Hz, Hₑ¹), 7.50-7.56 (m, 3H, H^{15, 15'}+ H¹⁶), 7.63-7.66 (m, 2H, H^{14, 14'}); ¹³C NMR (100 MHz, CDCl₃, δ): 22.93 (Me¹¹), 23.70 (C⁶H₂), 24.00 (C⁹H₂), 28.04 (Me¹⁰), 31.20 (C⁵H), 35.47 (C⁷H₂), 65.57 (C¹²H₂), 71.32 (C⁸), 82.20 (C¹H), 82.61 (C⁴), 123.77 (2C¹⁴H), 129.49 (2C¹⁵H), 131.34 (C¹⁶H), 144.73 (C¹³). Isomer **40 a**, colorless solid, R_{f} 0.35 (hexane-EtOAc 1:4). ¹H NMR (250 MHz, (CDCl₃, δ): 1.41 (s, 3H, Me¹⁰), 1.47 (br.s, (Me¹⁰), 32.16 (C⁵H), 35.46 (C⁷H₂), 65.94 (C¹²H₂), 71.38 (C⁸), 82.35 (C¹H), 82.73 (C⁴),3H, Me¹¹), 1.60-1.73 (m, 1H), 1.92-2.04 (m, 2H), 2.17-2.24 (m, 3H, Hₑ⁵ + Hₑ⁷ + Hₑ⁹), 2.41 (ddd, 1H, ²*J* = 14.2 Hz, ³*J*₇ₐ₆ₐ = 12.6 Hz, ³*J*₇ₐ₆ₑ = 7.6 Hz, Hₐ⁷), 3.13 (d, 1H, ²*J* = 13.9 Hz, H¹²), 3.61 (dd, 1H, ²*J* = 13.9 Hz, ⁴*J*_{12',11} = 0.4 Hz, H'¹²), 3.75 (m, 1H, Hₑ¹), 7.52-7.58 (m, 3H, H^{15, 15'} + H¹⁶), 7.67-7.71 (m, 2H, H^{14,14'}). Isomer **40 b',** colorless solid, mp 138-140°C (dec.), R_{f} 0.34 (hexane-EtOAc 1:4). ¹H NMR (250 MHz, CDCl₃, δ): 1.36 (s, 3H, Me¹⁰), 1.57 (br.dd, 1H, ²*J*₇ₑ₇ₐ = 13.4 Hz, ³*J*₇ₑ₆ₑ = 5.5 Hz, Hₑ⁷),1.83 (m, 1H, Hₐ⁶), 1.88-1.99 (m, 2H, Hₑ⁵ + Hₑ⁶), 1.89 (br.s, 3H, Me¹¹), 2.08 (ddd, 1H, ²*J* = 13.4 Hz, ³*J*₉ₐ₅ₑ = 2.9 Hz, ³*J*₉ₐ₁ₑ = 2.0 Hz, Hₐ⁹), 2.17 (br.ddd, 1H, ²*J*₇ₐ₇ₑ = ³*J*₇ₐ₆ₐ = 14.8 Hz, ³*J*₇ₐ₆ₑ = 5.6 Hz, Hₐ⁷), 2.35 (br.ddd, 1H, ²*J* = 13.4 Hz, ³*J*₉ₑ₅ₑ = 6.1 Hz, ³*J*₉ₑ₁ₑ = 3.6 Hz, Hₑ⁹), 2.72 (d, 1H, ²*J* = 13.7 Hz, H¹²), 3.11 (d, 1H, ²*J* = 13.7 Hz, H¹²), 3.75 (m, ³*J*₁ₑ₉ₑ = 3.6 Hz, ³*J*₁ₑ₉ₐ = 2.0 Hz, Hₑ¹), 7.52-7.57 (m, 3H, H^{15,} ^{15'}+ H¹⁶), 7.65-7.69 (m, 2H, H^{14, 14'}); ¹³C NMR (63 MHz, CDCl₃, δ): 21.97 (Me¹¹), 23.48 (C⁶H₂), 23.94 (C⁹H₂), 28.04 123.82 (2C¹⁴H), 129.39 (2C¹⁵H), 131.12 (C¹⁶H), 144.85 (C¹³). Isomer **40 a'**, a colorless solid, mp 153-155°C, R_{f} 0.32 (hexane-EtOAc 1:4). ¹H NMR (400 MHz, (CDCl₃, δ): 1.40 (s, 3H, Me¹⁰), 1.55 (br.s, 3H, Me¹¹), 1.63 (br.dd, 1H, ²*J* = 14.0 Hz, ³*J*₇ₑ₆ₑ = 5.6 Hz, Hₑ⁷), 1.83 (m, 1H, Hₑ⁵), 1.86 (dddd, 1H, ²*J* = ³*J*₆ₐ₇ₐ = 14.0 Hz, ³*J*₆ₐ₅ₑ = 5.9 Hz, ³*J*₆ₐ₇ₑ = 3.4 Hz, Hₐ⁶), 1.96 (m, 1H, ²*J* = 14.0 Hz, Hₑ⁶), 2.06 (ddd, 1H, ²*J* = 13.8 Hz, ³*J*₉ₐ₅ₑ = 2.8 Hz, ³*J*₉ₐ₁ₑ = 2.0 Hz, Hₐ⁹), 2.18 (ddd, 1H, ²*J* = 13.8 Hz, ³*J*₉ₑ₅ₑ = 6.4 Hz, ³*J*₉ₐ₁ₑ = 3.6 Hz, Hₑ⁹), 2.37 (br.ddd, 1H, ²*J* = ³*J*₇ₐ₆ₐ = 14.0 Hz, ³*J*₇ₐ₆ₑ = 6.5 Hz, Hₐ⁷), 3.31 and 3.35 (ABq, 2H, ²*J* = 14.0 Hz, H¹² + H'¹²), 3.70 (m, 1H, ³*J*₁ₑ₉ₑ = 3.6 Hz, ³*J*₁ₑ₉ₐ = 2.0 Hz, Hₑ¹), 7.47-7.56 (m, 3H, H^{15, 15'} + H¹⁶), 7.64-7.69 (m, 2H, H^{14, 14'}); ¹³C NMR (100 MHz, CDCl₃, δ): 22.08 (Me¹¹), 23.47 (C⁶H₂), 24.37 (C⁹H₂), 28.06 (Me¹⁰), 31.56 (C⁵H), 35.77 (C⁷H₂), 66.08 (C¹²H₂), 71.42 (C⁸), 81.94 (C¹H), 82.25 (C⁴), 123.83 (2C¹⁴H), 129.32 (2C¹⁵H), 130.87 (C¹⁶H), 144.91 (C¹³).

### Example 33: Preparation of (1R,4R,5R,8R)-8-acetoxy-4,8-dimethyl-4-phenylsulfinylmethyl-2,3-dioxabicyclo[3.3.1]nonanes (41a, a') and (1R,4S,5R,8R)-8-acetoxy-4,8-dimethyl-4-phenylsulfinylmethyl-2,3-dioxabicyclo[3.3.1]nonanes (41b, b') (Scheme 14).

To a solution of sulfides **13 a,b** (376 mg, 1.12 mmol, ratio **13a/13b** *ca*. 55:45) in EtOAc (25 mL) at -50°C was added a solution of MCPBA (330 mg of *ca*. 60%, *ca*. 1.15 mmol) in EtOAc (15 mL). The mixture was stirred at -30 ö -40°C for 3 h, poured into saturated NaHCO₃ (50 mL), extracted with hexane-EtOAc (4:6, 3 x 60 mL), dried (Na₂SO₄ + NaHCO₃) and evaporated. The sulfoxides were separated by MPLC (hexane-EtOAc, 1:1), total yield 364 mg (1.02 mmol, 91%). The least polar isomer **41b,** 68 mg (0.193 mmol), a colorless solid, mp 116-118°C, R_{f} 0.41 (hexane-EtOAc 1:1). IR (neat): 2980, 2929, 1734 v.s, 1445, 1372 v.s, 1257 v.s, 1232 v.s, 1186, 1153, 1086 v.s, 1056, 1022 v.s, 933, 826, 753, 735, 694 cm-¹. ¹H NMR (400 MHz, CDCl₃, δ): 1.65 (s, 3H, Me¹⁰), 1.80 (d, 3H, ⁴*J* = 0.65 Hz, Me¹¹), 1.82-1.89 (m, 2H, Hₑ⁶ + Hₐ⁶), 1.89 (ddd, 1H, ²*J* = 13.6 Hz, ³*J*₉ₐ₅ₑ = 3.2 Hz, ³*J*₉ₐ₁ₑ = 1.8 Hz, Hₐ⁹), 2.02 (m, 1H, Hₑ⁵), 2.03 (s, 3H, Me¹⁷CO), 2.18-2.29 (m, 2H, Hₐ⁷ +Hₑ⁷), 2.39 (br.ddd, 1H, ²*J* = 13.6 Hz, ³*J*₉ₑ₅ₑ = 5.7 Hz, ³*J*₉ₑ₁ₑ = 3.8 Hz, Hₑ⁹), 2.77 (dd, 1H, ²*J* = 13.4 Hz, ⁴*J* = 0.65 Hz, H'¹²), 2.92 (d, 1H, ²*J* = 13.4 Hz, H¹²), 4.47 (br.dd, ³*J*₁ₑ₉ₑ = 3.8 Hz, *J*₁ₑ₉ₐ = 1.8 Hz, Hₑ¹), 7.52-7.57 (m, 3H, H^{15, 15'}+ H¹⁶), 7.64-7.67 (m, 2H, H^{14, 14'}); ¹³C NMR (100 MHz, CDCl₃, δ): 22.38 (Me¹⁷CO), 22.45 (Me¹⁰), 22.88 (Me¹¹), 23.52 (C⁶H₂), 23.76 (C⁹H₂), 30.55 (C⁵H), 32.77 (C⁷H₂), 65.45 (C¹²H₂), 78.02 (C¹H), 82.50 (C⁸), 82.57 (C⁴), 123.69 (2C¹⁴H), 129.45 (2C¹⁵H), 131.33 (C¹⁶H), 144.57 (C¹³), 170.08 (C=O). Isomer **41 a** (78 mg, 0.221 mmol), a colorless oil, R_{f} 0.40 (hexane-EtOAc 1:1). ¹H NMR (400 MHz, (CDCl₃, δ): 1.44 (br.s, 3H, Me¹¹), 1.65 (s, 3H, Me¹⁰), 1.86 (dddd, 1H, ²*J* ≈ ³*J*₆ₐ₇ₐ = 14.0 Hz, ³*J*₆ₐ₅ₑ = 6.0 Hz, ³*J*₆ₐ₇ₑ = 3.5 Hz, Hₐ⁶), 1.95-2.00 (m, 1H, Hₑ⁶), 1.96 (ddd, 1H, ²*J* = 14.0 Hz, ³*J*₉ₐ₅ₑ = 4.0 Hz, ³*J*₉ₐ₁ₑ = 1.7 Hz, Hₐ⁹), 2.01 (s, 3H, Me¹⁷CO), 2.18-2.25 (m, 3H, Hₑ⁵ + Hₑ⁷ + Hₑ⁹), 2.31 (ddd, 1H, ²*J* = ³*J*₇ₐ₆ₐ = 14.0 Hz, ³*J*₇ₐ₆ₑ = 6.0 Hz, Hₐ⁷), 3.08 (d, 1H, ²*J* = 13.8 Hz, H¹²), 3.61 (dd, 1H, ²*J* = 13.8 Hz, ⁴*J*_{12',11} = 0.6 Hz, H'¹²), 4.52 (m, 1H, Hₑ¹), 7.50-7.58 (m, 3H, H^{15, 15'} + H¹⁶), 7.66-7.68 (m, 2H, H^{14, 14'}). ¹³C NMR (100 MHz, CDCl₃, δ): 22.36 (Me¹⁷CO), 22.43 (Me¹⁰), 23.36 (C⁶H₂), 23.55 (Me¹¹), 24.16 (C⁹H₂), 29.09 (C⁵H), 32.98 (C⁷H₂), 64.54 (C¹²H₂), 77.58 (C¹H), 82.37 (C⁸), 82.53 (C⁴), 123.65 (2C¹⁴H), 129.35 (2C¹⁵H), 131.10 (C¹⁶H), 144.38 (C¹³), 170.10 (C=O). Isomer **41b'** (96 mg, 0.272 mmol), a colorless solid, mp 105-107°C, R_{f} 0.36 (hexane-EtOAc 1:1). ¹H NMR (400 MHz, CDCl₃, δ): 1.62 (s, 3H, Me¹⁰), 1.76 (dddd, 1H, ³*J*₆ₐ₇ₐ = 14.2 Hz, ²*J*₆ₐ₆ₑ = 12.5 Hz, ³*J*₆ₐ₅ₑ = 7.2 Hz, ³*J*₆ₐ₇ₑ = 3.3 Hz, Hₐ⁶), 1.83 (ddd, 1H, ²*J* = 13.6 Hz, ³*J*₉ₐ₅ₑ = 3.3 Hz, ³*J*₉ₐ₁ₑ = 1.8 Hz, Hₐ⁹), 1.89 (br.s, 3H, Me¹¹), 1.90 (m, 1H, Hₑ⁵), 1.93-2.02 (m, 1H, ²*J*₆ₑ₆ₐ ≈ 12.5 Hz, Hₑ⁶), 2.02 (s, 3H, Me¹⁷CO), 2.07 (m, 1H, ²*J*₇ₐ₇ₑ = 14.8 Hz, ³*J*₇ₐ₆ₐ = 14.2 Hz, ³*J*₇ₐ₆ₑ = 5.6 Hz, Hₐ⁷), 2.14 (m, 1H, ²*J*₇ₑ₇ₐ ≈ 14.8 Hz, ³*J*₇ₑ₆ₑ = 6.6 Hz, Hₑ⁷), 2.35 (br.ddd, 1H, ²*J* = 13.6 Hz, ³*J*₉ₑ₅ₑ = 6.8 Hz, ³*J*₉ₑ₁ₑ = 3.3 Hz, Hₑ⁹), 2.67 (br.d, 1H, ²*J* = 13.6 Hz, H¹²), 3.10 (d, 1H, ²*J* = 13.6 Hz, H'¹²), 4.49 (br.d, ³*J*₁ₑ₉ₑ = 3.3 Hz, Hₑ¹), 7.49-7.57 (m, 3H, H^{15, 15'}+ H¹⁶), 7.63-7.67 (m, 2H, H^{14, 14'}); ¹³C NMR (100 MHz, CDCl₃, δ): 22.05 (Me¹⁰), 22.47 (Me¹⁷CO), 22.59 (Me¹¹), 23.46 (C⁶H₂), 23.80 (C⁹H₂), 31.60 (C⁵H), 32.92 (C⁷H₂), 65.82 (C¹²H₂), 78.34 (C¹H), 82.64 (C⁸), 82.81 (C⁴), 123.81 (2C¹⁴H), 129.42 (2C¹⁵H), 131.16 (C¹⁶H), 144.90 (C¹³), 170.22 (C=O). The most polar isomer **41 a'** (122 mg, 0.346 mmol), a colorless solid, mp 98-100°C, R_{f} 0.29 (hexane-EtOAc 1:1). ¹H NMR (400 MHz, (CDCl₃, δ): 1.56 (br.s, 3H, Me¹¹), 1.68 (s, 3H, Me¹⁰), 1.78 (dddd, 1H, ²*J* ≈ ³*J*₆ₐ₇ₐ = 14.0 Hz, ³*J*₆ₐ₅ₑ = 6.0 Hz, ³*J*₆ₐ₇ₑ = 3.2 Hz, Hₐ⁶), 1.80-1.88 (m, 2H, Hₑ⁵ + Hₐ⁹), 1.95 (m, 1H, ²*J* ≈ 14.0 Hz, Hₑ⁶), 2.01 (s, 3H, Me¹⁷CO), 2.20 (br.dd, 1H, ²*J* ≈ 14.8 Hz, ³*J*₇ₑ₆ₑ = 6.2 Hz, Hₑ⁷), 2.30 (ddd, 1H, ²*J* = 14.8 Hz, ³*J*₇ₐ₆ₑ = 5.9 Hz, Hₐ⁷), 3.31 and 3.355 (ABq, 2H, ²*J* = 14.0 Hz, H¹² + H'¹²), 4.47 (br.d, 1H, ³*J*₁ₑ₉ₑ = 3.6 Hz, Hₑ¹), 7.48-7.57 (m, 3H, H^{15, 15'} + H¹⁶), 7.66-7.69 (m, 2H, H^{14, 14'}). ¹³C NMR (100 MHz, CDCl₃, δ): 22.23 (Me¹¹), 22.48 (Me¹⁷CO), 22.53 (Me¹⁰), 23.42 (C⁶H₂), 24.17 (C⁹H₂), 30.90 (C⁵H), 33.20 (C⁷H₂), 66.04 (C¹²H₂), 77.75 (C¹H), 82.25 (C⁸), 82.67 (C⁴), 123.82 (2C¹⁴H), 129.35 (2C¹⁵H), 130.92 (C¹⁶H), 144.86 (C¹³), 170.22 (C=O).

### Example 34: Preparation of (1R,4R,5R)-4,8-dimethyl-4-phenylsulfinylmethyl-2,3-dioxabicyclo, [3.3.1]non-7-enes (42a,a'), (1R,4S,5R)-4,8-dimethyl-4-phenylsulfinylmethyl-2,3-dioxabicyclo [3.3.1]non-7-enes (42b,b'), (1R,4R,5R)-4-methyl-8-methylidene-4-phenylsulfinylmethyl-2,3-dioxabicyclo[3.3.1]nonanes (43a,a') and (1R,4S,5R)-4-methyl-8-methylidene-4-phenylsulfinylmethyl-2,3-dioxabicyclo[3.3.1]-nonanes (43b,b') (Scheme 15).

To a solution of sulfides **9 a,b** and **10 a,b** (138 mg, 0.50 mmol) in EtOAc (10 mL) at -30 ö -35°C was added a solution of MCPBA (128 mg of *ca*. 70%, *ca*. 0.52 mmol) in EtOAc (8 mL). The mixture was stirred at -30 ö -40°C for 1 h, poured into saturated NaHCO₃ (50 mL), extracted with EtOAc (3 x 30 mL), dried (Na₂SO₄ + NaHCO₃) and evaporated. The mixture of eight sulfoxides was fractionated by MPLC (hexane-EtOAc, from 1:4 to 1:1) to give 3 fractions of sulfoxides, total yield 135 mg (0.462 mmol, 92.3%). (i) The least polar fraction, separated 18 mg (total 0.061 mmol), a colorless solid, mp 136-139°C, R_{f} 0.49 (hexane-EtOAc 1:1), consisted of **42b** and **43b** (**42b/43b** *ca*. 85: 15). ¹H NMR (400 MHz, CDCl₃, δ): 1.625 (ddd, ²*J* = 13.4 Hz, ³*J*₉ₐ₅ₑ = 3.1 Hz, ³*J*₉ₐ₁ₑ = 1.9 Hz, Hₐ⁹ **42b**) and 1.70 (ddd, ²*J* = 13.1 Hz, ³*J*₉ₐ₅ₑ = 3.0 Hz, ³*J*₉ₐ₁ₑ = 2.2 Hz, Hₐ⁹ **43b**), total 1H; 1.79 (br.dd, *ca*. 2.5H, ⁴*J*_{10,7} = 3.8 Hz, ⁵*J*_{10,6e} = 2.0 Hz, Me¹⁰ **42b**); 1.810 (br.d, ⁴*J* = 0.55 Hz, Me¹¹ **43b**), 1.816 (br.d, ⁴*J* = 0.65 Hz, Me¹¹ **42b**), total 3H; 2.08 (m, *ca*. 0.15H, Hₑ⁶ **43b**); 2.16 (m, Hₑ⁵ **43b**) and 2.19 (m, Hₑ⁵ **42b**), total 1H; 2.27-2.42 (m, total *ca*. 2.3H); 2.60 (dddd, *ca*. 0.85H, ²*J* = 13.2 Hz, ³*J*₉ₑ₅ₑ = ³*J*₉ₑ₁ₑ = 3.5 Hz, ⁴*J*₉ₑ₆ₑ =1.2 Hz, Hₑ⁹ **42b**); 2.67 (m, *ca*. 0.15H, H⁷ **43b**); 2.77 (dd, ²*J* = 13.5 Hz, ⁴*J* = 0.65 Hz, H'¹² **42b**) and 2.855 (dd, ²*J* = 13.4 Hz, ⁴*J* = 0.55 Hz, H'¹² **43b**), total 1H; 2.88 (d, ²*J* = 13.5 Hz, H¹² **42b**) and 2.97 (d, ²*J* = 13.4 Hz, H¹² **43b**), total 1H; 4.17 (br.dd, ³*J*₁ₑ₉ₑ = 3.5 Hz, ³*J*₁ₑ₉ₐ = 1.9 Hz, Hₑ¹ **42b**) and 4.44 (br.dd, ³*J*₁ₑ₉ₑ = 3.5 Hz, ³*J*₁ₑ₉ₐ = 1.5 Hz, Hₑ¹ **43b**), total 1H; 4.930 (br.s, H¹⁰ **43b**) and 4.936 (br.s, H'¹⁰ **43b**), total *ca*. 0.3H; 5.73 (m, *ca*. 0.85H, H⁷ **42b**); 7.47-7.56 (m, total 3H, H^{15, 15'} + H¹⁶), 7.62-7.68 (m, total 2H, H^{14, 14'}); ¹³C NMR (100 MHz, CDCl₃, δ): 21.15 (Me¹⁰ **42b**), 23.17 (Me¹¹ **42b**, **43b**), 26.29 (C⁹H₂ **42b**), 27.20 (C⁶H₂ **43b**), 27.94 (C⁶H₂ **42b**), 29.80 (C⁹H₂ **43b**), 30.28 (C⁷H₂ **43b**), 30.35 (C⁵H **42b**), 31.58 (C⁵H **43b**), 65.47 (C¹²H₂ **43b**), 65.50 (C¹²H₂ **42b**), 76.83 (C¹H **42b**), 80.77 (C¹H **43b**), 82.19 (C⁴ **42b**), 113.31 (=C¹⁰H₂ **43b**), 123.74 (2C¹⁴H **42b**), 123.80 (2C¹⁴H **43b**), 126.00 (=C⁷H **42b**), 129.44 (2C¹⁵H **42b**), 129.51 (2C¹⁵H **43b**), 131.29 (C¹⁶H **42b**), 131.35 (C¹⁶H **43b**), 131.80 (C⁸= **42b**), 144.68 (C¹³ **42b**), 146.1 (C¹³ **43b**). The following fraction, separated 61 mg (total 0.209 mmol), colorless oil, R_{f} 0.43 (hexane-EtOAc 1:1).The fraction consisted of **42a , 43a , 42b'** and **43b'** (**42/43** *ca*. 94: 6; **42a/42b'** *ca*. 55: 45). ¹H NMR (400 MHz, CDCl₃, δ): 1.39 (br.d, ⁴*J* = 0.7 Hz, Me¹¹ **42a**), 1.875 (br.s, Me¹¹ **43b'**), 1.895 (br.s, Me¹¹ **42b'**), total 3H; 1.56 (m, Hₐ⁹ **43b'**), 1.63 (ddd, ²*J* = 13.1 Hz, ³*J*₉ₐ₅ₑ =3.2 Hz, ³*J*₉ₐ₁ₑ = 2.1 Hz, Hₐ⁹ **42b'**) and 1.75 (ddd, ²*J* = 13.4 Hz, ³*J*₉ₐ₅ₑ = ³*J*₉ₐ₁ₑ = 2.4 Hz, Hₐ⁹ **42a**), total *ca*. 1H; 1.765 (m, Me¹⁰ **42b'**) and 1.79 (m, Me¹⁰ **42a**), total *ca.* 3H; 1.99 (m, *ca*. 0.5H, Hₑ⁵ **42a**); 2.20 (m, *ca*. 0.5H, Hₐ⁶ **42a**); 2.26-2.34 (m, total *ca*. 1H, Hₑ⁵ + Hₐ⁶ **42b'**); 2.36-2.48 (m, total *ca*. 2H); 2.53-2.59 (m, total *ca*. 0.5H, Hₑ⁹ **42b', 43b'**); 2.55 (d, ²*J* = 13.8 Hz, H¹² **42b'**) and 3.13 (d, ²*J* = 13.9 Hz, H¹² **42a**), total 1H; 3.08 (br.d, ²*J* = 13.8 Hz, H'¹² **42b'**), 3.13 (br.d, ²*J* = 13.8 Hz, H'¹² **43b'**) and 3.66 (dd, ²*J* = 13.9 Hz, ⁴*J* = 0.7 Hz, H'¹² **42a**), total 1H; 4.15 (m, Hₑ¹ **42a, 42b'**) and 4.43 (br.d, Hₑ¹ **43a, 43b'**), total 1H; 4.895 (br.s, H¹⁰ **43a,b'**) and 4.90 (br.s, H'¹⁰ **43a,b'**), total *ca*. 0.12H; 5.68 (m, H⁷ **42b'**) and 5.78 (m, H⁷ **42a**), total *ca*. 0.95H; 7.46-7.56 (m, total 3H, H^{15, 15'} + H¹⁶), 7.61-7.68 (m, total 2H, H^{14, 14'}); ¹³C NMR (100 MHz, CDCl₃, δ): 21.08 (Me¹⁰ **42a, 42b'**), 21.87 (Me¹¹ **42b'**), 22.10 (Me¹¹ **43b'**), 24.09 (Me¹¹ **42a**), 26.20 (C⁹H₂ **42b'**), 26.50 (C⁹H₂ **42a**), 27.38 (C⁶H₂ **42a**), 27.78 (C⁶H₂ **42b'**), 29.21 (C⁵H **42b'**), 31.53 (C⁵H **42a**), 64.83 (C¹²H₂ **42a**), 66.19 (C¹²H₂ **42b'**), 76.42 (C¹H **42a**), 77.05 (C¹H **42b'**), 80.94 (C¹H **43a,b'**), 81.94 (C⁴ **42a**), 82.11 (C⁴ **42b'**), 113.10 (=C¹⁰H₂ **43a,b'**), 123.72 (2C¹⁴H **42a**), 123.76 (2C¹⁴H **42b'**), 126.60 (=C⁷H **42b'**), 126.93 (=C⁷H **42a**), 129.29(2C¹⁵H **42b'**), 129.34 (2C¹⁵H **42a**), 130.99 (C⁸= **42b'**), 131.01 (C¹⁶H **42b'**), 131.06 (C¹⁶H **42a**), 131.09 (C⁸= **42a**), 144.60 (C¹³ **42a**), 144.77 (C¹³ **42b'**), 146.28(C¹³ **43a,b'**). The most polar fraction, 56 mg (total 0.192 mmol), a colorless oil , R_{f} 0.35 (hexane-EtOAc 1:1).The fraction consisted of **42a'** and **43a'** (**42a'/43a'** *ca*. 91: 9). The isomers **42a'** and **43a'** were separated by additional MPLC (hexane-EtOAc 2:3) of the last fraction. Less polar isomer **43a'** (*ca*. 95% purity) was isolated as a colorless waxy solid, mp 78-82°C. ¹H NMR (400 MHz, CDCl₃, δ): 1.58 (m, 1H, Hₐ⁹), 1.61 (br.s, 3H, Me¹¹), 1.72 (dddd, 1H, ²*J* = ³*J*₆ₐ₇ₐ = 13.8 Hz, ³*J*₆ₐ₅ₑ = 6.4 Hz, ³*J*₆ₐ₇ₑ = 3.7 Hz, Hₐ⁶), 2.04 (dddd, 1H, ³*J*₅ₑ₆ₐ = ³*J*₅ₑ₉ₑ = 6.4 Hz, ³*J*₅ₑ₆ₑ = ³*J*₅ₑ₉ₐ = 3.2 Hz, Hₑ⁵), 2.18 (m, 1H, ²*J* = 13.8 Hz, Hₑ⁶), 2.41 (br.dd, 1H, ²*J* = 15.2 Hz, ³*J*₇ₑ₆ₑ = 6.0 Hz, Hₑ⁷), 2.51 (br.ddd, 1H, ²*J* = 13.6 Hz, ³*J*₉ₑ₅ₑ = 6.4 Hz, ³*J*₉ₑ₁ₑ = 3.5 Hz, Hₑ⁹), 3.06 (m, 1H, Hₐ⁷), 3.28 (br.d, 1H, ²*J* = 14.0 Hz, H¹²), 3.50 (br.d, 1H, ²*J* = 14.0 Hz, H'¹²), 4.37 (br.dd, 1H, ³*J*₁ₑ₉ₑ = 3.5 Hz, ³*J*₁ₑ₉ₐ = 1.5 Hz, Hₑ¹), 4.93 (br.dd, 1H, ²*J* ≈⁴*J*_{10,7a} = 2.2 Hz, H¹⁰), 4.96 (m, 1H, H'¹⁰), 7.52 (m, 1H, H¹⁶), 7.55 (m, 2H, H^{15, 15'}), 7.69 (m, 2H, H^{14, 14'}); ¹³C NMR (100 MHz, CDCl₃, δ): 22.48 (Me¹¹), 27.00 (C⁶H₂), 30.05 (C⁹H₂), 30.47 (C⁷H₂), 31.69 (C⁵H), 66.24 (C¹²H₂), 80.61 (C¹H), 82.49 (C⁴), 113.64 (=C¹⁰H₂), 123.85 (2C¹⁴H), 129.33 (2C¹⁵H), 130.91 (C¹⁶H), 146.22 (C¹³), 154.64 (C⁸). More polar isomer **42a'**, a colorless solid, mp 90-92°C. ¹H NMR (400 MHz, CDCl₃, δ): 1.51(br.s, 3H, Me¹¹), 2.64 (ddd, 1H, ²*J* = 13.3 Hz, ³*J*₉ₐ₅ₑ = 3.0 Hz, ³*J*₉ₐ₁ₑ = 2.2 Hz, Hₐ⁹), 1.79 (ddd, 3H, ⁴*J*_{10,7} = 2.7 Hz, ⁵*J*_{10,6a} ≈ ⁵*J*_{10,6e} = 1.6 Hz, Me¹⁰), 1.99 (m, 1H, Hₑ⁵), 2.23 (dddq, 1H, ²*J* = 19.2 Hz, ³*J*₆ₐ₇ = 5.4 Hz, ³*J*₆ₐ₅ₑ ≈ ⁵*J*₆ₐ₁₀ = 1.6 Hz, Hₐ⁶), 2.37-2.47 (m, 2H, Hₑ⁶ + Hₑ⁹), 3.30 (d, 1H, ²*J* = 14.0 Hz, H¹²), 3.44 (dd, 1H, ²*J* = 14.0 Hz, ⁴*J* = 0.35 Hz, H'¹²), 4.12 (m, 1H, ³*J*₁ₑ₉ₐ = ³*J*₁ₑ₉ₐ = 2.2 Hz, Hₑ¹), 5.78 (m, 1H, H⁷), 7.50 (m, 1H, H¹⁶), 7.53 (m, 2H, H^{15, 15'}), 7.68 (m, 2H, H^{14, 14'}); ¹³C NMR (100 MHz, CDCl₃, δ): 21.10 (Me¹⁰), 22.70 (Me¹¹), 26.47 (C⁹H₂), 27.35 (C⁶H₂), 30.95 (C⁵H), 66.09 (C¹²H₂), 76.54 (C¹H), 81.77 (C⁴), 123.79 (2C¹⁴H), 126.98 (C⁷H), 129.27 (2C¹⁵H), 130.82 (C¹⁶H), 131.56 (C⁸=), 144.93 (C¹³).

### Example 35. Determination of in vitro antimalarial activity of the compounds A.

The protozoan *Plasmodium falciparum* is a causative agent of malaria. The antimalarial activity of 2,3-dioxabicyclo[3.3.1]nonanes of type A was determined by measuring the incorporation of [³H]hypoxanthine, by the methods of Desjardins⁹ and Milhous,¹⁰ into chloroquinone-sensitive *Plasmodium falciparum* (NF54)¹¹ in the presence of varying drug concentrations. Dose-response curves that were fit to the experimental data by means of the Marquardt algorithm¹² were solved for the drug concentration that kills 50% of parasites (IC₅₀), and were analyzed for accuracy of fit (*R*² value, typically ≥0.99). Provisional IC₅₀ values were obtained in a survey of seven 5-fold dilutions (in triplicate) of stock solutions of chromatographically-purified or recrystallized test compound. Assays were later expanded to include ten concentrations (in quadruplicate) of ∼1.8-fold dilutions, which flank the provisional IC₅₀.

Tables 1-3 present tabulations of a number of compounds that have been synthesized along with the IC₅₀ (in nM).

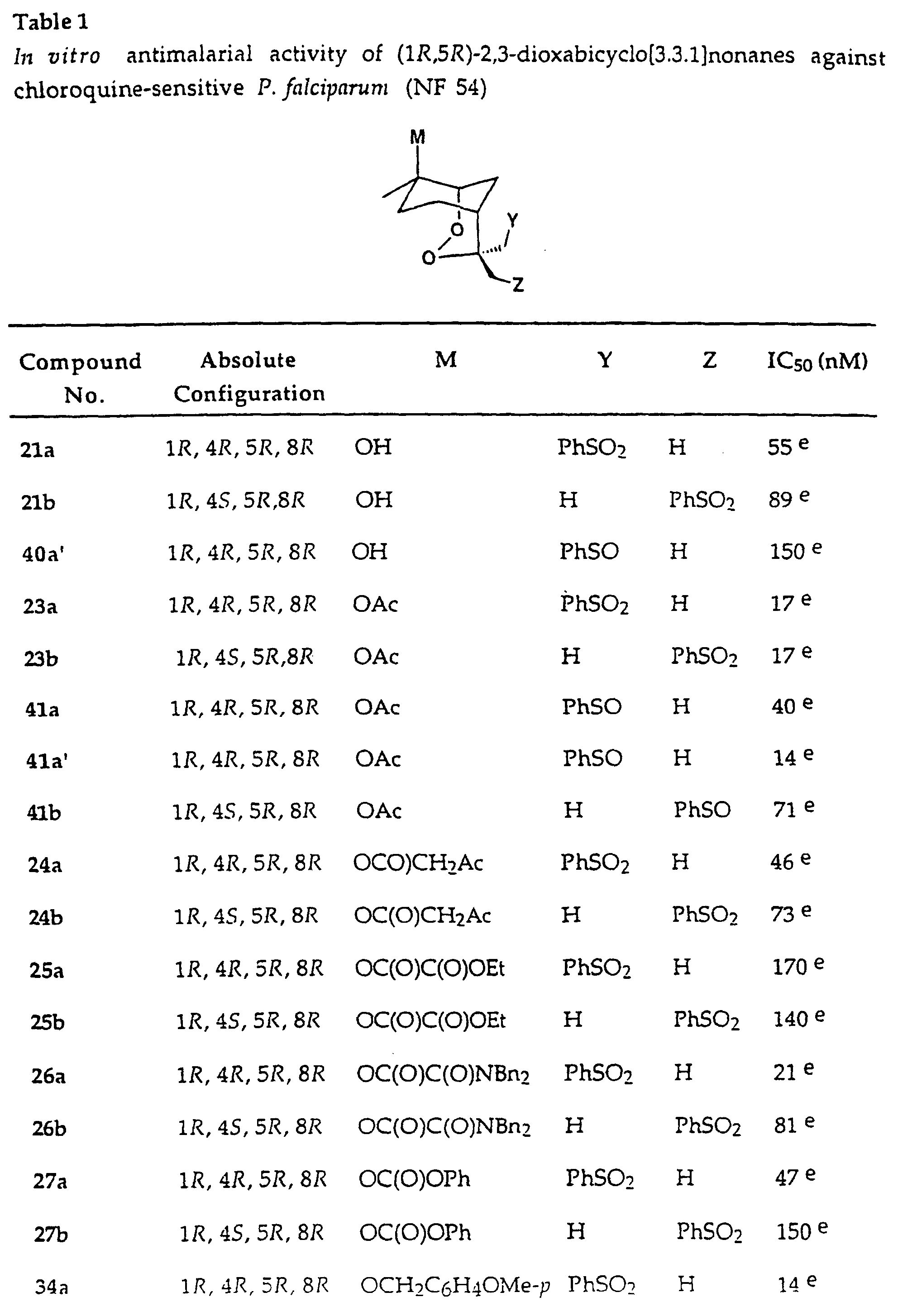

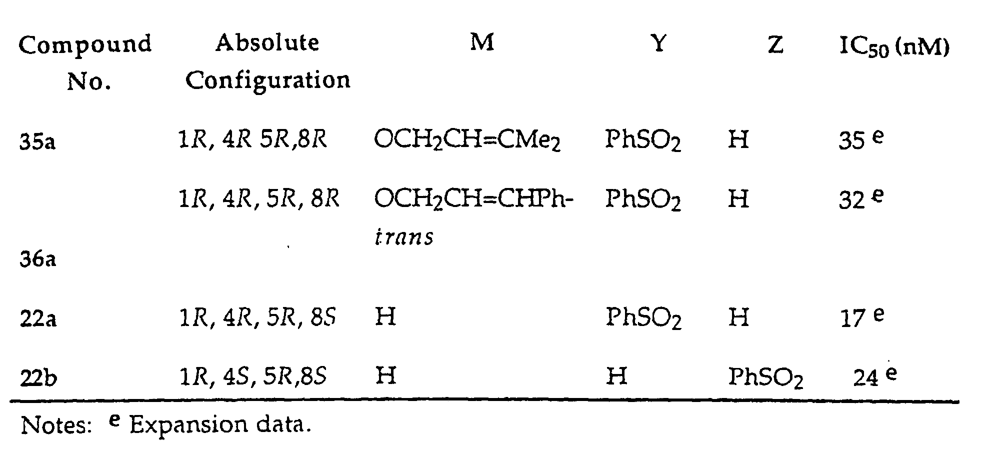

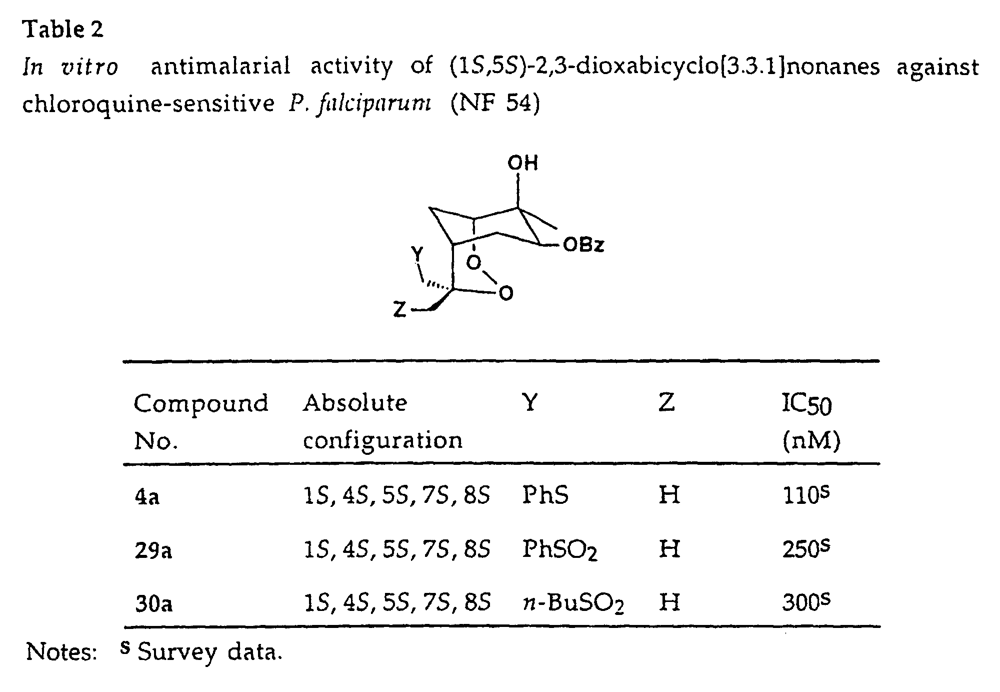

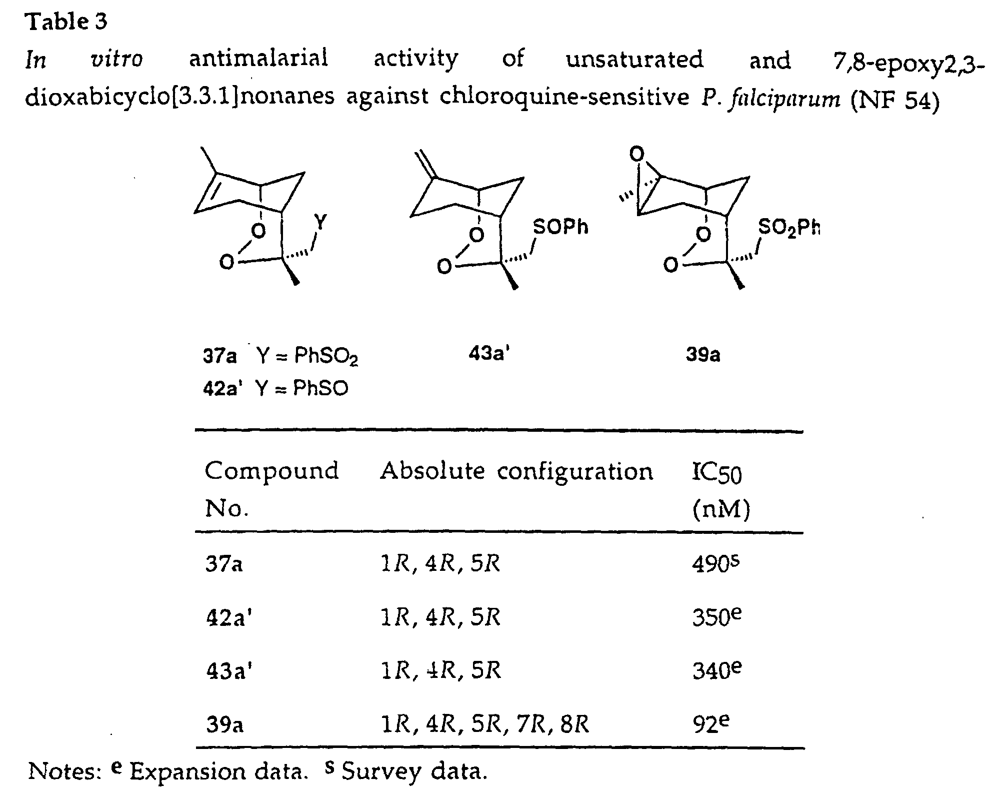

The results summarized in Tables 1-3 indicate that several of these compounds exhibit *in vitro* antimalarial activity of the same order of magnitude as that recorded under the same conditions for artemisin (IC₅₀ = 11 nM).¹³

### Example 36: Determination of in vivo antimalarial activity of the 2,3-dioxabicyclo[3.3.1]nonanes of formula A

Based on the *in vitro* antimalarial activity of compounds of type **A**, the *in vivo* antimalarial activity of two representatives, namely of compounds **23a** and **34a**, were tested. The results are described in Table 4.

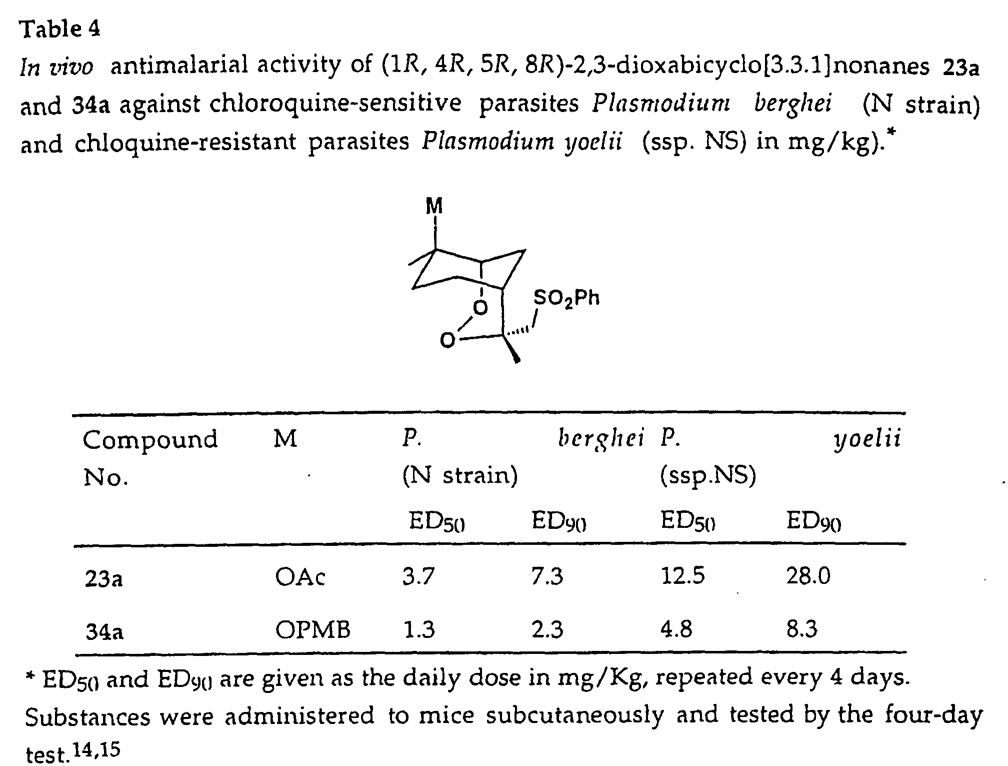

*In vivo* antimalarial activity of compounds **23a** and **34a** is similar to that of artemisinin (ED₅₀ = 0.95; and ED₉₀ = 2.5 against *P. berghei* and ED₅₀ = 5.8; and ED₉₀ = 10.0 against *P. yoelii*)¹⁵ and of arteflene (ED₅₀ = 2.7; and ED₉₀ = 3.9 against *P. berghei*).¹⁶

### REFERENCES

(1) *TDR News* (News from the WHO Division of Control of Tropical Diseases), **1994**,*46*,5.
(2) Cumming, J. N.; Ploypradith, P.; Posner, G. H. *Advances in Pharmacology* **1997***, 37*, 253.
(3) Ong, H. H.; Allen, R. C. *Ann. Rep. Med. Chem*. **1988**, *23*, 325.
(4) Tang, W.; Eisenbrand, G. In "Chinese Drugs of Plant Origin. Chmistry, Pharmacology, and use of Traditional and Modern Medicine "Springer-Verlag: Berlin, 1992; pp 159-174.
(5) Hofheinz, W.; Schmid, G.; Stohler, H. Eur. Pat. Appl. 311955, **1989**; C. A. *112*, **1990**,20999.
(6) Hofheinz, W.; Burgin, H.; Gocke, E.; Jaquet, C.; Masciadri, R.; Schmid, G.; Stohler, H.; Urwyler, H. *Trop. Med. Parasitol.* **1994**, *45*, 261.
(7) Jaquet, C.; Stohler, H. R.; Chollet, J.; Peters, W. *Trop. Med. Parasitol.* **1994**, *45*, 266.
(8) Kamchonwongpaisan, S.; McKeever, P.; Hossler, P.; Ziffer, H.; Meshnick, S. R. *Am, J*. *Trop. Med. Hyg*., **1997**, *56*, 7.
(9) Dejardins, R. E.; Canfield, C. J.; Haynes, J. D.; Chulay, J. D. *Antimicrob. Agents Chemother*. **1979**, *16*, 710.
(10) Milhous, W. K.; Weatherly, N F.; Bowdre, J. H.; Desjardins, R. *Antimicrob*. *Agents Chemother*. **1985**, *27*, 525.
(11) Ponnudurai, T.; Leeuwenberg, A. D. E. M.; Meuwissen, J. H. E. T. *Trop*. *Georgr*. *Med*. **1981**, *33*, 50.
(12) Bard, Y. *Nonlinear Parameter Estimation;* Academic Press; New York, **1974**, p.94.
(13) Posner, G. H.; Park, S. B.; González, L.; Wang, D.; Cumming, J. N.; Klinedinst, D.; Shapiro, T. A.; Bachi, M. D. *J. Am. Chem. Soc*. **1996**, *118*, 3537.
(14) Peters, W.; Robinson, B.L.; Rossier, J.C.; Jefford C.W. *Ann*. *Trop. Med. Parasitol*. **1993,** *87*, 1.
(15) Jefford, C.W.; Kohmoto, S.; Jaggi, D.; Timari, G.; Rossier, J.-C.; Rudaz, M. Barbuzzi, O.; Gerard, D.; Burger, U.; Kamalaprija, P.; Mareda, J.; Bernardinelli, G.; Manzanares, I.; Canfield, C.J.; Fleck, S.L.; Robinson, B.L.; Peters, W. *Helv. Chim. Acta*, **1995**, *78*, 647.
(16) Jaquet, C.; Stohler, R.H.; Chollet, J.; Peters, W. *Trop. Med. Parasitol*. **1994**, *45,* 266.

## Claims

1. A 2,3-dioxabicydo[3.3.1]nonane derivative, carrying, at position 4, a sulfur-containing functionality selected from the group consisting of sulfonyl, sulfinyl and sulfenyl, adhered to C(4) via methylene group, represented by the general structural formula **A**. 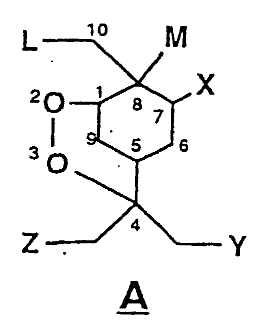 wherein:
X is hydrogen, hydroxy, C₁- C₂₀ alkoxy optionally substituted by C₁-C₂₀ alkoxy or by C₂-C₂₀ acyloxy, C₇-C₂₅ aralkoxy optionally substituted by C₁-C₂₀ alkoxy or by C₆-C₁₂ aryloxy, or C₂-C₂₀ acyloxy optionally substituted by C₁-C₂₀ alkoxy or by C₆-C₁₂ aryloxy; and
M is hydrogen, hydroxy, C₁-C₂₀ alkoxy, C₃-C₂₀ alkenyloxy, C₂-C₂₀ acyloxy optionally substituted by C₂-C₂₀ acyl or by C₂-C₂₀ acyloxy, C₇-C₂₅ aralkoxy, C₇-C₂₅ arylalkenyloxy, oxalyloxy substituted by C₁-C₂₀ alkoxy, di(C₁-C₂₀ alkyl)amino, C₁-C₂₀ alkyl(C₆-C₁₂ aryl)amino, or di(C₇-C₂₅ aralkyl)amino, or carbonyloxy substituted by C₆-C₁₂ aryloxy, di(C₁-C₂₀ alkyl)amino, C₁-C₂₀ alkyl (C₆-C₁₂ aryl)ammo, or di(C₇-C₂₅ aralkyl)amino; or
X and M together represent a carbon-carbon bond or an oxygen atom;
L is hydrogen or Land M together represent a carbon-carbon bond; and
either Z is a radical R-S(=O)ₙ- and Y is hydrogen, or
Y is R-S(=O)ₙ- and Z is hydrogen,
wherein R is straight or branched C₁-C₂₀ alkyl optionally substituted by C₁-C₂₀ alkoxy or by C₂-C₂₁ alkoxycarbonyl, C₅-C₇ cycloalkyl, C₆-C₁₂ aryl optionally substituted by C₁-C₂₀ alkyl, halogen or CF₃, or C₇-C₂₅ arylalkyl optionally substituted by C₁-C₂₀ alkyl, halogen or CF₃ ; and
n is 0, 1 or 2.

2. A compound according to claim 1 wherein R is a radical selected from: *n*-butyl; *tert*-butyl; cyclohexyl; 3-octyl; *n*-dodecyl; octyloxyethyl; ethoxycarbonylmethyl; phenyl; biphenyl; 4-fluorophenyl; 2,4-dichlorophenyl; 2,4-difluorophenyl; 2-naphthyl; benzyl; 4-phenylbutyl; 4'-(trifluoromethyl)benzyl; (triphenyl)methyl; 4-methylphenyl; 4-(trifluoromethyl)phenyl; 2,4-dimethylphenyl; 2,4-[di-(trifluoromethyl)]phenyl; 3,5-[di-(trifluoromethyl)]phenyl.

3. A compound according to claim 1 wherein X is selected from: methoxy; hexyloxy; dodeyloxy; (butoxy)ethoxy; acetyloxy; benzoyloxy; diphenylacetyloxy; (hexanoyloxy)ethoxy; butanoyloxy; octanoyloxy; dodecanoyloxy; phenethyloxy; methoxyacetyloxy; phenoxyaceyloxy; biphenyl-4-carbonoyloxy.

4. A compound according to any of claims 1-3 wherein M is selected from: methoxy; octyloxy; allyloxy; 3-(methyl)but-2-enyloxy; 4-(methoxy)benzyloxy; cinnamyloxy; acetyloxy; acetylacetyloxy; 3-(acetyloxy)but-2-enoyloxy; ethoxyoxalyloxy; (methyl)phenylaminoxalyloxy; di(benzyl)aminooxalyloxy; phenoxycarbonyloxy.

5. A compound according to claim 1 of structure **I characterized by** stereogenic centers (1*R*, 5*R*) of the formula: 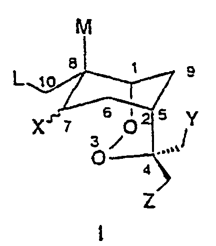 wherein L, M, X, Y and Z are as defined in claim 1.

6. A compound according to claim 1 of structure **II characterized by** stereogenic centers (1*S*, 5*S*) of the formula: 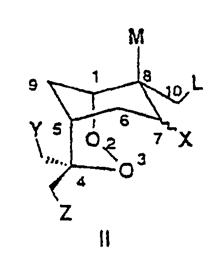 wherein L, M, X, Y and Z are as defined in claim 1.

7. A compound according to claim 5 of structure **Ia characterized by** stereogenic centers (4*R*) of the formula: 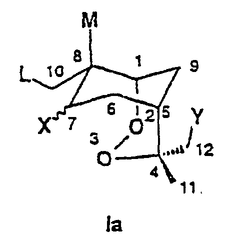 wherein L, M, X, Y are as defined in claim 1.

8. A compound according to claim 5 of structure **Ib characterized by** stereogenic centers (4*S*) of the formula: 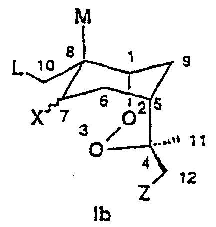 wherein L, M, X and Z are as defined in claim 1.

9. A compound according to claim 6 of structure **IIa characterized by** stereogenic centers (4*S*) of the formula: 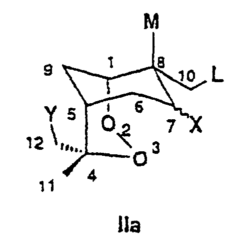 wherein L, M, X, Y are as defined in claim 1.

10. A compound according to claim 6 of structure **IIb characterized by** stereogenic centers (4*R*) of the formula: 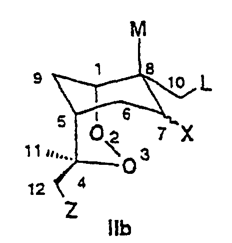 wherein L, M, X and Z are as defined in claim 1.

11. A compound according to claim 1 selected from: (1*R*,4*R*,5*R*,8*R*)-4,8-dimethyl-4-phenylsulfonylmethyl-2,3-dioxabicyclo [3.3.1]nonan-8-ol (**21a**); (1*R*,4*R*,5*R*,8*S*)-4,8-dimethyl-4-phenylsulfonylmethyl-2,3-dioxabicyclo [3.3.1]nonane (**22a**); (1*R*,4*S*,5*R*,8*S*)-4,8-dimethyl-4-phenylsulfonylmethyl-2,3-dioxabicyclo[3.3.1]nonane (**22b**); (1*R*,4*R*,5*R*,8*R*)-8-acetoxy-4,8-dimethyl-4-phenylsulfonylmethyl-2,3-dioxabicyclo [3.3.1]nonane (**23a**); (1*R*,4*S*,5*R*,8*R*)-8-acetoxy-4,8-dimethyl-4-phenylsulfonylmethyl-2,3-dioxabicyclo[3.3.1]nonane (**23b**); (1*R*,4*R*,5*R*,8*R*)-8-acetylacetoxy-4,8-dimethyl-4-phenylsulfonylmethyl-2,3-dioxabicyclo[3.3.1]nonane (**24a**); (1*R*,4*R*,5*R*,8*R*)-8-dibenzylaminooxalyloxy-4,8-dimethyl-4-phenylsulfonylmethyl-2,3-dioxabicyclo[3.3.1]nonane (**26a**); (1*R*,4*R*,5*R*,8*R*)-4,8-dimethyl-8-phenoxycarbonyloxy-4-phenylsulfonylmethyl-2,3-dioxabicyclo[3.3.1]nonane (**27a**); (1*R*,4*R*,5*R*,8*R*)-4,8-dimethyl-8-phenoxyacetoxy-4-phenylsulfonylmethyl-2,3-dioxabicyclo[3.3.1]nonane (**33a**); (1*R*,4*R*,5*R*,8*R*)-4,8-dimethyl-8-(*p*-methoxybenzyloxy)-4-phenylsulfonylmethyl-2,3-dioxabicyclo[3.3.1]nonane (**34a**); (1*R*,4*R*,5*R*,8*R*)-4,8-dimethyl-8-(γ,γ-dimethylallyloxy)-4-phenylsulfonylmethyl-2,3-dioxabicyclo [3.3.1]nonane (**35a**); (1*R*,4*R*,5*R*,8*R*)-8-(*trans*-cinnamyloxy)-4,8-dimethyl-4-phenylsulfonylmethyl-2,3-dioxabicyclo [3.3.1]nonane (**36a**); (1*R*,4*R*,5*R*,8*R*)-8-acetoxy-4,8-dimethyl-4-phenylsulfinylmethyl-2,3-dioxabicyclo[3.3.1]nonanes (**41a, a'**); (1*R*,4*S*,5*R*,8*R*)-8-acetoxy-4,8-dimethyl-4-phenylsulfinylmethyl-2,3-dioxabicyclo[3.3.1]nonane (**41b'**).

12. Pharmaceutical composition comprising a compound as defined in any of claims 1-11 and a pharmaceutically acceptable carrier.

13. Use of a compound as defined in any of claims 1-11 for the preparation of a pharmaceutical composition for the prevention and/or treatment of malaria.

## Patentansprüche

1. 2,3-Dioxabicyclo[3.3.1]nonanderivat, welches an der Position 4 eine schwefelhaltige Funktion trägt, ausgewählt aus Sulfonyl, Sulfinyl und Sulfenyl, welche an C(4) über eine Methylengruppe binden, dargestellt durch die allgemeine Strukturformel A 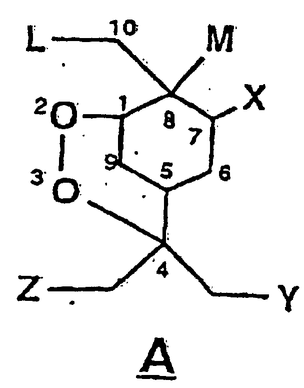 wobei:
X ein Wasserstoffatom, Hydroxy, C₁-C₂₀-Alkoxy, gegebenenfalls substituiert durch C₁-C₂₀-Alkoxy oder C₂-C₂₀-Acyloxy, C₇-C₂₅-Aralkoxy, gegebenenfalls substituiert durch C₁-C₂₀-Alkoxy oder durch C₆-C₁₂-Aryloxy, oder C₂-C₂₀-Acyloxy, gegebenenfalls substituiert durch C₁-C₂₀-Alkoxy oder durch C₆-C₁₂-Aryloxy, darstellt; und
M ein Wasserstoffatom, Hydroxy, C₁-C₂₀-Alkoxy, C₃-C₂₀-Alkenyloxy, C₂-C₂₀-Acyloxy, gegebenenfalls substituiert durch C₂-C₂₀-Acyl oder durch C₂-C₂₀-Acyloxy, C₇-C₂₅-Aralkoxy, C₇-C₂₅-Arylalkenyloxy, durch C₁-C₂₀-Alkoxy substituiertes Oxalyloxy, Di(C₁-C₂₀-alkyl)amino, C₁-C₂₀-Alkyl(C₆-C₁₂-aryl)amino, oder Di(C₇-C₂₅-aralkyl)amino, oder durch C₆-C₁₂-Aryloxy substituiertes Carbonyloxy, Di(C₁-C₂₀-alkyl)amino, C₁-C₂₀-Alkyl(C₆-C₁₂-aryl)amino, oder Di(C₇-C₂₅-aralkyl)amino darstellt; oder
X und M zusammen eine Kohlenstoff-Kohlenstoff-Bindung oder ein Sauerstoffatom darstellen;
L ein Wasserstoffatom darstellt oder L und M zusammen eine Kohlenstoff-Kohlenstoff-Bindung darstellen; und
entweder Z einen Rest R-S(=O)ₙ- und Y ein Wasserstoffatom darstellt, oder
Y für R-S(=O)ₙ- steht und Z ein Wasserstoffatom darstellt,
wobei R ein geradkettiger oder verzweigter C₁-C₂₀-Alkylrest, welcher gegebenenfalls durch C₁-C₂₀-Alkoxy oder C₂-C₂₁-Alkoxycarbonyl substituiert ist, C₅-C₇-Cycloalkyl, C₆-C₁₂-Aryl, gegebenenfalls substituiert durch C₁-C₂₀-Alkyl, Halogen oder CF₃, oder C₇-C₂₅-Arylalkyl, gegebenenfalls substituiert durch C₁-C₂₀-Alkyl, Halogen oder CF₃ ist; und
n gleich 0, 1 oder 2 ist.

2. Verbindung gemäß Anspruch 1, wobei R ein Rest ist, ausgewählt aus *n*-Butyl, *tert*-Butyl, Cyclohexyl, 3-Octyl, *n*-Dodecyl, Octyloxyethyl, Ethoxycarbonylmethyl, Phenyl, Biphenyl, 4-Fluorphenyl, 2,4-Dichlorphenyl, 2,4-Difluorphenyl, 2-Naphthyl, Benzyl, 4-Phenylbutyl, 4'-(Trifluormethyl)benzyl, (Triphenyl)methyl, 4-Methylphenyl, 4-(Trifluormethyl)phenyl, 2,4-Dimethylphenyl, 2,4-[Di-(trifluormethyl)]phenyl, 3,5-[Di-(trifluormethyl)]phenyl.

3. Verbindung gemäß Anspruch 1, wobei X ausgewählt ist aus: Methoxy, Hexyloxy, Dodecyloxy, (Butoxy)ethoxy, Acetyloxy, Benzoyloxy, Diphenylacetyloxy, (Hexanoyloxy)ethoxy, Butanoyloxy, Octanoyloxy, Dodecanoyloxy, Phenethyloxy, Methoxyacetyloxy, Phenoxyacetyloxy, Biphenyl-4-carbonoyloxy.

4. Verbindung gemäß einem der Ansprüche 1 bis 3, wobei M ausgewählt ist aus: Methoxy, Octyloxy, Allyloxy, 3-(Methyl)but-2-enyloxy, 4-(Methoxy)benzyloxy, Cinnamyloxy, Acetyloxy, Acetylacetyloxy, 3-(Acetyloxy)but-2-enoyloxy, Ethoxyoxalyloxy, (Methyl)phenylaminoxalyloxy, Di(benzyl)aminooxalyloxy, Phenoxycarbonyloxy.

5. Verbindung gemäß Anspruch 1 der Struktur **I**, **gekennzeichnet durch** stereogene Zentren (1R, 5R) der Formel: 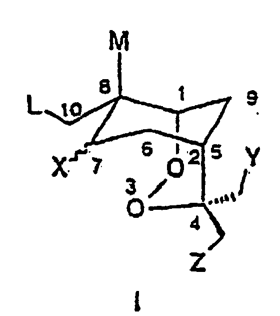 wobei L, M, X, Y und Z wie in Anspruch 1 definiert sind.

6. Verbindung gemäß Anspruch 1 der Struktur **II**, **gekennzeichnet durch** stereogene Zentren (1S, 5S) der Formel: 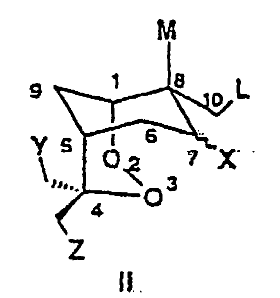 wobei L, M, X, Y und Z wie in Anspruch 1 definiert sind.

7. Verbindung gemäß Anspruch 5 der Struktur Ia, **gekennzeichnet durch** stereogene Zentren (4R) der Formel: 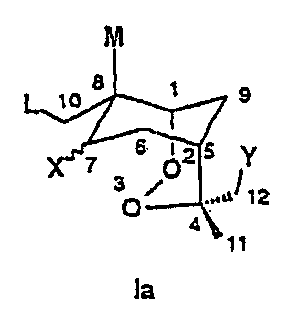 wobei L, M, X, Y wie in Anspruch 1 definiert sind.

8. Verbindung gemäß Anspruch 5 der Struktur Ib, **gekennzeichnet durch** stereogene Zentren (4S) der Formel: 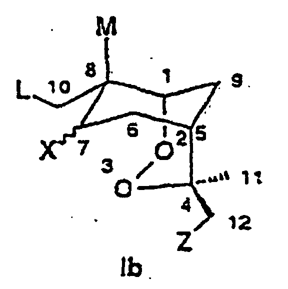 wobei L, M, X und Z wie in Anspruch 1 definiert sind.

9. Verbindung gemäß Anspruch 6 der Struktur IIa, **gekennzeichnet durch** stereogene Zentren (4S) der Formel: 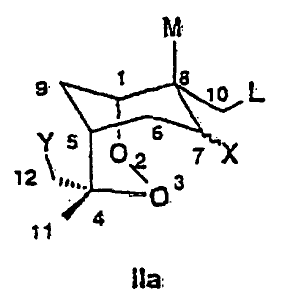 wobei L, M, X, Y wie in Anspruch 1 definiert sind.

10. Verbindung gemäß Anspruch 6 der Struktur IIb, **gekennzeichnet durch** stereogene Zentren (4R) der Formel: 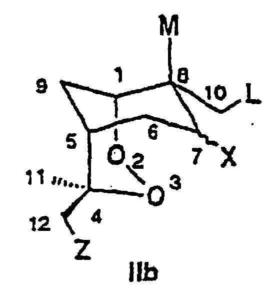 wobei L, M, X und Z wie in Anspruch 1 definiert sind.

11. Verbindung gemäß Anspruch 1 ausgewählt aus: (1R,4R,5R,8R)-4,8-Dimethyl-4-phenylsulfonylmethyl-2,3-dioxabicyclo[3.3.1]nonan-8-ol (21a); (1R,4R,5R,8S)-4,8-Dimethyl-4-phenylsulfonylmethyl-2,3-dioxabicyclo[3.3.1]nonan (22a); (1R,4S,5R,8S)-4,8-Dimethyl-4-phenylsulfonylmethyl-2,3-dioxabicyclo[3.3.1]nonan (22b); (1R,4R,5R,8R)-8-Acetoxy-4,8-dimethyl-4-phenylsulfonylmethyl-2,3-dioxabicyclo-[3.3.1]nonan (23a); (1R,4S,5R,8R)-8-Acetoxy-4,8-dimethyl-4-phenylsulfonylmethyl-2,3-dioxabicyclo[3.3.1]nonan (23b); (1R,4R,5R,8R)-8-Acetylacetoxy-4,8-dimethyl-4-phenylsulfonylmethyl-2,3-dioxabicyclo[3.3.1]nonan (24a); (1R,4R,5R,8R)-8-Dibenzylaminooxalyloxy-4,8-dimethyl-4-phenylsulfonylmethyl-2,3-dioxabicyclo-[3.3.1]nonan (26a); (1R,4R,5R,8R)-4,8-Dimethyl-8-phenoxycarbonyloxy-4-phenylsulfonylmethyl-2,3-dioxabicyclo[3.3.1]nonan (27a); (1R,4R,5R,8R)-4,8-Dimethyl-8-phenoxyacetoxy-4-phenylsulfonylmethyl-2,3-dioxabicyclo[3.3.1]nonan (33a); (1R,4R,5R,8R)-4,8-Dimethyl-8-(*p*-methoxybenzyloxy)-4-phenylsulfonylmethyl-2,3-dioxabicyclo[3.3.1]nonan (34a); (1R,4R,5R,8R)-4,8-Dimethyl-8-(γ,γ-dimethylallyloxy)-4-phenylsulfonylmethyl-2,3-dioxabicyclo[3.3.1]nonan (35a); (1R,4R,5R,8R)-8-(*trans*-Cinnamyloxy)-4,8-dimethyl-4-phenylsulfonylmethyl-2,3-dioxabicyclo[3.3.1]nonan (36a); (1R,4R,5R,8R)-8-Acetoxy-4,8-dimethyl-4-phenylsulfinylmethyl-2,3-dioxabicyclo[3.3.1]nonanen (41a, a'); (1R,4S,5R,8R)-8-Acetoxy-4,8-dimethyl-4-phenylsulfinylmethyl-2,3-dioxabicyclo[3.3.1]nonan (41b').

12. Arzneimittel umfassend eine Verbindung wie in einem der Ansprüche 1 bis 11 definiert und einen pharmazeutisch verträglichen Träger.

13. Verwendung einer Verbindung wie in einem der Ansprüche 1 bis 11 definiert für die Herstellung eines Arzneimittels zur Vorbeugung und/oder Behandlung von Malaria.

## Revendications

1. Dérivé 2,3-dioxabicyclo[3.3.1]nonane, portant, en position 4, une fonctionnalité contenant un sulfure choisi dans le groupe constitué de sulfonyle, sulfinyle et sulfényle, adhérant au C(4) via un groupe méthylène, représenté par la formule de structure générale A 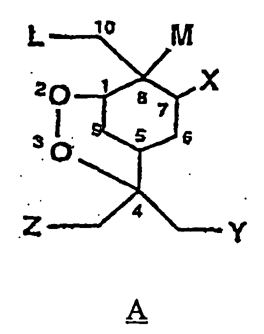 dans laquelle :
X est un hydrogène, un groupe hydroxy, alkoxy en C₁-C₂₀ éventuellement substitué par un alkoxy en C₁-C₂₀ ou par un acyloxy en C₂-C₂₀, un aralkoxy en C₇-C₂₅ éventuellement substitué par un alkoxy en C₁-C₂₀ ou par un aryloxy en C₆-C₁₂, ou un acyloxy en C₂-C₂₀ éventuellement substitué par un alkoxy en C₁-C₂₀ ou par un aryloxy en C₆-C₁₂ ; et
M est un hydrogène, un groupe hydroxy, alkoxy en C₁-C₂₀, alkényloxy en C₃-C₂₀, acyloxy en C₂-C₂₀ éventuellement substitué par un acyle en C₂-C₂₀ ou par un acyloxy en C₂-C₂₀, un aralkoxy en C₇-C₂₅, un arylalkényloxy, un oxalyloxy substitué par un alkoxy en C₁-C₂₀, un di(alkyle en C₁-C₂₀)amino, un alkyl(aryle en C₆-C₁₂)amino en C₁-C₂₀, ou di(aralkyle en C₇-C₂₅)amino, ou carbonyloxy substitué par un aryloxy en C₆-C₁₂, un di(alkyle en C₁-C₂₀)amino, un alkyl (aryle en C₆-C₁₂)amino en C₁-C₂₀, ou un di(aralkyle en C₇-C₂₅)amino ; ou
X et M ensemble représentent une liaison carbone-carbone ou un atome d'oxygène ;
L est un hydogène ou L et M ensemble représentent une liaison carbone-carbone ; et
soit Z est un radical R-S(=O)ₙ- et Y est un hydrogène, soit
Y est R-S(=O)ₙ- et Z est un hydrogène,
dans lequel R est un alkyle en C₁-C₂₀ linéaire ou branché éventuellement susbtitué par un alkoxy en C₁-C₂₀ ou par un alkoxycarbonyle en C₂-C₂₁, un cycloalkyle en C₅-C₇, un aryle en C₆-C₁₂ éventuellement substitué par un alkyle en C₁-C₂₀, un halogène ou CF₃, ou un arylalkyle en C₇-C₂₅ éventuellement substitué par un alkyle en C₁-C₂₀, un halogène ou CF₃ ; et
n est 0, 1 ou 2.

2. Composé selon la revendication 1 dans lequel R est un radical choisi parmi : Le *n*-butyle ; *tert-butyle;* cyclohexyle; 3-octyle; *n*-dodécyle ; octyloxyéthyle ; éthoxycarbonyliméthyle; phényle; biphényle ; 4-fluorophényle; 2,4-dichlorophényle; 2,4-difluorophényle; 2-naphthyle; benzyle; 4-phénylbutyle; 4'-(trifluorométhyle)-benzyle; (triphényle)méthyle; 4-méthylphényle ; 4-(trifluorométhyle)phényle; 2,4-diméthylphényle ; 2,4-[di-(trifluorométhyle)]phényle ; 3,5-[di-(trifluorométhyle)]-phényle.

3. Composé selon la revendication 1 dans lequel X est choisi parmi : méthoxy ; hexyloxy ; dodecyloxy; (butoxy)éthoxy; acétyloxy; benzoyloxy; diphénylacétyloxy ; (hexanoyloxy)éthoxy; butanoyloxy; octanoyloxy ; dodécanoyloxy; phénéthyloxy ; méthoxyacétyloxy; phénoxyacetyloxy ; biphényl-4-carbonoyloxy.

4. Composé selon l'une quelconque des revendications 1-3 dans lequel M est choisi parmi: méthoxy; octyloxy ; allyloxy; 3-(méthyl)but-2-ényloxy ; 4-(méthoxy)benzyloxy; cinnamyloxy ; acétyloxy ; acétylacétyloxy ; 3-(acétyloxy)but-2-énoyloxy; éthoxy-oxalyloxy; (méthyle)phénylaminoxalyloxy; di(benzyl)aminooxalyloxy; phénoxycarbonyloxy.

5. Composé selon la revendication 1 de structure **I caractérisée par** des centres stéréogéniques (1R, 5R) de formule : 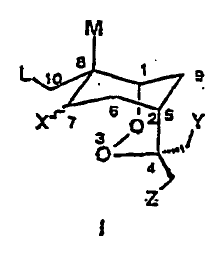 dans laquelle L, M, X, Y et Z sont tels que définis dans la revendication 1.

6. Composé selon la revendication 1 de structure **II caractérisé par** des centres stéréogéniques (1S, 5S) de formule : 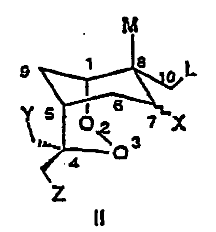 dans laquelle L, M, X, Y et Z sont tels que définis dans la revendication 1.

7. Composé selon la revendication 5 de structure **Ia caractérisé par** des centres stéréogéniques (4R) de formule : 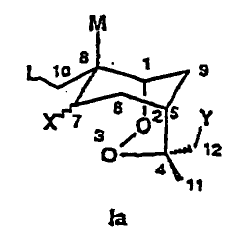 dans laquelle L, M, X, Y sont tels que définis dans la revendication 1.

8. Composé selon la revendication 5 de structure **Ib caractérisé par** des centres stéréogéniques (4S) de formule : 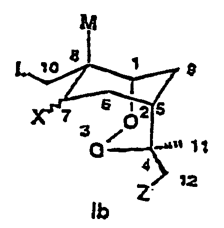 dans laquelle L, M, X et Z sont tels que définis dans la revendication 1.

9. Composé selon la revendication 6 de structure **IIa caractérisé par** des centres stéréogéniques (4S) de formule : 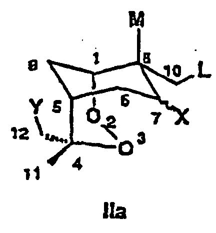 dans laquelle L, M, X, Y sont tels que définis dans la revendication 1.

10. Composé selon la revendication 6 de structure **IIb caractérisé par** des centres stéréogéniques (4R) de formule : 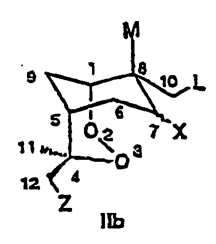 dans laquelle L, M, X et Z sont tels que définis dans la revendication 1.

11. Composé selon la revendication 1 choisi parmi : (1R,4R,5R,8R)-4,8-diméthyl-4-phénylsulfonylméthyl-2,3-dioxabicyclo [3.3.1]nonan-8-ol (21a) ; (1R,4R,5R,8S)-4,8-diméthyl-4-phénylsulfonylméthyl-2,3-dioxabicyclo[3.3.1]nonane (22a); (1R,4S,5R,8S)-4,8-diméthyl-4-phénylsulfonylméthyl-2,3-dioxabicyclo[3.3.1]nonane (22b) ; (1R,4R,5R,8R)-8-acétoxy-4,8-diméthyl-4-phénylsulfonylméthyl-2,3-dioxabicyclo [3.3.1]nonane (23a) ; (1R,4S,5R,8R)-8-acétoxy-4,8-diméthyl-4-phénylsulfonylméthyl-2,3-dioxabicyclo[3.3.1]nonane (23b) ; (1R,4R,5R,8R)-8-acétylacétoxy-4,8-diméthyl-4-phénylsulfonylméthyl-2,3-dioxabicyclo[3.3.1]nonane (24a); (1R,4R,5R,8R)-8-dibenzylaminooxalyloxy-4,8-diméthyl-4-phénylsulfonylméthyl-2,3-dioxabicyclo[3.3.1]nonane (26a); (1R,4R,5R,8R)-4,8-diméthyl-8-phénoxycarbonyloxy-4-phénylsulfonylméthyl-2,3-dioxabicyclo[3.3.1]nonane (27a) ; (1R,4R,5R,8R)-4,8-diméthyl-8-phenoxyacétoxy-4-phénylsulfonylméthyl-2,3-dioxabicyclo[3.3.1]nonane (33a); (1R,4R,5R,8R)-4,8-diméthyl-8-(p-méthoxybenzyloxy)-4-phénylsulfonylméthyl-2,3-dioxabicyclo[3.3.1]nonane (34a) ; (1R,4R,5R,8R)-4,8-diméthyl-8(γ,γ-diméthylallyloxy)-4-phénylsulfonylméthyl-2,3-dioxabicyclo [3.3.1]nonane (35a) ; (1R,4R,5R,8R)-8-(trans-cinnamyloxy)-4,8-diméthyl-4-phénylsulfonylméthyl-2,3-dioxabicyclo [3.3.1]nonane (36a) ; (1R,4R,5R,8R)-8-acétoxy-4,8-diméthyl-4-phénylsulfinylméthyl-2,3-dioxabicyclo[3.3.1]nonanes (41a, a'); (1R,4S,5R,8R)-8-acétoxy-4,8-diméthyl-4-phénylsulfinylméthyl-2,3-dioxabicyclo[3.3.1]nonane (41b').

12. Composition pharmaceutique comprenant un composé tel que défini dans l'une quelconque des revendications 1-11 et un support acceptable sur le plan pharmaceutique.

13. Utilisation d'un composé tel que défini dans l'une quelconque des revendications 1-11 pour la préparation d'une composition pharmaceutique pour la prévention et/ou le traitement de la malaria.
